(19) Europäisches Patentamt
European Patent Office
Office européen des brevets

(11) Veröffentlichungsnummer: **0 126 430 B1**

# (12) EUROPÄISCHE PATENTSCHRIFT

(45) Veröffentlichungstag der Patentschrift: **28.08.91**

(51) Int. Cl.⁵: **C07D 249/08**, C07C 43/178, C07C 43/295, C07C 41/03, A01N 43/64

(21) Anmeldenummer: **84105582.5**

(22) Anmeldetag: **16.05.84**

Die Akte enthält technische Angaben, die nach dem Eingang der Anmeldung eingereicht wurden und die nicht in dieser Patentschrift enthalten sind.

## (54) Verfahren zur Herstellung von 1-Triazolylethylether-Derivaten, sowie mikrobizide Mittel enthaltende neue 1-triazolyl-phenoxyphenylethylether-derivate als Wirkstoffe und deren Verwendung.

(30) Priorität: **19.05.83 CH 2730/83**

(43) Veröffentlichungstag der Anmeldung: **28.11.84 Patentblatt 84/48**

(45) Bekanntmachung des Hinweises auf die Patenterteilung: **28.08.91 Patentblatt 91/35**

(84) Benannte Vertragsstaaten: **AT BE CH DE FR IT LI LU NL SE**

(56) Entgegenhaltungen:
**EP-A- 0 047 594**
**EP-A- 0 052 424**
**EP-A- 0 077 479**
**EP-A- 0 082 340**
**AT-B- 371 451**
**DE-A- 2 735 872**
**GB-A- 2 064 520**

(73) Patentinhaber: **CIBA-GEIGY AG**
**Klybeckstrasse 141**
**CH-4002 Basel(CH)**

(72) Erfinder: **Nyfeler, Robert, Dr.**
**Bärenfelserstr. 8**
**CH-4057 Basel(CH)**
Erfinder: **Zondler, Helmut, Dr.**
**Oberwilerstr. 49**
**CH-4103 Bottmingen(CH)**
Erfinder: **Sturm, Elmar, Dr.**
**Klusstr. 66**
**CH-4147 Aesch(CH)**

(74) Vertreter: **Zumstein, Fritz, Dr. et al**
**Bräuhausstrasse 4**
**W-8000 München 2(DE)**

EP 0 126 430 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft insbesondere das nachfolgend beschriebene Verfahren zur Herstellung von 1-Triazolylethylether-Derivaten der Formel I

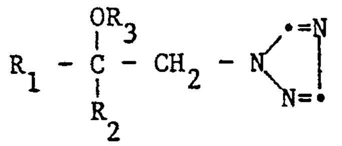

(I) ,

worin

$R_1$ für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cycloalkyl, unsubstituiertes oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Phenoxy, Halophenoxy, Phenyl, Benzyl, Halobenzyl, Nitro und/oder Cyano substituiertes Phenyl oder unsubstituiertes oder ein- bis dreifach durch Halogen, $C_1$-$C_6$-Haloalkyl, $C_1$-$C_6$-Alkoxy, $C_1$-$C_6$-Alkyl, Nitro und/oder Cyano substituiertes Benzyl steht;

$R_2$ für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cycloalkyl, unsubstituiertes oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Phenoxy, Halophenoxy, Phenyl, Benzyl, Halobenzyl, Nitro und/oder Cyano substituiertes Phenyl oder unsubstituiertes oder ein- bis dreifach durch Halogen, $C_1$-$C_6$-Haloalkyl, $C_1$-$C_6$-Alkoxy, $C_1$-$C_6$-Alkyl, Nitro und/oder Cyano substituiertes Benzyl steht; und

$R_3$ unsubstituiertes oder durch $C_1$-$C_3$-Alkoxy substituiertes $C_1$-$C_6$-Alkyl, $C_3$-$C_4$-Alkenyl, Benzyl oder Halobenzyl bedeutet.

Unter dem Begriff Alkyl selbst oder als Bestandteil eines anderen Substituenten, wie Alkoxy, Haloalkyl, Haloalkoxy, sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl, Heptyl, Octyl, Nonyl, Decyl, Undecyl, Dodecyl sowie ihre Isomeren, wie z.B. Isopropyl, Isobutyl, tert.-Butyl, Isopentyl. Die Vorsilbe Halo in der Bezeichnung eines Substituenten bedeutet hier und im folgenden, dass dieser Substituent einfach bis perhalogeniert auftreten kann. Halogen und Halo stehen stellvertretend für Fluor, Chlor, Brom oder Jod. Halogenalkyl steht somit für einen einfach bis perhalogenierten Alkylrest, wie z.B. $CHCl_2$, $CH_2F$, $CCl_3$, $CH_2Cl$, $CHF_2$, $CH_2CH_2Br$, $C_2Cl_5$, $CHBr_2$, $CHBrCl$, vorzugsweise für $CF_3$. Alkenyl steht z.B. für Propenyl-(1), Allyl, Butenyl-(1), Butenyl-(2) oder Butenyl-(3). Cycloalkyl steht je nach Zahl der genannten Kohlenstoffatome z.B. für Cyclopropyl, Cyclobutyl, Cyclopentyl, Cyclohexyl, Cycloheptyl, Cyclooctyl.

Eine wichtige und somit bevorzugte Untergruppe von Mikrobiziden bilden Verbindungen der Formel I, worin

$R_1$ und $R_2$ unabhängig voneinander für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cycloalkyl, Phenyl, ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Phenyl, durch Phenoxy, Halophenoxy, Phenyl, Halophenyl, Benzyl oder Halobenzyl substituiertes Phenyl, Benzyl oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Benzyl stehen; und $R_3$ $C_1$-$C_6$-Alkyl, durch $C_1$-$C_3$-Alkoxy substituiertes $C_1$-$C_6$-Alkyl, $C_3$-$C_4$-Alkenyl, Benzyl oder Halogenbenzyl bedeutet.

Bevorzugt sind ferner Mikrobizid der Formel I,
worin $R_1$ und $R_2$ unabhängig voneinander für
$C_1$-$C_6$-Alkyl, durch $C_1$-$C_3$-Alkoxy oder $C_3$-$C_6$-Cycloalkyl substituiertes $C_1$-$C_2$-Alkyl, $C_3$-$C_6$-Cycloalkyl, Phenyl, ein- bis dreifach durch Halogen, $C_1$-$C_3$-Haloalkyl, $C_1$-$C_3$-Alkoxy, $C_1$-$C_3$-Alkyl, Nitro und/oder Cyano substituiertes Phenyl, durch Phenoxy, Halophenoxy, Phenyl, Benzyl oder Halobenzyl substituiertes Phenyl, Benzyl oder ein- bis dreifach durch Halogen, $C_1$-$C_3$-Haloalkyl, Methyl, Methoxy, Nitro und/oder Cyano substituiertes Benzyl stehen; und $R_3$ $C_1$-$C_4$-Alkyl, durch $C_1$-$C_3$-Alkoxy substituiertes $C_1$-$C_2$-Alkyl, $C_3$-$C_4$-Alkenyl, Benzyl oder Halobenzyl bedeutet.

Besonders bevorzugt sind Mikrobizide der Formel I, worin $R_1$ für Phenyl, ein- bis dreifach durch Halogen oder einfach durch Phenoxy oder Halophenoxy substituiertes Phenyl steht; $R_2$ $C_1$-$C_6$-Alkyl oder $C_3$-$C_7$-Cycloalkyl bedeutet und $R_3$ für $C_1$-$C_6$-Alkyl, durch $C_1$-$C_3$-Alkoxy substituiertes $C_1$-$C_6$-Alkyl, $C_3$-$C_4$-Alkenyl, Benzyl oder Halobenzyl steht.

Ein Teil der Verbindungen der Formel I ist aus der Literatur bekannt. So werden z.B. in der europäischen Offenlegungsschrift 0,052,424 pflanzenfungizide aktive Triazol- und Imidazol-Derivate der

Formel X

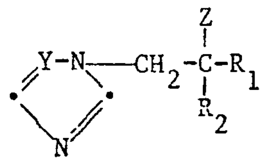

(X)

beansprucht,
worin $R_1$ für $-CH=CH-X$, $-C\equiv C-X$ oder $-CH_2CH_2X$ steht, wobei X Wasserstoff, Alkyl, Hydroxyalkyl, Alkoxyalkyl, Cycloalkyl oder gegebenenfalls substituiertes Aryl, Aralkyl, Aryloxyalkyl oder Heterocyclyl bedeutet; $R_2$ für Alkyl, Cycloalkyl oder substituiertes Aryl steht; Z Chlor, Cyano oder die Gruppe $OR_3$ bedeutet, wobei $R_3$ für Wasserstoff, Alkyl, Alkenyl oder Aralkyl steht und Y für =N- oder =CH- steht und wobei deren Säureadditionssalze und Metallkomplexe ebenfalls eingeschlossen sind. In der genannten EP-OS 0,052,424 wird vorgeschlagen, dass man die Ether der Formel (X) [Z = $OR_3$] dadurch herstellt, dass man den zugrundeliegenden tertiären Alkohol der Formel XI

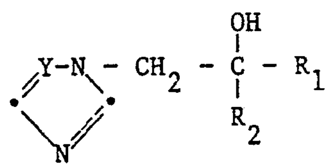

(XI) ,

mit einem entsprechenden Halogenid in Gegenwart einer geeigneten Base umsetzt. Konkrete Reaktionsbedingungen für diese Veretherung werden nicht offenbart.

In der Europäischen Offenlegungsschrift 0,047,594 wird die unter den Umfang der Formel I fallende Verbindung 1-(1,2,4-Triazol-1-yl)-bis-2-(4-fluorphenyl)-2-methoxy-ethan als fungizid und wuchsregulierender Wirkstoff beansprucht. Ihre Herstellung wird, ausgehend von dem zugrundeliegenden tertiären Alkohol, beschrieben.

Weitere Verbindungen der Formel I werden als Pflanzenfungizide in der Europäischen Offenlegungsschrift Nr. 0,082,340 beschrieben, dabei handelt es sich um Substanzen der allgemeinen Formel XX

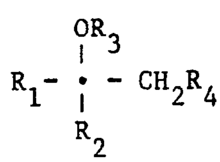

(XX) ,

worin
$R_1$ für Phenyl, ein- bis dreifach durch Halogen, $C_1$-$C_3$-Haloalkyl, Nitro, $C_1$-$C_3$-Alkoxy, $C_1$-$C_8$-Alkyl und/oder Cyano substituiertes Phenyl, durch Phenyl oder Phenoxy substituiertes Phenyl, Naphthyl, ein- oder zweifach durch Halogen, Nitro und/oder $C_1$-$C_3$-Alkyl substituiertes Naphthyl, Benzyl oder ein- oder zweifach durch Halogen, Nitro und/oder $C_1$-$C_3$-Alkyl substituiertes Benzyl steht;
$R_2$ für Phenyl, ein- oder dreifach durch Halogen, $C_1$-$C_3$-Haloalkyl, Nitro, $C_1$-$C_3$-Alkoxy, $C_1$-$C_8$-Alkyl und/oder Cyano substituiertes Phenyl, durch Phenyl oder Phenoxy substituiertes Phenyl, $C_1$-$C_{10}$-Alkyl, $C_3$-$C_8$-Cycloalkyl oder $C_3$-$C_8$-Cycloalkyl-($C_1$-$C_4$-alkyl) steht;
$R_3$ $C_1$-$C_{12}$-Alkyl, $C_2$-$C_4$-Alkenyl, $C_3$-$C_4$-Alkinyl, Benzyl oder ein- oder zweifach durch Halogen, Nitro und/oder $C_1$-$C_3$-Alkyl substituiertes Benzyl bedeutet und
$R_4$ für eine Azolylgruppe steht.
Eingeschlossen sind ihre Säureadditionssalze, quaternären Azoliumsalze und Metallkomplexe.

Das dort beschriebene Verfahren zur Herstellung der Verbindungen der Formel XX ist dadurch gekennzeichnet, dass man den zugrundeliegenden tertiären Alkohol [$R_3$ = H] alkyliert.

Der Nachteil dieser bekannten Herstellungsverfahren besteht darin, dass man jeweils die Stufe der zugrundeliegenden tertiären Alkohole durchlaufen muss. Bekanntlich bereitet aber gerade die Veretherung tertiärer Alkohole, aufgrund der sterischen Hinderung am tertiären Kohlenstoffatom, besondere Schwierigkeiten [z.B. konkurrierende Eliminierungsreaktionen) und liefert nur in exceptionellen Fällen zufriedenstellende Ausbeuten und reine Endprodukte.

Der vorliegenden Erfindung liegt daher die Aufgabe zugrunde, einen neuen praktikablen Weg zur Herstellung der 1-Triazolylethylether-Derivate der Formel I aufzuzeigen, der ohne die Zwischenstufe der entsprechenden tertiären Alkohole auskommt und die Titelverbindungen in guten Ausbeuten und hoher Reinheit liefert.

Es wurde nun überraschend gefunden, dass die 1-Triazolylethylether-Derivate der Formel I sich dadurch herstellen lassen, dass man

a) ein Oxiran der Formel II

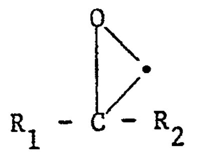

(II)

bei Temperaturen von -20° bis +100° C in Gegenwart eines sauren Katalysators bzw. eines sauren Kondensationsmittels mit einem Alkohol der Formel III

$R_3$ - OH (III)

zu einem Glykolmonoether der Formel IV reagieren lässt, und

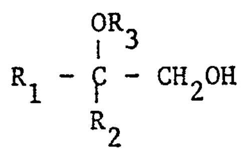

(IV)

b) anschliessend den Glykolmonoether der Formel IV oder einen seiner Ester in Gegenwart eines säurebindenden bzw. Kondensationsmittels bei Temperaturen von 0° bis 150° C mit einem Triazol der Formel V

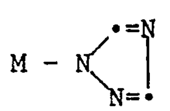

(V)

umsetzt, wobei die Substituenten $R_1$, $R_2$ und $R_3$ in den Formeln II bis IV wie unter Formel I definiert sind und M in Formel V für Wasserstoff oder ein Metallatom steht.

Geeignete saure Katalysatoren bzw. saure Kondensationsmittel sind z.B.

a) Protonensäuren, wie $HClO_4$, $H_2SO_4$, HCl, HF, HBr, $H_3PO_4$, Alkylsulfonsäuren ($CH_3SO_3H$, $C_2H_5SO_3H$, $CF_3SO_3H$, etc.), Arylsulfonsäuren (Benzolsulfonsäure, p-Brombenzolsulfonsäure, p-Toluolsulfonsäure usw.) oder Ionenaustauscher in der $H^+$-Form; bevorzugt sind $HClO_4$, $H_2SO_4$ und Ionenaustauscher.

b) Lewis-Säuren, wie $BF_3$, $BF_3$-Etherat [$BF_3$-$(C_2H_5)_2O$], $BCl_3$, $AlCl_3$, $AlBr_3$, $SnCl_4$, $TiCl_4$, $ZnJ_2$, $ZnCl_2$ etc.; bevorzugt sind $BF_3$ und $BF_3$-Etherat.

Im allgemeinen werden $10^{-3}$ bis 1 Aequivalente an Katalysator bzw. Kondensationsmittel pro Aequivalent Epoxid II eingesetzt, vorzugsweise 0,1 bis 1 Aequivalent Katalysator bzw. Kondensationsmittel.

Der erste Teilschritt a), die Reaktion des Oxirans II mit dem Alkohol III, kann in An- oder Abwesenheit eines üblichen reaktionsinerten organischen Lösungsmittels oder Lösungsmittelgemisches durchgeführt werden. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.Butylmethylether usw.), Anisol, Dioxan, Tetrahydrofuran; Nitrile wie Acetonitril, Propionitril usw.; N,N-Dimethylformamid, N,N-Dimethylacetamid; Dialkylsulfoxide wie Dimethylsulfoxid; Ketone wie Aceton, Diethylketon, Methylethylketon usw. und Gemische solcher Lösungsmittel

untereinander. Besonders eignet sich jedoch überschüssiger Alkohol der Formel III als Lösungsmittel. Die Reaktionstemperaturen liegen vorzugsweise zwischen 0° und 40° C. Die Reaktionsdauer beträgt ca. 0,5 bis 72 Stunden, überwiegend 0,5 bis 16 Stunden.

Die Reaktion a) kann prinzipiell auch ohne Katalysator bzw. Kondensationsmittel durchgeführt werden, dann müssen jedoch drastische Reaktionsbedingungen, insbesondere hohe Temperaturen gewählt werden, was zu zahlreichen Nebenprodukten und langen Reaktionszeiten führt, was somit keinem wirtschaftlich praktikablen Herstellungsverfahren entspricht.

Die Ringöffnung eines Oxirans mit einem Alkanol im Sinne der obigen Teilreaktion a) ist im Prinzip aus der Literatur bekannt. So wird z.B. die Herstellung von 2-Phenyl-2-methoxyethanol-1 gemäss folgender Gleichung

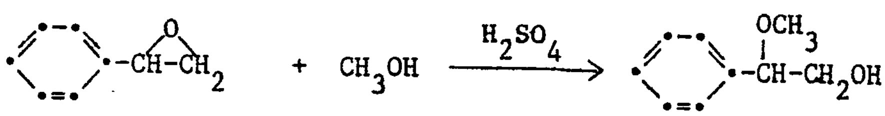


in [W. Reeve and I. Christoffel, J.Amer.Chem.Soc., 72, 1480 (1950)] beschrieben. Die Herstellung weiterer Glykolmonoether der Formel IV mit $R_1$ = H, $CH_3$, $\overline{C}F_3$, $CH_3CHBr$, $(CH_3)_3CCH_2$, $R_2$ = H, $CH_3$ und $R_3$ = $CH_3$, $C_2H_5$ aus entsprechenden Oxiranen wird in [Epoxy Resins, Chemistry and Technology, C.A. May, Y. Tanaka, Marcel Dekker (1973)] berichtet. Ein Vorschlag für die weitere Umsetzung dieser Glykolmonoether mit 1,2,4-Triazol oder eines seiner Metallsalze ist keiner der genannten Literaturstellen zu entnehmen. Es fehlen dort ferner jegliche Fingerzeige auf eine Verwendung der Glykolmonoether der Formel IV als Zwischenprodukte zur Herstellung wertvoller agrochemischer, insbesondere mikrobizider oder wuchreguliender Wirkstoffe.

Neu sind Glykolmonoether der Formel IV, worin R für ein durch $C_1$-$C_6$-Alkoxy oder $C_3$-$c_6$-Cycloalkyl substituiertes $C_1$-$C_6$-Alkyl, $C_3$-$C_8$-Cycloalkyl, Phenyl, ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Phenyl, durch Phenoxy, Halophenoxy, Phenyl, Halophenyl, Benzyl oder Halobenzyl substituiertes Phenyl, Benzyl oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Benzyl steht; $R_2$ für $C_1$-$C_{12}$-Alkyl oder einen unter $R_1$ angegebenen Rest steht; und $R_3$ $C_1$-$C_6$-Alkyl, durch $C_1$-$C_3$-Alkoxy substituiertes $C_1$-$C_6$-Alkyl, $C_3$-$C_4$-Alkenyl, Benzyl oder Halobenzyl bedeutet. Diese wichtige Untergruppe soll hier und im folgenden die Bezeichnung IV' tragen.
Innerhalb dieser Gruppe sind insbesondere jene Glykolmonoether der Formel IV'' bevorzugt, worin $R_1$ Phenyl, ein- bis dreifach durch Halogen oder einfach durch Phenoxy oder Halophenoxy substituiertes Phenyl bedeutet; $R_2$ für $C_1$-$C_6$-Alkyl oder $C_3$-$C_7$-Cycloalkyl steht und $R_3$ für $C_1$-$C_6$-Alkyl, durch $C_1$-$C_3$-Alkoxy substituiertes $C_1$-$C_6$-Alkyl, $C_3$-$C_4$-Alkenyl, Benzyl oder Halobenzyl steht.

Die Glykolmonoether der Formel IV' sind neu und stellen besonders entwickelte Zwischenprodukte zur Herstellung der wertvollen Wirkstoffe der Formel I dar. Auf Grund ihrer strukturellen Beschaffenheit lassen sie sich leicht [vgl. Teilreaktion b)] in die Verbindungen der Formel I überführen. Darüberhinaus zeigen Verbindungen der Formel IV' biozide, insbesondere insektizide und emulgierende Eigenschaften.

Die Verbindungen der Formel IV' sind daher einschliesslich ihres Herstellungsverfahrens [Reaktion a)] ein Bestandteil dieser Erfindung.

Typische Vertreter der Formel IV' sind z.B.:
2-(2,4-Dichlorphenyl)-2-methoxy-pentanol-1
2-(2,4-Dichlorphenyl)-2-ethoxy-pentanol-1
2-(2,4-Dichlorphenyl)-2-isopropoxy-pentanol 1
2-(2,4-Dichlorphenyl)-2-(2-methoxyethoxy)-pentanol-1
2-(2,4-Dichlorphenyl)-2-(2-fluorbenzyloxy)-pentanol-1
2-(4-Fluorphenyl)-2-butoxy-propanol-1
2-(4-Fluorphenyl)-2-butoxy-butanol-1
2-(4-Fluorphenyl)-2-(buten-2-yloxy)-butanol-1
2-(4-Chlorphenyl)-2-benzyloxy-pentanol-1
2-(2,4-Dichlorphenyl)-2-methoxy-propanol-1
2-(2,4-Dichlorphenyl)-2-propoxy-propanol-1
2-(2,4-Dichlorphenyl)-2-butoxy-propanol-1
2-(2,4-Dichlorphenyl)-2-allyloxy-propanol-1
2-(2,4-Dichlorphenyl)-2-methallyloxy-propanol-1

2-(2,4-Dichlorphenyl)-2-(4-chlorbenzyloxy)-propanol-1
2-(2,4-Dichlorphenyl)-2-methoxy-butanol-1
2-(2,4-Dichlorphenyl)-2-propoxy-butanol-1
2-(2,4-Dichlorphenyl)-2-allyloxy-butanol-1
2-(2,4-Dichlorphenyl)-2-propoxy-pentanol-1
2-(2,4-Dichlorphenyl)-2-butoxy-pentanol-1
2-(2,4-Dichlorphenyl)-2-allyloxy-pentanol-1
2-(2,4-Dichlorphenyl)-2-(buten-2-yloxy)-pentanol-1
2-(2,4-Dichlorphenyl)-2-benzyloxy-pentanol-1
2-(2,4-Dichlorphenyl)-2-methoxy-3-methylbutanol-1
2-(2,4-Dichlorphenyl)-2-methoxy-heptanol-1
2-(2,4-Dichlorphenyl)-2-cyclohexyl-2-methoxy-ethanol-1
2-(4-Brom-2-chlorphenyl)-2-methoxy-propanol-1
2-(4-Brom-2-chlorphenyl)-2-methoxy-propanol-1
2-(2-Chlor-4-fluorphenyl)-2-methoxy-propanol-1
2-(2-Chlor-4-fluorphenyl)-2-methoxy-butanol-1
2-(4-(4-Chlorphenoxy)-phenyl)-2-methoxy-butanol-1
2-(4-(4-Chlorphenoxy)-phenyl)-2-methoxy-propanol
2-(2-Chlor-4-fluorphenyl)-2-allyloxy-pentanol-1
2-(4-Brom-2-chlorphenyl)-2-butoxy-pentanol-1-
2-(4-(4-Fluorphenoxy)-phenyl)-2-methoxy-butanol-1
2-(4-(2,4-Dichlorphenoxy)-phenyl)-2-methoxy-butanol-1-Der

zweite Teilschritt bei der Herstellung der Verbindungen der Formel I [= Reaktion b)], d.h. die Umsetzung eines Glykolmonoethers der Formel IV oder eines seiner Ester wird vorzugsweise bei Temperaturen zwischen 20° und 100°C im Beisein eines Kondensationsmittels bzw. eines säurebindenden Mittels durchgeführt. Geeignete Mittel dieser Art sind beispielsweise tertiäre Amine wie Triethylamin, Tripropylamin, Dimethylethylamin usw, Pyridine und Pyridinbasen (4-Dimethylaminopyridin, 4-Pyrrolidylaminopyridin usw.), Oxide, Hydroxide und Hydride, Carbonate und Hydrogencarbonate von Alkali- und Erdalkalimetallen (CaO, BaO, NaOH, KOH, NaH, $Ca(OH)_2$, $KHCO_3$, $Ca(HCO_3)_2$, $K_2CO_3$, $Na_2CO_3$ usw.), sowie Alkaliacetate wie $CH_3COONa$, $CH_3COOK$ usw.. Darüberhinaus auch Alkalialkoholate wie $C_2H_5ONa$, $(C_3H_7\text{-n})ONa$, $CH_3ONa$, $C_2H_5OK$ usw., oder auch Substanzkombinationen wie Triphenylphosphin/Azodicarbonsäuredimethylester.

In einigen Fällen kann es von Vorteil sein, wenn man anstelle des freien 1,2,4-Triazols V (M = H), letzteres zuerst, beispielsweise in situ mit einem Alkoholat, in das entsprechende Salz, vorzugsweise ein Alkalimetallsalz, insbesondere Natrium- oder Kaliumsalz, überführt und anschliessend dieses Salz in Gegenwart einer der genannten Basen mit dem Glykolmonoether IV bzw. einem seiner Ester umsetzt.

Die Reaktion b) wird bevorzugt in einem relativ polaren, jedoch reaktionsinerten organischen Lösungsmittel durchgeführt, z.B. in Pyridinen, N,N-Dimethylformamid, N,N-Dimethylacetamid, Dimethylsulfoxid, Acetonitril, Benzonitril, Hexamethylphosphortriamid, Hexamethyl-phosphorsäuretriamid und anderen. Derartige Lösungsmittel können in Kombination mit weiteren, üblichen, reaktionsinerten Lösungsmitteln [vgl. bei Teilraktion a)] wie z.B. aliphatischen oder aromatischen Kohlenwasserstoffen, z.B. Benzol, Toluol, Xylolen, Hexan, Petrolether, Chlorbenzol, Nitrobenzol u.a. verwendet werden.

Ferner kann es ebenfalls von Vorteil sein, wenn man vor der Umsetzung von IV mit V, die freie Hydroxylgruppe in IV reaktionsfähig verestert und somit in eine andere reaktionsfähige nukleofuge Abgangsgruppe überführt und anschliessend den resultierenden Ester von IV mit V weiterreagieren lässt. Dabei sollen hier und im folgenden unter einer reaktionsfähigen nukleofugen Abgangsgruppe vorzugsweise Substituenten verstanden werden wie z.B. Halogen: [wie Fluor, Chlor, Brom oder Jod, vorzugsweise Chlor oder Brom]; Sulfonyloxygruppen, z.B. Fluorsulfonyloxy oder bevorzugt $-OSO_2R_a$; Acyloxygruppen, bevorzugt $-OCO-R_a$ und Isoharnstoffreste, bevorzugt

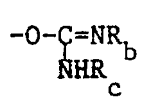 ,

wobei $R_a$, $R_b$ und $R_c$ unabhängig voneinander bevorzugt für $C_1$-$C_3$-Alkyl, $C_1$-$C_3$-Haloalkyl oder gegebenenfalls durch Halogen, Methyl, Nitro, Trifluormethyl und/oder Methoxy substituiertes Phenyl stehen.

Der Austausch der freien Hydroxylgruppe in den Verbindungen der Formel IV gegen eine andere reaktionsfähige nukleofuge Abgangsgruppe wird bevorzugt in einem reaktionsinerten Lösungsmittel durchgeführt. Beispiele solcher Lösungsmittel sind: Aromatische und aliphatische Kohlenwasserstoffe wie Benzol,

Toluol, Xylole, Petrolether, Ligroin oder Cyclohexan; halogenierte Kohlenwasserstoffe wie Chlorbenzol, Methylenchlorid, Ethylenchlorid, Chloroform, Tetrachlorkohlenstoff oder Tetrachlorethylen; Ether und etherartige Verbindungen wie Diethylether, Diisopropylether, t-Butylmethylether, Dimethoxyethan, Dioxan, Tetrahydrofuran oder Anisol; Ester wie Ethylacetat, Propylacetat oder Butylacetat; Nitrile wie Acetonitril oder Verbindungen wie Dimethylsulfoxid, Dimethylformamid und Gemische solcher Lösungsmittel untereinander.

Die Einführung der Abgangsgruppe erfolgt nach allgemein bekannten Methoden. Bedeutet die Abgangsgruppe Chlor, so wird als Reagenz z.B. Phosphoroxychlorid, Phosphortrichlorid, Phosphorpentachlorid oder vorzugsweise Thionylchlorid eingesetzt. Man arbeitet im allgemeinen bei Temperaturen von 0° bis +120° C. Bedeutet die Abgangsgruppe Brom, verwendet man bevorzugt Phosphortribromid oder Phosphorpentabromid und führt die Reaktion bei 0° bis +50° C durch. Steht sie für eine der Gruppen $-OSO_2R_a$, $-OCO-R_a$ oder

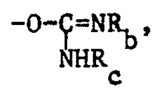

so wird als Reagenz üblicherweise das entsprechende Säurechlorid bzw. Amidinochlorid eingesetzt. Hierbei ist es zweckmässig, wenn die Reaktion bei Temperaturen von -20° bis +50° C, vorzugsweise -10° bis +30° C, und in Gegenwart einer schwachen Base wie Pyridin oder Triethylamin durchgeführt wird.

Die Ausgangsprodukte der Formeln II, III und V sind allgemein bekannt oder lassen sich nach an sich bekannten Methoden herstellen.

Das erfindungsgemässe Verfahren wird durch die weiter unten aufgeführten Beispiele näher erläutert.

Einen weiteren wichtigen Teil der vorliegenden Erfindung bilden die im Umfang der Formel I neuen Triazolylethylether-derivate.

Neu sind z.B. folgende mikrobizid, wertvolle Verbindungen der Formel I, nämlich diejenigen der nachstehenden Formel Ia

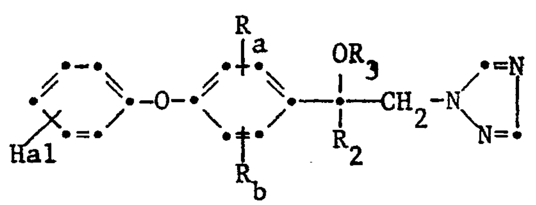

(Ia),

worin

Hal für Halogen steht; $R_a$ und $R_b$ unabhängig voneinander für Wasserstoff, Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano stehen; und $R_2$ und $R_3$ die unter Formel I angegebenen Bedeutungen haben.

Diese neuen Verbindungen der Formel Ia sind ein Teil dieser Erfindung.

Innerhalb der neuen Verbindungen der Untergruppe Ia sind auf Grund ihrer ausgeprägten mikrobiziden, vor allem pflanzenfungiziden Wirkung, die Verbindungen der Formel I bevorzugt, worin Hal für Fluor, Chlor und/oder Brom steht; $R_a$ und $R_b$ unabhängig voneinander für Wasserstoff, Halogen, $C_1$-$C_3$-Haloalkyl (vorzugsweise $CF_3$), $C_1$-$C_3$-Haloalkoxy (vorzugsweise $OCF_3$), $C_1$-$C_3$-Alkoxy, $C_1$-$C_3$-Alkyl (vorzugsweise Methyl oder Ethyl), Nitro und/oder Cyano stehen; $R_2$ für $C_1$-$C_6$-Alkyl steht; und $R_3$ unsubstituiertes oder durch $C_1$-$C_3$-Alkoxy substituiertes $C_1$-$C_6$-Alkyl (vorzugsweise $C_1$-$C_4$-Alkyl), $C_3$-$C_4$-Alkenyl (vorzugsweise Allyl), Benzyl oder Halobenzyl bedeutet.

Besonders bevorzugte Vertreter der Untergruppe Ia sind beispielsweise:

1-(1H-1,2,4-Triazol-1-yl)-2-methoxy-2-[4-(4-chlorphenoxy)-phenyl]-butan;
1-(1H-1,2,4-Triazol-1-yl)-2-methoxy-2-[4-(4-fluorphenoxy)-phenyl]-butan;
1-(1H-1,2,4-Triazol-1-yl)-2-methoxy-2-[4-(2,4-dichlorphenoxy)-phenyl]-butan;
1-(1H-1,2,4-Triazol-1-yl)-2-methoxy-2-[4-(4-chlorphenoxy)-phenyl]-propan;
1-(1H-1,2,4-Triazol-1-yl)-2-methoxy-2-[4-(4-chlorphenoxy)-2-chlorphenyl]-butan;
1-(1H-1,2,4-Triazol-1-yl)-2-methoxy-2-[4-(4-chlorphenoxy)-phenyl]-3-methyl-butan;
1-(1H-1,2,4-Triazol-1-yl)-2-allyloxy-2-[4-(4-chlorphenoxy)-2-chlorphenyl]-pentan;
1-(1H-1,2,4-Triazol-1-yl)-2-methoxy-2-[4-(4-chlorphenoxy)-2-chlorphenyl]-pentan;
1-(1H-1,2,4-Triazol-1-yl)-2-allyloxy-2-[4-(4-chlorphenoxy)-2-chlorphenyl]-butan;

1-(1H-1,2,4-Triazol-1-yl)-2-(2-methylallyloxy)-2-[4-(4-chlorphenoxy)-2-chlorphenyl]-butan;
1-(1H-1,2,4-Triazol-1-yl)-2-allyloxy-2-[4-(4-chlorphenoxy)-2-chlorphenyl]-propan;
1-(1H-1,2,4-Triazol-1-yl)-2-methoxy-2-[4-(4-chlorphenoxy)-2-chlorphenyl]-propan;
1-(1H-1,2,4-Triazol-1-yl)-2-methoxy-2-[4-(4-chlorphenoxy)-2-methylphenyl]-propan;
1-(1H-1,2,4-Triazol-1-yl)-2-allyloxy-2-[4-(4-chlorphenoxy)-2-methylphenyl]-propan;
1-(1H-1,2,4-Triazol-1-yl)-2-methoxy-2-[4-(4-chlorphenoxy)-2-methylphenyl]-butan;
1-(1H-1,2,4-Triazol-1-yl)-2-(2-methylpropoxy)-[4-(4-chlorphenoxy)-2-chlorphenyl]-butan;
1-(1H-1,2,4-Triazol-1-yl)-2-allyloxy-2-[4-(4-chlorphenoxy)-2-methylphenyl]-pentan;
1-(1H-1,2,4-Triazol-1-yl)-2-methoxy-2-[4-(4-chlorphenoxy)-2-methylphenyl]-butan;
1-(1H-1,2,4-Triazol-1-yl)-2-allyloxy-2-[4-(4-chlorphenoxy)-2-methylphenyl]-butan;
1-(1H-1,2,4-Triazol-1-yl)-2-propoxy-2-[4-(4-chlorphenoxy)-2-chlorphenyl]-pentan;
1-(1H-1,2,4-Triazol-1-yl)-2-n-propoxy-2-[4-(4-chlorphenoxy)-2-chlorphenyl]-butan;
1-(1H-1,2,4-Triazol-1-yl)-2-n-propoxy-2-[4-(4-chlorphenoxy)-2-chlorphenyl]-propan;
1-(1H-1,2,4-Triazol-1-yl)-2-n-propoxy-2-[4-(4-chlorphenoxy)-2-methylphenyl]-propan.

Die Verbindungen der Formel Ia sind bei Raumtemperatur Feststoffe, Oele oder überwiegend Harze, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Dabei sind die Triazolyl-Derivate im Umfang der Formel I bevorzugt. Die erfindungsgemässen Wirkstoffe der Formel I zeichnen sich durch eine sehr gute mikrobizide Aktivität und problemlose Anwendung aus.

Ausser nach dem eingangs beschriebenen Verfahren können die neuen Verbindungen der Formel Ia auch dadurch
hergestellt werden, dass man eine Verbindung der Formel IIa

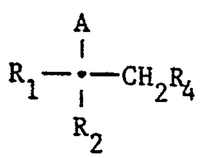

(II a)

mit einer Verbindung der Formel IIIa

$R_3$-W (IIIa)

reagieren lässt, wobei die Substituenten $R_1$, $R_2$, $R_3$ und $R_4$ wie unter Formel Ia definiert sind, A und W für -OH, -OM oder eine der üblichen Abgangsgruppen stehen, M ein Alkali- oder Erdalkalimetallatom repräsentiert, mit der Massgabe, dass die Reaktionspartner IIa und IIIa stets so gewählt werden, dass entweder eine MO- oder HO-Funktion mit einer Abgangsgruppe oder zwei Hydroxyfunktionen miteinander zur Reaktion gelangen.

Unter einer üblichen Abgangsgruppe sollen die eingangs beschriebenen Abgangsgruppen verstanden werden.

Soweit es sich um Alkohole oder Alkoholate der Formel IIa handelt (A = OH oder OM), wird man Verbindungen der Formel Ia in der Praxis durch übliche Verätherung mit einer Verbindung der Formel IIIa herstellen,worin W ein Halogenid, bevorzugt ein Chlorid oder Bromid, bedeutet. Dabei arbeitet man im Temperaturberich von 0° bis 150° C entweder ohne Lösungsmittel, bevorzugt aber in atropen Lösungsmitteln wie aromatischen und aliphatischen Kohlenwasserstoffen, Ether und etherartigen Verbindungen (Diethylether, Dioxan, Tetrahydrofuran [THF] etc.), Acetonitril, Dimethylformamid [DMF] und anderen, die dem Fachmann bei Verätherungsreaktionen geläufig sind. Auch die Herstellung im Phasentransfer-Verfahren ist empfehlenswert.

Alkohole vom Typ der Formel IIa (A = OH) sind aus der Literatur bekannt oder können analog zu den dort beschriebenen Methoden hergestellt werden.

In allen Fällen, in denen die Substituenten $R_1$ und $R_2$ in den Verbindungen der Formel Ia verschieden sind, besitzen die Verbindungen der Formel I nachbarständig zur Sauerstoffunktion ein Asymmetriezentrum (*)

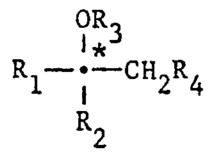

(I)

und können daher in zwei enantiomeren Formen vorliegen. Im allgemeinen entsteht bei der Herstellung dieser Substanzen ein Gemisch beider enantiomerer Formen. Dieses lässt sich auf die üblichen Methoden der Enantiomerentrennung z.B. durch fraktionierte Kristallisation eines diastereomeren Salzgemisches mit einer optisch aktiven starken Säure oder säulenchromatographisch an einem optisch aktiven Träger und mit einem optisch aktiven Eluierungsmittel in die optischen Antipoden aufspalten. Beide Antipoden zeigen eine unterschiedliche mikrobizide Aktivität. Sofern nicht speziell hervorgehoben, liegt bei der Nennung einer Verbindung der Formel Ia stets ein Gemisch beider enantiomerer Formen vor.

Es hat sich überraschenderweise gezeigt, dass die Aktivsubstanzen der Formel Ia bzw. entsprechende Mittel ein für praktische Bedürfnisse sehr günstiges Mikrobizidspektrum vor allem gegen phytopathogene Pilze aufweisen. So besitzen die Verbindungen der Formel Ia sehr günstige kurative, präventive und systemische Wirkung zum Schutz von Plfanzen, insbesondere Kulturpflanzen, ohne diese nachteilig zu beeinflussen.

Mit den Wirkstoffen der Formel Ia können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe der Formel Ia sind gegen die den folgenden Klassen angehörenden phytopathogenen Pilze wirksam: Fungi imperfecti (z.B. Botrytis, Helminthosporium, Fusarium Septoria, Cercospora und Alternaria); Basidiomyceten (z.B. die Gattungen Hemileia, Rhizoctonia, Puccinia); insbesondere wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft auch Mittel, die als mindestens eine Aktivsubstanz eine Verbindung der Formel Ia enthalten, sowie die Verwendung der Mittel oder der Wirkstoffe allein zur Bekämpfung und/oder Verhütung eines Befalls von Mikroorganismen.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer):oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops).

Wirkstoffe der Formel Ia werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel Ia werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100g bis 2 kg AS/ha, insbesondere bei 200 g bis 600 g AS/ha.

Die Formulierungen d.h. die den Wirkstoff der Formel Ia und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden). Diese Massnahmen sind dem Fachmann geläufig.

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon,Dimethylsulfoxid oder Dimethylformamid, sowie gegegenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel Ia nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:

"Mc Cutcheon's Detergents and Emulsifiers Annual" BC Publishing Corp., Ridgewood, New Jersey, 1981.
Helmuth Stache "Tensid-Taschenbuch", Carl Hanser-Verlag, München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% fester oder flüssiger Zusatzstoffe und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die nachfolgenden Beispiele dienen zur näheren Erläuterung der Erfindung, ohne dieselbe einzuschränken.

## Herstellungder Ausgangsprodukte

Beispiel I: Herstellung von

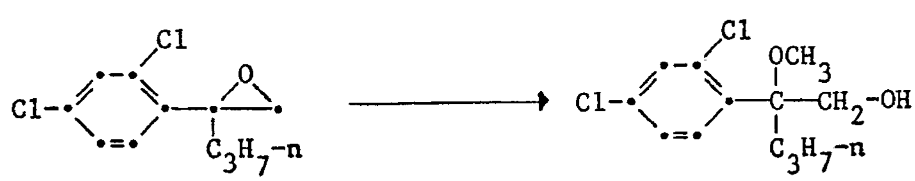

2-(2,4-Dichlorphenyl)-2-methoxypentanol-1

a) Zu einer Lösung von 20,7 g (90 mMol) 2-(2,4-Dichlorphenyl)-1,2-epoxypentan in 100 ml absolutem Methanol werden 12,5 ml Bortrifluorid-Ethyletherat langsam zugetropft. Durch gelegentliches Kühlen wird die Temperatur bei 18-20° C gehalten. Anschliessend wird die Temperatur weitere 6 Stunden bei 18-20° C gehalten und das Gemisch dann noch 1 Stunde bei 30° C gerührt. Die Lösung wird auf eiskalte, verdünnte Natriumbicarbonat-Lösung gegossen und das resultierende Gemisch zweimal mit Diethylester

extrahiert. Die vereinigten Ether-Extrakte werden zweimal mit halbgesättigter Natriumbicarbonat-, dann zweimal mit Natriumchlorid-Lösung gewaschen, über Natriumsulfat getrocknet, filtriert und eingeengt. Man erhält 26,2 g einer gelblichen, schwach viskosen Flüssigkeit, die mittels einer Vigreux-Kolonne im Hochvakuum destilliert wird. Das derart erhaltene 2-(2,4-Dichlorphenyl)-2-methoxypentanol-1 ist ein farbloses, viskoses Oel mit Sdp. 88-90 °C/10,7$\cdot 10^{-3}$ mbar, $n_D^{50}$ 1.5322. Ausbeute 12,1 g (= 51% der Theorie), 100 MHz -$^1$H-NMR($CDCl_3$): $\delta$= 7.15-7.6 ppm (m, 3H,aromatisch); 4.1 ppm (dd, 2H,-$C\underline{H}_2OH$); 3.25 ppm (s, 3H, -$OC\underline{H}_3$); 1.7-2.3 ppm

$$(m,\ 3H, -O-\overset{|}{\underset{|}{C}}-\underline{CH}_2-CH_2CH_3,\ -O\underline{H}\ ;$$

1H verschwindet beim Austausch mit $D_2O$); 0.7-1.25 ppm (m, 5H, -$CH_2C\underline{H}_2C\underline{H}_3$).

| Analyse [%]: | | berechnet | gefunden |
|---|---|---|---|
| | C | 54,8 | 55,0 |
| | H | 6,1 | 6,2 |
| | Cl | 26,9 | 26,9 |

b) Eine Lösung von 3,1 g (13,5 mMol) 2-(2,4-Dichlorphenyl)-1,2-epoxypentan in 15 ml absolutem Methanol wird bei 22-25 °C zu einer Lösung von 0,3 ml konzentrierter Schwefelsäure (95-97%) in 15 ml absolutem Methanol getropft und das Gemisch bei 22-25 °C gerührt. Nach 5,5 Stunden ist gaschromatographisch kein Epoxid mehr nachweisbar, aber das gewünschte Produkt in ca. 75%iger Ausbeute. Die Lösung wird auf eiskalte verdünnte Natriumbicarbonatlösung gegossen und das Gemisch zweimal mit Diethylether extrahiert. Die vereinigten Extrakte werden dreimal mit halbgesättigter Natriumchlorid-Lösung gewaschen, über Natriumsulfat getrocknet, filtriert und eingeengt. Ausbeute an Rohprodukt 3,8 g einer gelblichen, schwach viskosen Flüssigkeit. Nach der säulenchromatographischen Reinigung (Kieselgel; Essigsäureethylester/Petroleumbenzin 1:4) erhält man 2,3 g (= 65,9% der Theorie) des reinen 2-(2,4-Dichlorphenyl)-2-methoxypentanol-1, dessen Daten mit den unter a) angegebenen übereinstimmen.

c) Die Variation der Reaktionsparameter führt zu folgenden Ergebnissen:

| Katalysator | Ausgangs-produkt [Aequivalente] | Reaktions-temperatur [°C] | Reaktions-dauer [Stunden] | Ausbeute [%] |
|---|---|---|---|---|
| Perchlorsäure 70% | 0,5 | 0° | 86 | 75 1) |
| Perchlorsäure 70% | 0,5 | 20° | 22 | 80 1) |
| Perchlorsäure 70% | 0,5 | 20° | 22 | 70 2) |
| Perchlorsäure 70% | 0,5 | 65° | 0,25 | 64 1) |
| Dowex 50W($H^+$)® * | 0,5 | 20° | 18 | 62 1) |

1) gaschromatographische Bestimmung, mittels Eichkurven und internem Standard,

2) isolierte Ausbeute.

*) stark saurer Ionenaustauscher mit $SO_3H$-Gruppen in der $H^+$-Form.

Beispiel II: Herstellung von

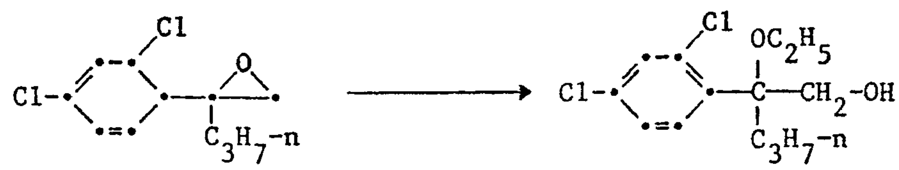

2-(2,4-Dichlorphenyl)-2-ethoxy-pentanol-1

27,6 g (120 mMol) 2-(2,4-Dichlorphenyl)-1,2-epoxypentan werden in 60 ml Ethanol gelöst. Man lässt bei 0° C eine Lösung von 17,4 g (120 mMol) Bortrifluorid-Etherat in 60 ml Ethanol zutropfen, wobei die Innentemperatur durch Kühlung mit Eiswasser auf 0° -5° C gehalten wird. Danach lässt man das Gemisch bei +7° C langsam weiterreagieren. Sobald sich gaschromatographisch kein Epoxid mehr nachweisen lässt, wird das Reaktionsgemisch mit Methylenchlorid extrahiert. Die vereinigten Extrakte werden mit Wasser gewaschen, über Natriumsulfat getrocknet, filtriert und im Vakuum eingeengt. Die Ausbeute an Rohprodukt beträgt 32,3 g. Die säulenchromatographische Reinigung (Kieselgel; Petrolether/Essigsäureethylester 4:1) führt zu 15,96 g (53% der Theorie, unter Berücksichtigung einer 90%igen Reinheit des eingesetzten Epoxids). Sdp. 90° C/0,07 mbar. 100 MHz-$^1$H-NMR ($CDCl_3$): $\delta$ = 7.0-7.6 ppm (m, aromatische H); 3.7-4.4 ppm (m, $-CH_2OH$); 3.2-3.6 ppm (q, $-OCH_2CH_3$); 1.6-2.2 ppm (m, $-CH_2-$, OH); 0.7-1.4 ppm (t, $-OCH_2CH_3$ und

$$m, -\overset{|}{\underset{|}{C}}-CH_2CH_3)$$

| | | berechnet | gefunden |
|---|---|---|---|
| Analyse [%] | C | 56,3 | 56,3 |
| | H | 6,5 | 6,8 |
| | Cl | 25,6 | 25,6 |

Beispiel III: Herstellung von

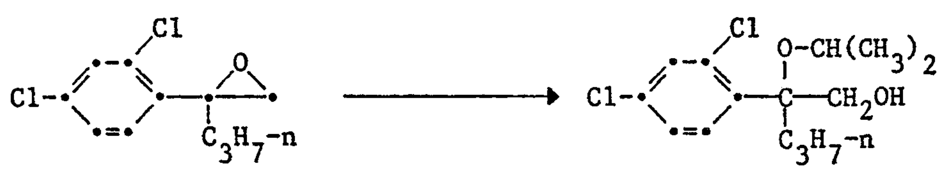

2-(2,4-Dichlorphenyl)-2-isopropoxypentanol-1

Zu einer Lösung von 17,3 g (75 mMol) 2-(2,4-Dichlorphenyl)-1,2-epoxypentan in 90 ml 2-Propanol werden bei 20-24° C 9,3 ml (75 mMol) Bortrifluorid-Etherat getropft. Die klare Lösung wird 24 Stunden bei Raumtemperatur stehengelassen, dann auf verdünnte, eiskalte Natriumbicarbonat-Lösung gegossen und das Gemisch zweimal mit Diethylether extrahiert. Die vereinigten Etherextrakte werden nochmals mit halbgesättigter Natriumchlorid-Lösung gewaschen, über Natriumsulfat getrocknet, filtriert, das Filtrat eingeengt und säulenchromatographisch (Kieselgel; Essigsäureethylester/Petroleumbenzin 1:7) gereinigt. Das Produkt fällt als farbloses Oel an. $n_D^{50}$ 1.5168.

100 $MH_z$-$^1$H-NMR ($CDCl_3$): $\delta$ = 7.1-7.8 ppm (m, 3H aromatisch); 3.7-4.4 ppm (m, 3H, $-CH_2-OH$, $-OCH(CH_3)_2$); 1.5-2.3 ppm (m, 3H, $-CH_2-CH_2CH_3$, -OH, Signal von 1H verschwindet bei Zugabe von $D_2O$); 1.25 ppm (dd, 6H, $-OH(CH_3)_2$); 0.8-1.4 ppm

(m, 5H, $-\overset{|}{\underset{|}{C}}-CH_2C\underline{H}_2C\underline{H}_3$).

| | | berechnet | gefunden |
|---|---|---|---|
| Analyse [%] | C | 57,7 | 57,8 |
| | H | 6,9 | 6,9 |
| | Cl | 24,4 | 24,3 |

Beispiel IV: Herstellung von

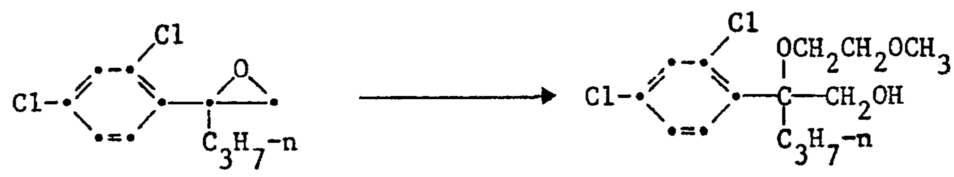

2-(2,4-Dichlorphenyl)-2-(2-methoxyethoxy)-pentanol-1

Zu einer Lösung von 3,45 g (15 mMol) 2-(2,4-Dichlorphenyl)-1,2-epoxypentan in 60 ml 2-Methoxyethanol werden bei 24-28° C 2,0 ml (16,5 mMol) Bortrifluorid-Ethyletherat zugetropft. Die Reaktionslösung bleibt noch 1,75 Stunden bei Raumtemperatur stehen und wird dann auf eiskalte, verdünnte Natriumbicarbonat-Lösung gegossen. Das Gemisch wird zweimal mit Diethylether extrahiert. Die vereinigten Etherextrakte werden zweimal mit Wasser und einmal mit halbgesättigter Natriumchlorid-Lösung gewaschen, über Natriumsulfat getrocknet, filtriert und eingeengt. Nach der säulenchromatographischen Reinigung (Kieselgel; Essigsäureethylester/Petroleumbenzin 1:8, dann 1:4) erhält man 2,0 g (= 43,3% der Theorie) des reinen Produktes als farbloses, viskoses Oel. $n_D^{50}$ 1.5161.

100 MHz-$^1$H-NMR ($CDCl_3$):δ = 7,1-7,6 ppm (m, 3H aromatisch); 4,05 ppm (dd, 2H, $-C\underline{H}_2-OH$); 3,2-3,7 ppm (m, 8H, $-OC\underline{H}_2C\underline{H}_2O-CH_3$, $-O\underline{H}$, 1H verschwindet nach Zugabe von $D_2O$); 1.7-2.4 ppm

(m, 2H $-\overset{|}{\underset{|}{C}}-C\underline{H}_2-CH_2-CH_3$);

0.75-1.4 ppm

(m, 5H, $-\overset{|}{\underset{|}{C}}-CH_2C\underline{H}_2C\underline{H}_3$).

| | | berechnet | gefunden |
|---|---|---|---|
| Analyse [%] | C | 54,7 | 55.1 |
| | H | 6,6 | 6,7 |
| | Cl | 23,1 | 22,5 |

Beispiel V: Herstellung von

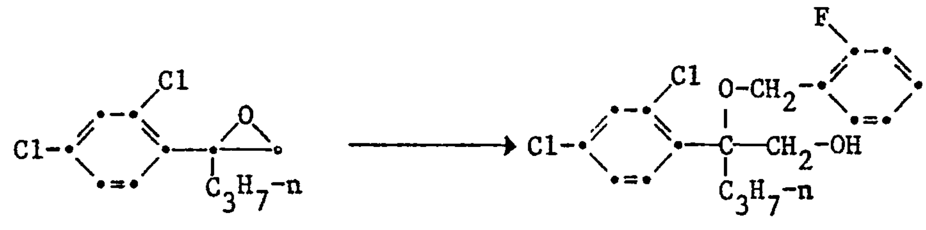

2-(2,4-Dichlorphenyl)-2-(2-fluorbenzyloxy)-pentanol-1

Zu einer Lösung von 2,0 ml (16,5 mMol) Bortrifluorid-Ethyletherat in 10 ml Diethylether lässt man bei 0°-2°C ein Gemisch von 11,6 g (92 mMol) 2-Fluorbenzylalkohol und 21,9 g (92 mMol) 2-(2,4-Dichlorphenyl)-1,2-epoxypentan zutropfen. Man lässt die Lösung über Nacht bei +7°C weiterreagieren und arbeitet wie in Beispiel 1 durch Extraktion und Säulenchromatographie auf. Man erhält 2,95 g des reinen Produkts als farbloses Oel.

100 MHz-$^1$H-NMR ($CDCl_3$): $\delta$ = 6.8-7.7 ppm (m, 7H aromatisch); 4.5 ppm

$$(s,\ 2H, -\overset{|}{\underset{|}{C}}-OC\underline{H_2}-);$$

3.9-4.4 ppm (m, 2H, -C$\underline{H_2}$OH); 1.7-2.3 ppm (m, 3H, -C$\underline{H_2}$CH$_2$CH$_3$ und O$\underline{H}$); 0.7-1.4 ppm (m, 5H, -C$\underline{H_2}$C$\underline{H_3}$).

| | | berechnet | gefunden |
|---|---|---|---|
| Analyse [%] | C | 60,5 | 60,4 |
| | H | 5,4 | 5,5 |
| | F | 5,3 | 5,3 |
| | Cl | 19,9 | 19,6 |

Beispiel VI: Herstellung von

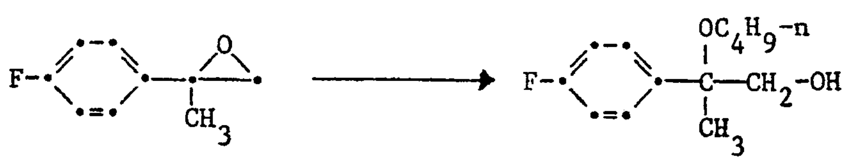

2-(4-Fluorphenyl)-2-(n-butoxy)-propanol-1

Zu einer Lösung von 2,13 g (0,016 Mol) Bortrifluorid-Ethyletherat in 5,55 g (75 mMol) n-Butanol lässt man unter Kühlung bei 5°-7°C Innentemperatur eine Lösung von 4,56 g (30 mMol) 2-(4-Fluorphenyl)-1,2-epoxypropan in 5,55 g (75 mMol) n-Butanol langsam zutropfen und hält die Temperatur weitere 4 Stunden bei +7°C. Danach arbeitet man das Reaktionsgemisch durch Extraktion (Methylenchlorid) und Waschen mit Wasser auf. Die organische Phase wird nach dem Trocknen über Natriumsulfat filtriert und im Vakuum eingeengt. Die Destillation des Rohproduktes ergibt 4,48 g (= 66% der Theorie) des reinen Endproduktes als farbloses Oel. Sdp. 120-125°C/20 mbar.

100 MHz-$^1$H-NMR ($CDCl_3$): $\delta$ = 6.8-7.5 ppm (m, 4H aromatisch); 3.5 ppm (q, 2H, -C$\underline{H_2}$OH); 3.2 ppm (m, 2H, -OC$\underline{H_2}$-); 2.3 ppm (breit, 1H, -O$\underline{H}$); 1.6 ppm (s, 3H, -C$\underline{H_3}$); 1.4 ppm (m, 4H, -C$\underline{H_2}$C$\underline{H_2}$-); 0.9 ppm (t, 3H, -C$\underline{H_3}$).

| | | berechnet | gefunden |
|---|---|---|---|
| Analyse [%] | C | 69,0 | 69,2 |
| | H | 8,5 | 8,3 |
| | F | 8,4 | 8,5 |

Beispiel VII: Herstellung von

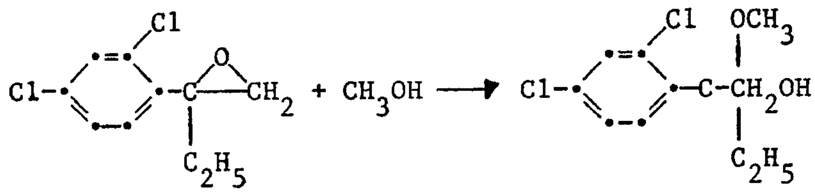

2-(2,4-Dichlorphenyl)-2-methoxybutanol-1

Zu einer Lösung von 16,25 g (75 m Mol) 2-(2,4-Dichlorphenyl)-1,2-epoxybutan von 95%iger Reinheit in 30 ml Methanol werden 10,64 g Bortrifluorid-Etherat gelöst in 30 ml Methanol bei 4-6° C zugetropft. Danach lässt man die klare Lösung im Wasserbad auf Raumtemperatur (20° C) kommen und arbeitet nach 16 Stunden durch Extraktion mit Wasser und Chloroform auf. Nach dem Waschen der Chloroformlösung mit $NaHCO_3$ wird mit $Na_2SO_4$ getrocknet und das Lösungsmittel am Rotationsverdampfer entfernt. Man erhält 19 g Rohprodukt, das durch Chromatographie mittels einer Kieselgelsäure unter Verwendung eines Gemisches aus 4 Teilen Petroläther und 1 Teil Essigester gereinigt wird. Nach der Entfernung des Lösungsmittels bei 60° C im Vakuum bei 20 mbar erhält man 13,35 g (75,2%) Reinprodukt als farbloses Oel.
60 MHz - $^1$H-NMR ($DCCl_3$): $\delta$= 7.1 - 7.6 ppm (m, 3H, aromatisch); 3.7 - 4.6 ppm (m, -$CH_2$OH); 3.28 ppm (s, $OCH_3$); 1.6 - 2.3 ppm (m, $CH_2CH_3$, OH); 0.67 ppm (t, $CH_2CH_3$).

Beispiel VIII: Herstellung von

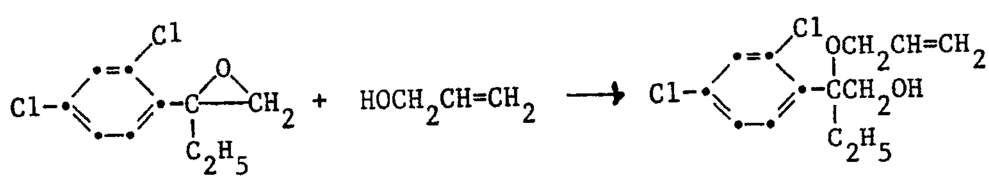

2-(2,4-Dichlorphenyl)-2-allyloxybutanol-1.

Zu einer Lösung von 5,67 g Bortrifluorid-Etherat (40 mMol) in 23,2 g Allylalkohol (400 mMol) lässt man bei 7-8° C 8,68 g (38 mMol) 2-(2,4-Dichlorphenyl)-1,2-epoxybutan von 95%iger Reinheit zutropfen. Man hält das Gemisch über Nacht im Wasserbad bei 20° C und extrahiert die Lösung mit Chloroform und Wasser. Nach dem Trocknen der organischen Phase mit $Na_2SO_4$ und dem Entfernen des Lösungsmittels am Rotationsverdampfer erhält man 11,3 g Rohprodukt, das mittels einer Chromatographiesäure (Kieselgel; Laufmittel 4 Teile Petrolether/1 Teil Essigester) gereinigt wird. Die Ausbeute an Reinprodukt beträgt 5,28 g (50,5 % d. Therorie) farbloses Oel. Brechungsindex $n_D^{50}$ = 1,5291. 60 MHz - $^1$H-NMR($DCCl_3$): $\delta$ = 7.1-7.6 ppm (m,3H,aromatisch); 5.0-6.5 ppm (m, 3H; olefinisch): 3.8-4.4 ppm (m, 4H; 2 x $OCH_2$) 1.5-2.5 ppm (m; 3H $CH_2CH_3$,OH); 0,67 ppm (t, 3H, $CH_2CH_3$).

Beispiel IX: Herstellung von

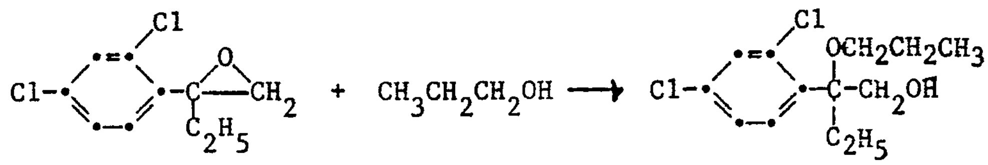

2-(2,4-Dichlorphenyl)-2-propoxybutanol-1.

Man löst 8,68 g (0,04 Mol) 2-(2,4-Dichlorphenyl)-1,2-epoxybutan von 95 %iger Reinheit in 12 g (0,2 Mol) n-Propanol, und lässt bei 20° C eine Lösung von 5,68 g (0,04 Mol) Bortrifluorid-Etherat in 12 g (0,2 Mol) n-Propanol zutropfen. Nach 24 Stunden wird mit Chloroform extrahiert, das Lösungsmittel am Rotationsverdampfer entfernt und der Rückstand durch Säulenchromatographie mit Kieselgel gereinigt. Die Ausbeute beträgt 5,41 g (48,8 %) an farblosem Oel. 60 MHz - $^1$H-NMR ($DCCl_3$) : $\delta$ = 7,0-7,6 ppm (m, 3H, arom.); 3,7-4,4 ppm (m, 2H, $CH_2OH$); 3,3 ppm (t, 2H, $OCH_2CH_2$-); 1,4-2,6 (m, 5H, $2xCH_2$, OH); 0,5-1,3 ppm (m, 6H, $2xCH_3$).

| | | berechnet | gefunden |
|---|---|---|---|
| Analyse (%) | C | 56,33 | 56,69 |
| | H | 6,55 | 6,62 |
| | Cl | 25,58 | 25,18 |

Beispiel X: Herstellung von

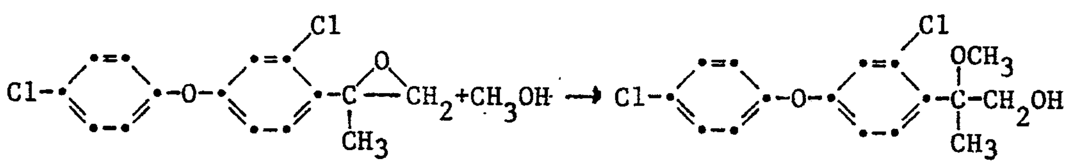

2-[2-Chlor-4-(4-chlorphenoxy)-phenyl]-2-methoxypropanol-1.

10,0 g (0,034 Mol) 2-[2-Chlor-4-(4-chlorphenoxy)-phenyl]-1,2-epoxypropan werden bei Raumtemperatur in 30 ml Methanol gelöst und durch Zugabe von 10 Tropfen einer Lösung von 4,8 g Bortrifluorid-Etherat in 10 ml Methanol zur Reaktion gebracht. Durch Eiskühlung hält man die Temperatur unter 25° C; bereits nach 10 Minuten ist im Dünnschichtchromatogramm kein Epoxyd mehr nachweisbar. Die Aufarbeitung durch Extraktion mit Wasser und Chloroform, gefolgt von einer Säulenchromatographie (Kieselgel; 4 Teile Petrolether/1 Teil Essigester) des Rohproduktes ergibt 9,37 g (84,5 %) reines Produkt als farbloses Oel. 60 MHz-$^1$H-NMR ($DCCl_3$): $\delta$ = 6,7-7,6 ppm (m, 7H, arom.); 3,7-4,2 ppm (m, 2H, $OCH_2$); 3.2 ppm (s, 3H, $OCH_3$); 2,1-2,4 ppm (t, 1H, OH); 1,7 ppm (s, 3H, $CH_3$).

| | | berechnet | gefunden |
|---|---|---|---|
| Analyse (%) | C | 58,73 | 59,36 |
| | H | 4,93 | 5,18 |
| | Cl | 21,67 | 21,04 |

## Herstellung der Endprodukte

Beispiel 1: Herstellung von

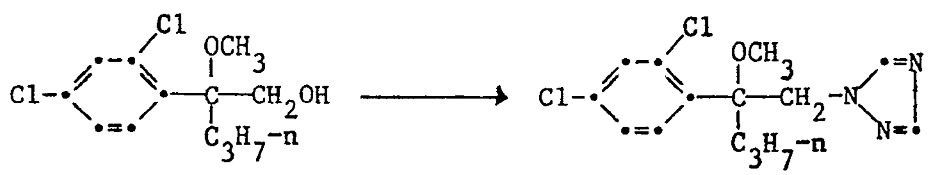

(1)

1-(1H-1,2,4-Triazol-1-yl)-2-methoxy-2-(2,4-dichlorphenyl)-pentan

a) Herstellung des Zwischenproduktes 1-(Methylsulfonyloxy)-2-methoxy-2-(2,4-dichlorphenyl)-pentan

Zu einer Lösung von 4,2 g (16 mMol) 2-(2,4-Dichlorphenyl)-2-methoxypentanol-1und 0,1 g 4-Dimethylaminopyridin in 50 ml Pyridin lässt man bei 20°-25° C 1,6 ml (21 mMol) Methansulfochlorid zutropfen. Nach kurzer Zeit beginnt die Ausscheidung farbloser Kristalle. Die Lösung wird 16 Stunden stehengelassen, anschliessend auf Eiswasser gegossen und zweimal mit Diethylether extrahiert. Die vereinigten Etherextrakte werden je zweimal mit Wasser und eiskalter, verdünnter Salzsäure sowie mit halbgesättigter Natriumchloridlösung gewaschen, über Natriumsulfat getrocknet, filtriert und eingeengt. Das auf diese Weise erhaltene Rohprodukt kann ohne weitere Reinigung in der nächsten Stufe eingesetzt werden. Zur Charakterisierung wird eine kleine Probe des Rohprodukts säulenchromatographisch (Kieselgel; Essigsäureethylester/Petroleumbenzin 1:4) gereinigt. Man erhält ein farbloses Oel; $n_D^{50}$ 1.5197.
100 MHz-$^1$H-NMR ($CDCl_3$): $\delta$= 7.2-7-7 ppm (m, 3H , aromatisch); 4.75 ppm (dd, 2H, -$\underline{CH_2}$-$OSO_2CH_3$); 3.35 ppm (s, 3H, -$O\underline{CH_3}$); 2.9 ppm (s, 3H, $\underline{CH_3}SO_2$); 1.8-2.4 ppm

$$(m,\ 2H,\ -\overset{|}{\underset{|}{C}}-CH_2-\underline{CH_2}CH_3);$$

0.8-1.4 ppm (m, 5H, -$CH_2$-$\underline{CH_2}\underline{CH_3}$).

| | | berechnet | gefunden |
|---|---|---|---|
| Analyse [%] | C | 45,8 | 46,0 |
| | H | 5,3 | 5,4 |
| | S | 9,4 | 9,3 |
| | Cl | 20,8 | 20,7 |

b) Herstellung des Endproduktes

Das nach a) hergestellte Rohprodukt wird in 50 ml absolutem Dimethylsulfoxid gelöst und mit 2,2 g (24 mMol) 1,2,4-Triazol-Natriumsalz versetzt. Das Reaktionsgemisch wird 16 Stunden bei einer Badtemperatur

von 120° C gerührt. Die entstehende dunkle Lösung wird auf Raumtemperatur abgekühlt, auf Eiswasser gegossen und zweimal mit Essigsäureethylester extrahiert. Die vereinigten Extrakte werden viermal mit halbgesättigter Natriumchloridlösung gewaschen, über Natriumsulfat getrocknet, filtriert und eingeengt. Das Rohprodukt wird säulenchromatographisch (Kieselgel; Essigsäureethylester/Petroleumbenzin 1:1) gereinigt und führt zu 3,2 g (= 64% der Theorie) eines farblosen Oels. Sdp. 170-175° C/0,04 mbar.
60 MHz-$^1$H-NMR ($CDCl_3$): $\delta$ = 7.75 und 7.60 ppm (2s, 2H des Triazols); 6.9-7.5 ppm (m, 3H, aromatisch); 4.8 ppm (4 Linien-AB-Spektrum, 2H, $CH_2N$); 3.4 ppm (s, 3H, $OCH_3$); 1.7-2.5

(m, 2H, $O-\overset{|}{\underset{|}{C}}-CH_2$);

0.7-1.5 ppm (m, 5H, $CH_2CH_3$).

Beispiel 2: Herstellung von

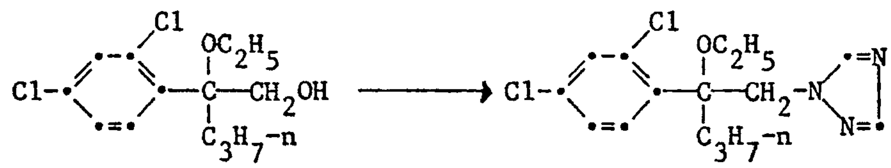

(2)

1-(1H-1,2,4-Triazol-1-yl)-2-ethoxy-2-(2,4-dichlorphenyl)-pentan

a) Herstellung des Zwischenproduktes 1-(Methylsulfonyloxy)-2-ethoxy-2-(2,4-dichlorphenyl)-pentan

6,93 g (25 mMol) 2-(2,4-Dichlorphenyl)-2-ethoxypentanol-1 werden in 20 ml Pyridin gelöst und durch Zugabe von 3,15 g (27,5 mMol) Methansulfochlorid in das entsprechende Mesylat überführt. Die Reaktion verläuft exotherm unter Ausscheidung von Pyridin-Hydrochlorid. Nach einer Reaktionsdauer von einer Stunde wird Wasser zugegeben und mit Diethylether extrahiert. Die vereinigten Extrakte werden mit halbgesättigter Natriumchloridlösung gewaschen, über Natriumsulfat getrocknet, filtriert und im Vakuum vom Lösungsmittel befreit. Ausbeute 8,6 g.
100 MHz-$^1$H-NMR ($CDCl_3$): $\delta$ = 4.7 ppm

(d, 2H, $-\overset{|}{\underset{|}{C}}-\underline{CH_2}OSO_2-$);

3.2-3.6 ppm (q, 2H, $-OCH_2CH_3$); 2.8 ppm (s, 3H, $-OSO_2CH_3$); 1.8-2.5 ppm (m, 2H, $-CH_2CH_2CH_3$); 1.3 ppm (t, 3H, $-OCH_2CH_3$); 0.7-1.3 ppm (m, 5H, $-CH_2CH_2CH_3$).

b) Herstellung des Endproduktes

Das gemäss a) hergestellte Mesylat wird mit dem Natriumsalz des 1,2,4-Triazols umgesetzt. Hierzu löst man 0,83 g Natrium in 30 ml Methanol, fügt 2,5 g 1,2,4-Triazol zu und entfernt das Lösungsmittel im Vakuum. Dem verbleibenden Natriumsalz wird eine Lösung von 8,55 g Mesylat in 30 ml Dimethylsulfoxid zugesetzt. Nach fünfstündigem Erhitzen auf einem Bad von 120° C wird das Gemisch auf Raumtemperatur abgekühlt und mit Diethylether extrahiert. Die vereinigten Extrakte werden mit Wasser gewaschen, über Natriumsulfat getrocknet und im Vakuum eingeengt. Ausbeute an Rohprodukt 7,6 g. Das Rohprodukt wird säulenchromatographisch (Kieselgel; Essigsäureethylester/Petroleumbenzin 1:4) gereinigt und führt zu einem farblosen Oel, das nach einigen Tagen kristallisiert. Nach Umkristallisation aus 16 ml Petrolether erhält man 3,7 g des Endprodukts. Smp. 67° C.
100 MHz-$^1$H-NMR ($CDCl_3$): $\delta$ = 7.63 und 7.72 ppm (s, 2H des Triazols); 7.0-7.4 ppm (m, 3H,aromatisch); 4.8 ppm (m, 2H, $-CH_2N$); 3.2-3.8 ppm (m, 2H, $-OCH_2CH_3$); 1.7-2.4 ppm (m, 2H, $-CH_2CH_2CH_3$); 1.3 ppm (t, 3H, $-OCH_2CH_3$); 0.7-1.4 ppm (m, 5H, $-CH_2CH_2CH_3$).

| | | berechnet | gefunden |
|---|---|---|---|
| Analyse [%] | C | 54,9 | 55,0 |
| | H | 5,8 | 5,9 |
| | N | 12,8 | 12,9 |
| | Cl | 21,6 | 21,8 |

Beispiel 3: Herstellung von

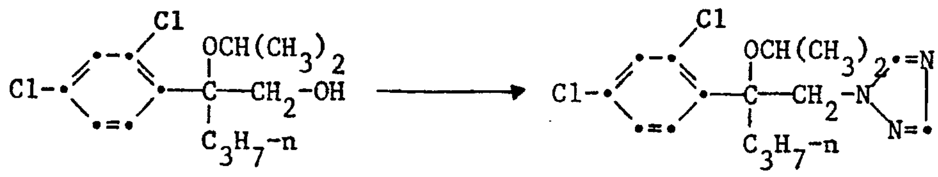

(3)

1-(1H-1,2,4-Triazol-1-yl)-2-isopropoxy-2-(2,4-dichlorphenyl)-pentan

a) Herstellung des Zwischenproduktes 1-(Methylsulfonyloxy)2-isopropoxy-2-(2,4-dichlorphenyl)-pentan

Zu einer Lösung von 8,0 g (27 m Mol) 2-(2,4-Dichlorphenyl)-2-isopropoxypentanol-1 und 0,1 g 4-Dimethylaminopyridin in 60 ml absolutem Pyridin lässt man unter Kühlung (Eisbad) 2,8 ml (35 mMol) Methansulfochlorid zutropfen, lässt das Gemisch 16 Stunden bei Raumtemperatur stehen, engt es ein und versetzt den Rückstand mit Diethylether. Die Diethyletherphase wird zweimal mit eiskalter, 2N-Salzsäure und zweimal mit halbgesättigter Natriumchloridlösung gewaschen, über Natriumsulfat getrocknet, filtriert und im Vakuum eingeengt. Zur Charakterisierung wird eine kleine Probe säulenchromatographisch (Kieselgel; Essigsäureethylester/Petroleumbenzin 1:4) gereinigt und das resultierende Oel durch Zusatz von Petroleumbenzin zur Kristallisations gebracht. Man erhält farblose Kristalle mit Smp. 58-61 °C.

| | | berechnet | gefunden |
|---|---|---|---|
| Analyse [%] | C | 48,8 | 48,9 |
| | H | 6,0 | 6,1 |
| | S | 8,7 | 9,1 |
| | Cl | 19,2 | 19,1 |

b) Herstellung des Endproduktes

Das gemäss a) hergestellte Mesylat wird in 100 ml aboslutem Dimethylsulfoxid gelöst und mit 3,2 g (35 mMol) 1,2,4-Triazol-Natriumsalz versetzt. Das Reaktionsgemisch wird 8 Stunden bei einer Badtemperatur von 120 °C gerührt, dann auf Raumtemperatur abgekühlt, auf Eiswasser gegossen und das Gemisch zweimal mit Essigsäureethylester extrahiert. Die vereinigten Extrakte werden viermal mit halbgesättigter Natriumchlorid-Lösung gewaschen, über Natriumsulfat getrocknet, filtriert und eingeengt. Das Rohprodukt wird säulenchromatographisch (Kieselgel; Essigsäureethylester/Petroleumbenzin 1:1) gereinigt und führt zu einem farblosen Oel. $n_D^{50}$ = 1.5299.

100 MHz-$^1$H-NMR ($CDCl_3$): $\delta$ = 7.75 und 7.65 ppm (s, 2H des Triazols); 7.1-7.6 ppm (m, 2H,aromatisch); 4.85 ppm (dd, 2H, $CH_2$-N); 4.0 ppm (m, 1H, $-CH(CH_3)_2$); 0.8-2.6 ppm (m, 14H, $-CH(CH_3)_2$, $-CH_2-CH_2-CH_3$);

| | | berechnet | gefunden |
|---|---|---|---|
| Analyse [%] | C | 56,2 | 55,9 |
| | H | 6,2 | 6,0 |
| | N | 12,3 | 12,0 |
| | Cl | 20,7 | 20,5 |

Beispiel 4: Herstellung von

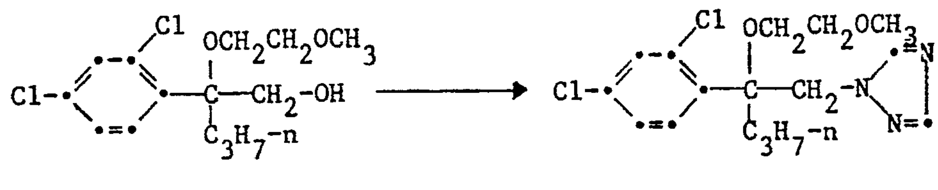

(4)

2-(1H-1,2,4-Triazol-1-yl)-2-(2,4-dichlorphenyl)-2-(2-methoxyethyoxy)-pentan

a) Herstellung des Zwischenproduktes 1-(Methylsulfonyloxy)-2-(2-methoxy-ethoxy)-2-(2,4-dichlorphenyl)-pentan

1,4 g (4,6 mMol) 2-(2,4-Dichlorphenyl)-2-(2-methoxyethoxy)-pentanol-1 und 50 mg 4-Dimethylaminopyridin werden in 10 ml Pyridin gelöst und bei Raumtemperatur mit 0,43 ml (5,5 mMol) Methansulfochlorid versetzt. Das Reaktionsgemisch bleibt 16 Stunden bei Raumtemperatur stehen und wird dann auf Eiswasser gegossen und zweimal mit Diethylether extrahiert. Die vereinigten Ether-Extrakte werden zweimal mit verdünnter, eiskalter Salzsäure und zweimal mit halbgesättigter Natriumchloridlösung gewaschen, über Natriumsulfat getrocknet, filtriert und das Filtrat eingeengt. Das Rohprodukt (1,7 g = 100% der Theorie), das als gelbliches, viskoses Oel anfällt, kann ohne Reinigung in der nächsten Stufe eingesetzt werden. Zur Charakterisierung wird eine kleine Probe säulenchromatographisch (Kieselgel; Essigsäureethylester/Petroleumbenzin 1:3, dann 1:2) gereinigt. Man erhält ein farbloses, viskoses Oel mit $n_D^{30}$ 1.5114. 60 MHz-$^1$H-NMR ($CDCl_3$): $\delta$ = 7.2-7.8 ppm (m, 3H, aromatisch); 4.75 ppm (dd, 2H, $-CH_2OSOCH_3$); 3.5-3.8 ppm (breites s, 4H, $-CH_2CH_2OCH_3$); 3.4 ppm (s, 3H, $-OCH_3$); 2.9 ppm (s, 3H, $-OSO_2CH_3$); 0.8-2.4 ppm (m, 7H,

$$-\overset{|}{\underset{|}{C}}-CH_2CH_2CH_3).$$

| | | berechnet | gefunden |
|---|---|---|---|
| Analyse [%] | C | 46,8 | 46,2 |
| | H | 5,8 | 5,7 |
| | S | 8,3 | 8,0 |
| | Cl | 18,4 | 19,2 |
| | O | 20,8 | 20,7 |

b) Herstellung des Endproduktes

12 g des nach a) hergestellten Rohproduktes (ausgehend von 30 mMol 2-(2,4-Dichlorphenyl)-2-(2-methoxyethoxy)-pentanol-1) werden in 50 ml absolutem Dimethylsulfoxid gelöst und mit 4 g (44 mMol) 1,2,4-Triazol-Natriumsalz versetzt. Das Gemisch wird 10 Stunden bei einer Badtemperatur von 120 °C gerührt, die entstehende dunkle Lösung auf Raumtemperatur abgekühlt, auf Eiswasser gegossen und das

Gemisch zweimal mit Essigsäureethylester extrahiert. Die vereinigten Extrakte werden mit halbgesättigter Natriumchlorid-Lösung gewaschen, über Natriumsulfat getrocknet, filtriert und eingeengt. Der Rückstand wird säulenchromatographisch (Kieselgel; Essigsäureethylester/Petroleumbenzin 1:2 bis 4:1) gereinigt und liefert 5,3 g (49,4% der Theorie über 2 Stufen) des Endproduktes in Form eines hellgelben Oels mit $n_D^{50}$ 1.5297.

60 MHz-$^1$H-NMR ($CDCl_3$): $\delta$ = 8.0 ppm (s, 1H, Triazol-H); 7.7 ppm (s, 1H, Triazol-H); 7.0-7.6 ppm (m, 2H, aromatisch); 4.85 ppm (dd, 2H,$CH_2$-Triazol); 3.6-4.0 ppm

$$(m, 4H, -\overset{|}{\underset{|}{C}}-O-\underline{CH_2}\underline{CH_2}-OCH_3);$$

3.5 ppm (s, 3H, -$OCH_3$); 0.7-2.7 ppm

$$(m, 7H, -\overset{|}{\underset{|}{C}}-\underline{CH_2}\underline{CH_2}\underline{CH_3}).$$

| | | berechnet | gefunden |
|---|---|---|---|
| Analyse [%] | C | 53,6 | 53,1 |
| | H | 5,9 | 5,8 |
| | N | 11,7 | 11,6 |
| | Cl | 19,8 | 20,1 |

Beispiel 5: Herstellung von

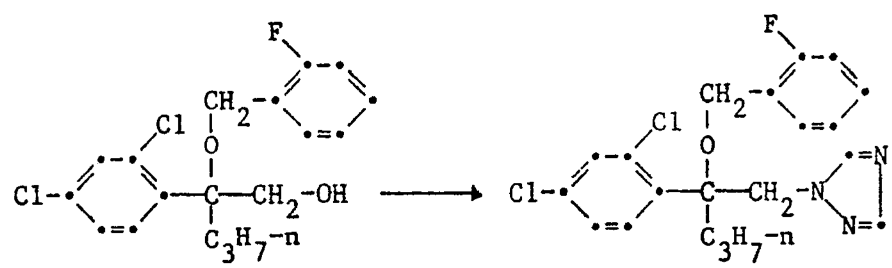
(5)

1-(1H-1,2,4-Triazol-1-yl)-2-(2-fluorbenzyloxy)-2-(2,4-dichlorphenyl)-pentan

a) Herstellung des Zwischenproduktes 1-(Methylsulfonyloxy)-2-(2-fluorbenzyloxy)-2-(2,4-dichlorphenyl)-pentan

6,5 g 2-(2,4-Dichlorphenyl)-2-(2-fluorbenzyloxy)-pentan-1 werden in 15 ml Pyridin gelöst und mit 2,5 g Methansulfochlorid versetzt. In einer exothermen Reaktion entsteht unter Abscheidung von Pyridinhydrochlorid das Mesylat, das analog zu Beispiel 4 durch Extraktion (mit Wasser und Methylenchlorid) isoliert wird. Ausbeute 8,16 g.

60 MHz-$^1$H-NMR ($CDCl_3$): $\delta$ = 7.1-7.7 ppm (m, 6H, aromatisch); 4.9 ppm (s, 2H, -$CH_2$-); 4.6 ppm (s, 2H, -$CH_2$-); 2.85 ppm (s, 3H, -$CH_3$); 2.0-2.5 ppm

$$(m, 2H, -\overset{|}{\underset{|}{C}}-\underline{CH_2}CH_2CH_3);$$

0.7-1.6 ppm

$(m,\ 5H,\ -\overset{|}{\underset{|}{C}}-CH_2C\underline{H}_2C\underline{H}_3)$.

b) Herstellung des Endproduktes

Durch Umsetzung des nach a) hergestellten Mesylats mit dem Natriumsalz des Triazols (hergestellt aus 0,6 g Natrium und 1,88 g 1,2,4-Triazol) erhält man nach einer zu Beispiel 4 b) analogen Arbeitsweise 2,4 g des rohen Endproduktes, das analog zu Beispiel 4 gereinigt wird und 1,3 g des reinen Endproduktes in Form eines zähflüssigen Oels liefert.
60 MHz-$^1$HNMR ($CDCl_3$): $\delta$ = 7.75 und 7.65 ppm (zwei s, 2H des Triazols); 7.5-7.6 ppm (m, 6H, aromatisch); 4.9 ppm (4 Linien, AB-Spektrum,

$-C\underline{H}_2$);

4.6 ppm (4 Linien, AB-Spektrum, $-C\underline{H}_2-$); 1.9-2.5 ppm

$(m,\ 2H,\ -\overset{|}{\underset{|}{C}}-C\underline{H}_2CH_2CH_3)$;

0.7-1,5 ppm

$(m,\ 5H,\ -\overset{|}{\underset{|}{C}}-CH_2C\underline{H}_2C\underline{H}_3)$.

Beispiel 6: Herstellung von

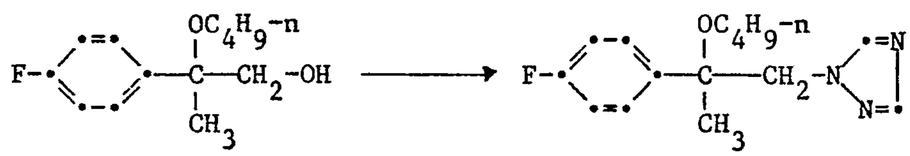

(6)

1-(1H-1,2,4-Triazol-1-yl)-2-(n-butoxy)-2-(4-fluorphenyl)-propan

a) Herstellung des Zwischenproduktes 1-(Methylsulfonyloxy)-2-(n-butoxy-2-(4-fluorphenyl)-propan

3,42 g (15 mMol) 2-(4-Fluorphenyl)-2-(n-butoxy)-propanol-1 werden in 10 ml Pyridin gelöst und mit 1,9 g (17 mMol) Methansulfochlorid versetzt. In einer exothermen Reaktion entsteht unter Abscheidung von Pyridinhydrochlorid das Mesylat. Man extrahiert letzteres mit Eiswasser und Chloroform, trocknet die organische Phase mit $Na_2SO_4$ und engt das Filtrat im Vakuum ein. Das Mesylat fällt als viskoses Oel an.
60 MHz-$^1$H-NMR ($CDCl_3$): $\delta$ = 6.8-7.5 ppm (m, 4H, aromatisch); 4,15 ppm (q, 2H, $-C\underline{H}_2OSO_2-$); 2.9 ppm (s, 3H, $-OSO_2C\underline{H}_3$).

b) Herstellung des Endproduktes

Das gemäss a) hergestellte Mesylat wird mit dem Natriumsalz des 1,2,4-Triazols umgesetzt. Hierzu löst man 0,52 g (23 mMol) Natrium in 20 ml absolutem Methanol und versetzt diese Lösung mit 1,55 g (23 mMol) 1,2,4-Triazol. Anschliessend wird das Methanol im Vakuum entfernt und zu dem kristallinen Rückstand das nach a) hergestellte in 20 ml Dimethylsulfoxid gelöste Mesylat zugefügt. Man erhitzt das Gemisch 6,5 Stunden bei einer Badtemperatur von 130 °C, lässt auf Raumtemperatur abkühlen, versetzt mit Wasser und extrahiert mit Chloroform. Die vereinigten Extrakte werden mit halbgesättigter Natriumchloridlösung gewaschen, über Natriumsulfat getrocknet, filtriert und das Filtrat im Vakuum eingeengt. Die 3,74 g des erhaltenen Rohproduktes werden säulenchromatographisch (Kieselgel, Essigsäureethylester/Petroleumbenzin 1:1) gereinigt und ergeben 1,57 g des reinen Endproduktes in Form eines Oels.

60 MHz-$^1$H-NMR ($CDCl_3$): = 8.10 und 7.85 ppm (2s, 2H des Triazols); 6,9-7.4 ppm (m, 4H, aromatisch); 4.3 ppm (s, 2H, $-CH_2N$); 2.9-3.4 ppm (m, 2H, $OCH_2$); 1.2-1.7 ppm

$$(m,\ 7H,\ -\overset{|}{\underset{|}{C}}-CH_3 \text{ und } OCH_2CH_2CH_2CH_3);$$

0.7-1.1 ppm (m, 3H, $OCH_2CH_2CH_2CH_3$).

| | | berechnet | gefunden |
|---|---|---|---|
| Analyse [%] | C | 65,0 | 65,3 |
| | H | 7,3 | 7,4 |
| | N | 15,2 | 15,0 |
| | F | 6,9 | 6,8 |

Beispiel 7: Herstellung von

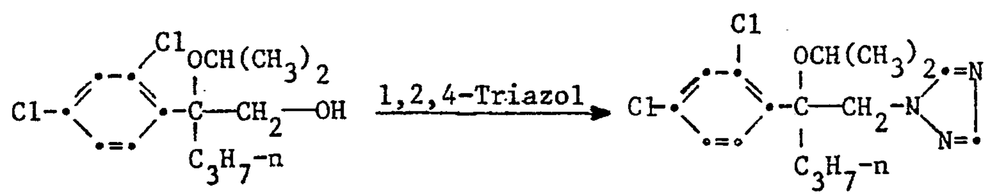

(3)

1-(1H-1,2,4-Triazol-1-yl)-2-isopropoxy-2,4-dichlorphenyl)-pentan

Zu einer Lösung von 5,8 g (20 mMol) 2-(2,4-Dichlorphenyl)-2-isopropoxy-pentanol-1, 7,9 g (30 mMol) Triphenylphosphin und 1,66 g (24 mMol) 1,2,4-Triazol in 100 ml absolutem Tetrahydrofuran wird bei Raumtemperatur eine Lösung von 5,5 g (30 mMol) Azodicarbonsäuredimethylester getropft und das Reaktionsgemisch anschliessend 3 Stunden bei Raumtemperatur, dann nochmals 16 Stunden bei 50° C gerührt, eingedampft, der Rückstand mit 100 ml Diethylether digeriert, der unlösliche Rückstand abfiltriert, die Ether-Phase erneut eingeengt und säulenchromatographisch (Kieselgel; Essigsäureethylester/Petroleumbenzin 1:1) gereinigt. Man erhält ein farbloses Oel, dessen physikalische Daten mit denen in Beispiel 3b) völlig übereinstimmen.

Beispiel 3 Herstellung von

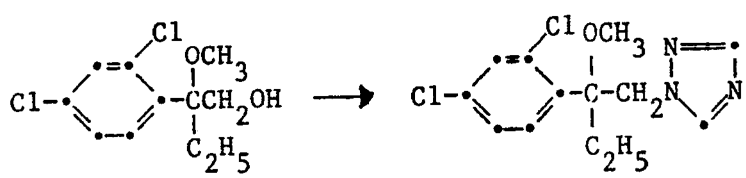

1-(1H-1,2,4-Triazol-1-yl)-2-methoxy-2-(2,4-dichlorphenyl)-butan.

a) Zwischenprodukt 1-(Methylsulfonyloxy)-2-methoxy-2-(2,4-dichlorphenyl)-butan.

Zu einer Lösung von 13,34 g (53 mMol) 2-(2,4-Dichlorphenyl)-2-methoxybutanol-1 und 5,95 g (59 mMol) Triethylamin in 70 ml Tetrahydrofuran werden unter Kühlung bei 20 - 28° C 6,75 g (59 mMol) Methansulfochlorid gelöst in 20 ml Tetrahydrofuran zugetropft, wobei sich sofort Triethylamin-Hydrochlorid abscheidet. Nach dem Absaugen des Salzes und der Entfernung des Lösungsmittels am Rotationsverdampfer wird das Rohprodukt in Chloroform gelöst und zweimal mit Wasser gewaschen. Nach dem Trocknen der organischen Phase mit $Na_2SO_4$ und dem Einengen am Rotationsverdampfer bei 60° C und 20 mbar erhält man 17,4 g farbloses Oel, das im Dünnschichtchromatogramm keine Verunreinigungen erkennen lässt. Brechungsindex $n_D^{50}$ 1.5247. 60 MHz-$^1$H-NMR ($DCCl_3$): $\delta$ = 7.2-7.7 ppm (m, 34, aromatisch); 4.5-5.1 ppm (m,2H,$OCH_2$); 3.3 ppm (s, 3H, $OCH_3$); 2,9 ppm (s, 3H, $OCH_3$), 1.5-2.4 ppm (m, 2H, $C\underline{H}_2CH_3$); 0.7 ppm (t, 3H, $CH_2C\underline{H}_3$).

| | | berechnet | gefunden |
|---|---|---|---|
| Analyse (%) | C | 44,1 | 44,6 |
| | H | 4,9 | 5,0 |
| | S | 9,8 | 9,7 |
| | Cl | 21,7 | 21,7 |

b) Endprodukt:

6,54 g (0,20 Mol) des nach a) hergestellten Mesylats werden mit dem Natriumsalz des Triazols (hergestellt aus 0,58 g Natrium und 1,73 g Triazol wie im Beispiel 2b beschrieben) durch 4-stündiges Erhitzen in 40 ml wasserfreiem Dimethylsulfoxyd umgesetzt. Das Reaktionsgemisch wird danach mit Essigester versetzt und dreimal mit Wasser extrahiert. Nach dem Trocknen der organischen Phase mit $Na_2SO_4$ und dem Entfernen des Lösungsmittels am Rotationsverdampfer erhält man 6,04 g Oel als Rohprodukt, das nach der Reinigung mittels einer Kieselgelsäule (Gemisch Essigester/Petrolether 1:1) 2,91 g (48,5%) Reinprodukt ergibt. 2,85 g dieses Produktes lassen sich bei 115-120° C und einem Druck von 0,03 Torr destillieren; man erhält 2,74 g farbloses Destillat. Brechungsindex $n_D^{50}$ = 1,5501.
60 MHz -$^1$H-NMR ($DCCl_3$): $\delta$ = 7.63 und 7.70 ppm (2s, 2H, Triazol), 7.0-7.5 ppm (m, 3H, aromatisch); 4.5-5.3 ppm (m, 2H, $CH_2N$); 3,35 ppm (s, 3H, $OCH_3$); 1.9-2.7 ppm (m,2H, $C\underline{H}_2CH_3$); 0.80 ppm (t, 3H, $CH_2C\underline{H}_3$).

| | | berechnet | gefunden |
|---|---|---|---|
| Analyse (%) | C | 52,02 | 52,31 |
| | H | 5,04 | 5,00 |
| | N | 14,00 | 13,95 |
| | Cl | 23,62 | 23,93 |

<u>Beispiel 9</u>: Herstellung von

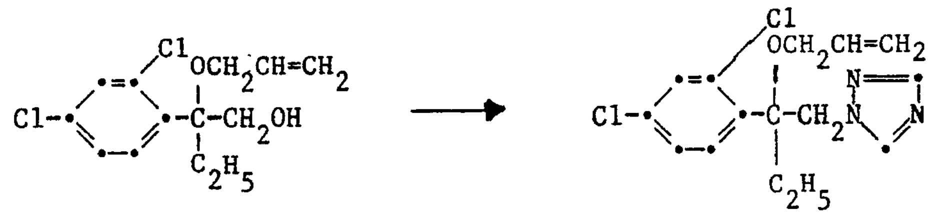

1-(1H-1,2,4-Triazol-1-yl)-2-allyloxy-2-(2,4-dichlorphenyl)-butan.

a) Zwischenprodukt 1-(Methylsulfonyloxy)-2-allyloxy-2-(2,4-dichlorphenyl)-butan.

Zu einer Lösung von 4,98 g (0,018 Mol) des Alkohols nach Beispiel VIII wird 2,01 g (0,02 Mol) Triethylamin in 25 ml Tetrahydrofuran lässt man bei Zimmertemperatur unter leichter Kühlung 2,28 g (0,02 Mol) Methansulfochlorid gelöst in 10 ml Tetrahydrofuran zutropfen. Nach dem Absaugen des abgeschiedenen Triethylamin-Hydrochlorids wird am Rotationsverdampfer eingeengt, und das so erhaltene Rohprodukt chromatographisch auf einer Kieselgelsäule gereinigt (Laufmittel Petrolether/Essigester 3:1). Die Entfernung des Lösungsmittels am Rotationsverdampfer bei 60° Badtemperatur und einem Druck von 20 mbar ergibt 5,70 g (89,2%) Reinprodukt als farbloses Oel. Brechungsindex $n_D^{50}$ 1,5224. 60 MHz- $^1$H-NMR ($DCCl_3$): $\delta$ = 7.2-7.7 ppm (m, 3H, aromatisch); 5.1-6.3 ppm (m, 3H, olefinisch); 4.8 ppm (s, 2H, C-$CH_2OSO_2$); 3.7-4.2 ppm (m, 2H, $OCH_2$); 2,9 ppm (s, 3H, $OSO_2CH_3$); 1.7-2,4 ppm (m, 2H, $\underline{CH_2}CH_3$); 0.75 ppm (t, 3H, $CH_2\underline{CH_3}$).

| | | berechnet | gefunden |
|---|---|---|---|
| Analyse (%) | C | 47,60 | 47,89 |
| | H | 5,14 | 5,20 |
| | S | 9,08 | 8,87 |
| | Cl | 20,07 | 19,88 |

b) Endprodukt:

5,32 g (0,0151 Mol) des nach a) hergestellten Mesylats werden mit dem Natriumsalz des Triazols (hergestellt aus 1,20 g Triazol und 0,40 g Natrium in Methanol wie im Beispiel 2 b beschrieben) durch 11-stündiges Rühren in 20 ml trockenem Dimethylsulfoxyd bei 120° C umgesetzt. Man versetzt mit 500 ml Wasser und extrahiert mit Chloroform. Nach dem Trocknen der organischen Phase mit $Na_2SO_4$ wird das Chloroform am Rotationsverdampfer entfernt und der Rückstand (4,58 g Oel) mittels einer Kieselgelsäule (Laufmittel Petrolether/Essigester 2:1 bis 1:1) gereinigt. Man entfernt das Lösungsmittel aus den Reinfraktionen am Rotationsverdampfer und erhält 3,31 g (67,2 %) Produkt als farbloses Oel. 60 MHz-$^1$H-NMR ($DCCl_3$): $\delta$ = 7.73 und 7.65 ppm (2s, 2H, Triazol); 6,9-7.4 ppm (m, 3H, aromatisch); 5.0-6.4 ppm (m, 3H olefinisch); 4.5-5.1 ppm (m, 2H, $NCH_2$); 3.6-4.4 ppm (m, 2H, $OCH_2$); 1.8-2.6 ppm (m, 2H, $\underline{CH_2}CH_3$); 0.8 ppm (t, 3H, $CH_2\underline{CH_3}$).

| | | berechnet | gefunden |
|---|---|---|---|
| Analyse (%) | C | 55,23 | 55,53 |
| | H | 5,25 | 5,48 |
| | N | 12,88 | 12,55 |
| | Cl | 21,74 | 21,16 |

Beispiel 10: Herstellung von

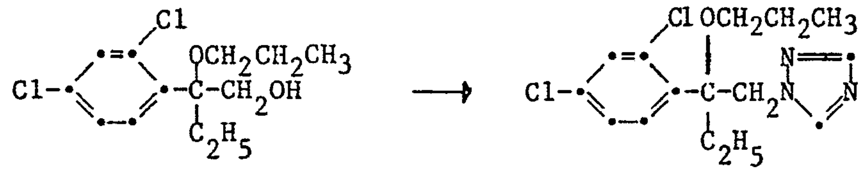

1-(1H-1,2,4-Triazol-1-yl)-2-propoxy-2-(2,4-dichlorphenyl)butan.

a) Zwischenprodukt 1-(Methylsulfonyloxy)-2-propoxy-2-(2,4-dichlorphenyl)-butan.

4,78 g (0,017 Mol) des Alkohols nach Beispiel IX werden in 50 ml THF mit 2,27 g (0,02 Mol) Methansulfochlorid in Gegenwart von 0,00 g (0,02 Mol) Triethylamin wie zuvor umgesetzt. Nach dem Absaugen des Triethylamin-Hydrochlorids wird eingeengt, und der Rückstand durch Säulenchromatographie (Kieselgel; Petrolether/Essigester = 4:1) gereinigt. Man erhält 5,64 g (92,3 %) farbloses Oel. 60 MHz-$^1$H-NMR($DCCl_3$): δ = 7.1-7.8 ppm (m,3H, aromat.); 4.5-4.9 ppm (m,2H,$CH_2OS$); 3.2-3.5 ppm (m, 2H, $OCH_2$); 2.8 ppm (s, 3H, $OSO_2CH_3$); 1.6-2.4 ppm (m; 4H, 2x$CH_2$); 0.6-1.3 ppm (m, 6H, 2x$CH_3$).

b) Endprodukt:

0,45 g (0,02 g-Atom) Natrium werden in 20 ml Methanol gelöst. Danach fügt man 1,35 g (0,02 Mol) Triazol zu und engt die Lösung zur Trockene ein. Durch zweimaliges Einengen mit Toluol am Rotationsverdampfer werden Methanolreste vollständig entfernt. Das so erhaltene Natriumsalz des Triazols wird in 40 ml wasserfreiem Dimethylsulfoxyl mit 5,35 g (0,016 Mol) des nach a) hergestellten Mesylats 5 Stunden auf 120° C erhitzt. Die Aufarbeitung durch Extraktion mit Wasser und Chloroform, gefolgt von einer chromatographischen Reinigung mittels einer Kieselgel-Säule, ergibt 2,93 g (54,8 %)farbloses, bei Raumtemperatur erstarrendes Oel. Durch Umkristallisation aus Petrolether erhält man 2,43 g (45,4 %) farblose Kristalle von Smp. 74-75° C. 60 MHz- H-NMR($DCCl_3$): δ = 7.72 und 7.62 ppm (2S, 2H, Triazol); 7.0-7.5 ppm (m, 3H, aromatisch); 4.5-5.1 ppm (m, 2H, $CH_2N$); 3.1-3.7 ppm (m, 2H, $OCH_2$); 1.4-2.8 ppm (m, 4H, 2x$CH_3$);0.6-1.2 ppm (m, 6H, 2x$CH_3$).

Beispiel 11: Herstellung von

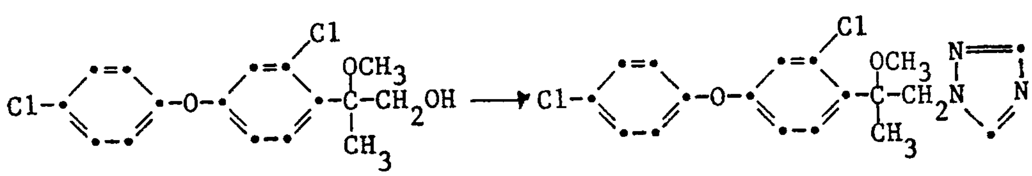

1-(1H-1,2,4-Triazol-1-yl)-2-methoxy-2-[2-chlor-4-(4-chlorphenoxy)-phenyl]-propan.

a) Zwischenprodukt 1-(Methylsulfonyloxy)-2-methoxy-2-[2-chlor-4-(4-chlorphenoxy)-phenyl]-propan

9,37 g (0,029 Mol) des Alkohols nach Beispiel X werden in Gegenwart von 3,33 g (0,033 Mol) Triethylamin mit 3,77g (0,033 Mol) Methansulfochlorid in 100 ml Tetrahydrofuran umgesetzt. Nach dem Absaugen des Triethylamin-Hydrochlorids wird das Filtrat am Rotationsverdampfer zu 12,4 g öligem Rohprodukt eingeengt. 7,91 g werden mittels Säulenchromatographie(Kieselgel; 2 Teile Petrolether, 1Teil Essigester) gereinigt und ergeben 6,89 g (93,1 %) reines Mesylat als farbloses Oel. 60 MHz-$^1$H-NMR ($DCCl_3$): δ = 6.8-7.6 ppm (m, 7H, aromatisch); 4.3-4.7 ppm (m, 2H, $CH_2OS$); 3.23 ppm (s, 3H, $OCH_3$); 2.96 ppm (s, 3H, $OSO_2CH_3$); 1.78 ppm (s, 3H, C-$CH_3$).

| | | berechnet | gefunden |
|---|---|---|---|
| Analyse (%) | C | 50,38 | 50,58 |
| | H | 4,48 | 4,72 |
| | S | 7,91 | 7,65 |
| | Cl | 17,50 | 17,11 |

b) Endprodukt

Aus 0,46 g Natrium und 1,37 g Triazol wird in Methanol wie zuvor beschrieben, das Natriumsalz des Triazols hergestellt. Man fügt eine Lösung von 6,69 g (0,0165 Mol) des nach a) hergestellten Mesylats in 50 ml wasserfreiem Dimethylsulfoxyd zu und rührt 9 Stunden bei 120 °C. Die Extraktion des Reaktionsgemisches mit Chloroform und Wasser ergibt nach der Entfernung des Lösungsmittels 6,87 g Rohprodukt, das durch Säulenchromatographie (Kieselgel; 1 Teil Petrolether/1 Teil Essigester) gereinigt wird, und 3,23 g (51,8 %) Reinprodukt als farbloses Oel liefert. 60 MHz-$^1$H-NMR ($DCCl_3$): $\delta$ = 8.00 und 7.82 ppm (2S, 2H, Triazol); 6.7-7.5 ppm (m, 7H, aromatisch); 4.6 ppm (s, 2H, $CH_2N$); 3.2 ppm (s, 3H, $OCH_3$); 1.7 ppm (s, 3H, $C\text{-}CH_3$).

Wie beschrieben erhält man auch die nachfolgend angeführten Substanzen der Formel I

Tabelle: Verbindungen der Formel

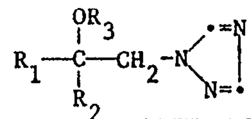

(I)

| Verb. Nr. | $R_1$ | $R_2$ | $R_3$ | Physik.Konst. [°C] [%] Analysen |
|---|---|---|---|---|
| 1 | $C_6H_3Cl_2(2,4)$ | $C_3H_7$-n | $CH_3$ | Sdp. 170-175°/0,04 mbar |
| 2 | $C_6H_3Cl_2(2,4)$ | $C_3H_7$-n | $C_2H_5$ | Smp. 67°C |
| 3 | $C_6H_3Cl_2(2,4)$ | $C_3H_7$n | $CH(CH_3)_2$ | $n_D^{50}$ 1.5299 |
| 4 | $C_6H_3Cl_2(2,4)$ | $C_3H_7$-n | $CH_2CH_2OCH_3$ | $n_D^{50}$ 1.5297 |
| 5 | $C_6H_3Cl_2(2,4)$ | $C_3H_7$-n | $CH_2-C_6H_4F(2)$ | vgl.Herstellungsbeisp. 5 |
| 6 | $C_6H_4F(4)$ | $CH_3$ | $C_4H_9$-n | berechnet gefunden<br>C 65,0 65,3<br>H 7,3 7,4<br>N 15,2 15,0<br>F 6,9 6,8 |
| 7 | $C_6H_4F(4)$ | $C_2H_5$ | $C_4H_9$-n | berechnet gefunden<br>C 66,0 66,2<br>H 7.6 7,5<br>N 14.4 14,5<br>F 6,5 6,1 |
| 8 | $C_6H_4F(4)$ | $C_2H_5$ | $CH_2CH$=$CHCH_3$ | berechnet gefunden<br>C 66,4 65,5<br>H 6,9 6,9<br>N 14,5 14.8<br>F 6,6 6,6 |
| 9 | $C_6H_4Cl(4)$ | $C_3H_7$-n | $CH_2C_6H_5$ | Sdp. 170-176°/0,02 mbar |

Tabelle: (Fortsetzung)

| Verb. Nr. | $R_1$ | $R_2$ | $R_3$ | Physik.Konst. [°C] Analysen |
|---|---|---|---|---|
| 10 | $C_6H_3Cl_2$(2,4) | $CH_3$ | $CH_3$ | berechnet gefunden<br>C 50,4 50,7<br>H 4,6 4,9<br>N 14,6 14,0 |
| 11 | $C_6H_3Cl_2$(2,4) | $CH_3$ | $C_3H_7$-n | Smp. 62-64° |
| 12 | $C_6H_3Cl_2$(2,4) | $CH_3$ | $C_4H_9$-n | Sdp. 190-200°/0,02 mbar |
| 13 | $C_6H_3$(2,4) | $CH_3$ | $CH_2CH=CH_2$ | Smp. 68-70° |
| 14 | $C_6H_3Cl_2$(2,4) | $CH_3$ | $CH-C(CH_3)=CH_2$ | Sdp. 165-175°/0,02 mbar |
| 15 | $C_6H_3Cl_2$(2,4) | $CH_3$ | $CH_2-[C_6H_5Cl(4)]$ | berechnet gefunden<br>C 54,5 54,6<br>H 4,1 4,2<br>N 10,6 10,5 |
| 16 | $C_6H_3Cl_2$(2,4) | $C_2H_5$ | $CH_3$ | Sdp. 170-175°/0,o4 mbar |
| 17 | $C_6H_3Cl_2$(2,4) | $C_2H_5$ | $C_3H_7$-n | Sdp. 192-198°/0,03 mbar |
| 18 | $C_6H_3Cl_2$(2,4) | $C_2H_5$ | $CH_2CH=CH_2$ | berechnet gefunden<br>C 55,2 55,0<br>H 5.2 5,2<br>N 12,9 13,0<br>Cl 21,7 21,7 |

Tabelle: (Fortsetzung)

| Verb. Nr. | $R_1$ | $R_2$ | $R_3$ | Physik.Konst. [°C] Analysen |
|---|---|---|---|---|
| 19 | $C_6H_3Cl_2$(2,4) | $C_3H_7$-n | $C_3H_7$-n | 60 MHz-$^1$HNR ($CDCl_3$): $\delta$= 7.8 u. 7.7 ppm (2s, 2H des Triazols); 6.9-6.5 ppm (m. 3H, aromatisch); 4.8 ppm (4 Linien-AB-Spektru,, 2H,$CH_2N$); 3.1-3.7 ppm (m. 2H, $OCH_2$); 1.7-2.6 ppm (m, 12H. $OCH_2CH_2CH_2CH_3$ und $OCCH_2CH_2CH_3$). |
| 20 | $C_6H_3Cl_2$(2,4) | $C_3H_7$-n | $C_4H_9$-n | Sdp. 189-195°/0,02 mbar |
| 21 | $C_6H_3Cl_2$(2,4) | $C_3H_7$-n | $CH_2CH=CH_2$ | Sdp. 200-210/0,02 mbar |
| 22 | $C_6H_3Cl$(2,4) | $C_3H_7$-n | $CH_2CH=CHCH_3$ | Sdp. 180-190°/0,02 mbar |
| 23 | $C_6H_3Cl_2$(2,4) | $C_3H_7$-n | $CH_2-C_6H_5$ | berechnet gefunden<br>C 61,6 61.6<br>H 5,6 5,7<br>N 10,8 10,5<br>Cl 18,2 18,0 |
| 24 | $C_6H_3Cl_2$(2,4) | $CH(CH_3)_2$ | $CH_3$ | Sdp. 158-162°/0,03 mbar |
| 25 | $C_6H_3Cl_2$(2.4) | $C_5H_{11}$-n | $CH_3$ | Sdp. 255°/0,04 mbar |
| 26 | $C_6H_3Cl_2$(2,4) | Cyclohexyl | $CH_3$ | Sdp. 200-210°/0,1 mbar |
| 27 | $C_6H_3Br(4)Cl(2)$ | $CH_3$ | $CH_3$ | Sdp. 230°/0,05 mbar |

Tabelle: (Fortsetzung)

| Verb. Nr. | $R_1$ | $R_2$ | $R_3$ | Physik.Konst. [°C] Analysen |
|---|---|---|---|---|
| 28 | $C_6H_3Br(4)Cl(2)$ | $C_3H_7$-n | $CH_3$ | |
| 29 | $C_6H_3Cl(2)F(4)$ | $C_3H_7$-n | $CH_3$ | Sdp. 190°/0,04 mbar |
| 30 | 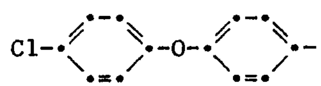 | $CH_3$ | $CH_3$ | $n_D^{50}$ 1.5653 |
| 31 | 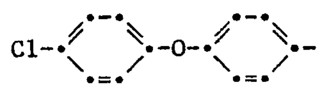 | $C_2H_5$ | $CH_3$ | berechnet gefunden<br>C 63,8 64,3<br>H 5,6 5,7<br>N 11,7 11,5<br>Cl 9,9 9,6 |
| 32 | $C_6H_3F_2(2,4)$ | $C_3H_7$-n | $CH_3$ | Sdp.164-170°/0,025 mbar |
| 33 | $C_6H_3Cl_2(2,4)$ | $C_4H_9$-n | $CH_3$ | Sdp. 245-250°/0,05 mbar |

Tabelle: (Fortsetzung)

| Verb. Nr. | $R_1$ | $R_2$ | $R_3$ | Physik.Konst. [°C] Analysen |
|---|---|---|---|---|
| 34 | $C_6H_3Cl(2)F(4)$ | $C_2H_5$ | $CH_3$ | Oel |
| 35 | $C_6H_3Cl(2)F(4)$ | $C_2H_5$ | $CH_2CH=CH_2$ | Oel |
| 36 | 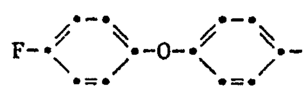 | $C_2H_5$ | $CH_3$ | Smp. 58-60° |
| 37 | 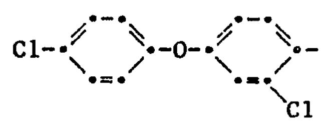 | $C_2H_5$ | $CH_3$ | $n_D^{50}$ 1.5689 |

Tabelle: (Fortsetzung)

| Verb. Nr. | $R_1$ | $R_2$ | $R_3$ | Physik.Konst. [°C] Analysen |
|---|---|---|---|---|
| 38 | $C_6H_5F_4$ | $C_6H_5F(4)$ | $CH_3$ | Smp. 98-100° |
| 39 | $C_6H_3Cl_2(2,4)$ | $C_6H_5$ | $CH_3$ | Smp. 128-131° |
| 40 | $C_6H_3Cl_2(2,4)$ | $C_6H_5$ | $CH_2-C_6H_5$ | Smp. 118-119° |
| 41 | $C_6H_4Cl(4)$ | $C_6H_5$ | $CH_3$ | Smp. 126-127° |
| 42 | $C_6H_4Cl(4)$ | $C_6H_5$ | $CH_2-C_6H_5$ | Smp. 116-119° |
| 43 | $C_6H_3Cl_2(2,4)$ | $C_6H_5$ | $CH_2CH=CH_2$ | Smp. 130-132° |
| 44 | $C_6H_4Cl(4)$ | Cyclohexyl | $CH_3$ | Smp. 118-119° |
| 45 | $C_6H_4Cl(4)$ | Cyclohexyl | $CH_2-C_6H_5$ | Smp. 116-118° |
| 46 | $C_6H_4F(4)$ | Cyclohexyl | $CH_3$ | Smp. 100-101° |
| 47 | $C_6H_5$ | Cyclopropyl | $CH_3$ | Oel |
| 48 | $C_6H_4F(4)$ | Cyclohexyl | $CH_2C_6H_5$ | Smp. 74-75° |
| 49 | $C_6H_3Cl_2(2,4)$ | $C_3H_7$-n | $CH_2-C_6H_5$ | Oel |
| 50 | $C_6H_4Cl(4)$ | $C_3H_7$-n | $CH_3$ | Sdp. 245-250°/0,5mbar |
| 51 | $C_6H_3Cl(2)F(4)$ | $C_3H_7$-n | $CH_2C_6H_5$ | Oel |
| 52 | $C_6H_4Cl(4)$ | $C_4H_9$-n | $CH_3$ | Sdp. 185-195°/0,01mbar |
| 53 | $C_6H_5$ | $(CH_3)_3C$ | $CH_3$ | Sdp. 150-160°/0,1mbar |
| 54 | $C_6H_5$ | $CH(CH_3)_2$ | $CH_3$ | Sdp. 250°/0,04 mbar |
| 55 | $C_6H_5$ | $CH(CH_3)_2$ | $CH_2-C_6H_5$ | Sdp. 250°/0,04 mbar |
| 56 | $C_6H_4CH_3(4)$ | $C_4H_9$-n | $CH_3$ | Sdp. 170-180°/0,5mbar |

Tabelle: (Fortsetzung)

| Verb. Nr. | $R_1$ | $R_2$ | $R_3$ | Physik.Konst. [°C] |
|---|---|---|---|---|
| 57 | $C_6H_5$ | Cyclopentyl | $CH_3$ | Smp. 91-92° |
| 58 | $C_6H_5$ | Cyclohexyl | $CH_3$ | Sdp. 190-200°/0,01mbar |
| 59 | $C_6H_5CH_2$ | $C_6H_5Cl(4)$ | $CH_3$ | Smp. 88-90° |
| 60 | $C_6H_3Cl_2(2,4)$ | Cyclohexyl | $CH_3$ | Sdp. 200-210°/0,mbar |
| 61 | $C_6H_3Cl(2)F(4)$ | $CH_3$ | $CH_2C_6H_5$ | Sdp. 250°/0,04mbar |
| 62 | $C_6H_3Cl(2)Br(4)$ | $CH_3$ | $CH_2C_6H_5$ | Sdp. 219°/0,02 mbar |
| 63 | $C_6H_3Cl_2(2,4)$ | $CH_3$ | $CH_2CH=CHCH_3$ | Sdp. 190-200°/0,02mbar |
| 64 | $C_6H_3Cl_2(2,4)$ | $C_3H_7$-n | $CH_2-\underset{CH_3}{C}=CH_2$ | Sdp. 170-180°/0,02mbar |
| 65 | $C_6H_3Cl_2(2,4)$ | $C_3H_7$-n | $CH_2CH=CHCH_3$ | Sdp. 180-190°/0,o2 mbar |
| 66 | $C_6H_3Cl(2)F(4)$ | $C_3H_7$-n | $CH_2-CH=CH_2$ | Sdp. 210°/0,04 mbar |
| 67 | $C_6H_3Cl(2)F(4)$ | $C_3H_7$-n | $C_3H_7$-n | Sdp. 208°/,0,04 mbar |
| 68 | $C_6H_3Cl_2(2,4)$ | $C_2H_5$ | $C_3H_7$-n | Sdp. 192-175°/0,03mbar |
| 69 | $C_6H_3Cl_2(2,4)$ | $C_3H_7$-n | $CH_2CH(CH_3)CH_3$ | Viskoses Oel |
| 70 | $C_6H_3Cl_2(2,4)$ | $C_3H_7$-n | $CH(CH_3)_2$ | $n_D^{50}$ 1.5299 |
| 71 | $C_6H_3Cl(2)Br(4)$ | $C_3H_7$-n | $CH_2C_6H_5$ | Oel |
| 72 | $C_6H_3Cl(2)Br(4)$ | $C_3H_7$-n | $CH_2CH=CH-CH_3$ | Sdp.195-198°/0,01mbar |
| 73 | $C_6H_3Cl(2)Br(4)$ | $C_3H_7$-n | $CH_3$ | Sdp.178-185°/0,02mbar |

Tabelle: (Fortsetzung)

| Verb. Nr. | $R_1$ | $R_2$ | $R_3$ | Physik.Konst. [°C] Analysen |
|---|---|---|---|---|
| 74 | $C_6H_3Cl(2)Br(4)$ | $C_3H_7$-n | $C_4H_9$-n | Sdp. 167-174°/0,01 mbar |
| 75 | $C_6H_4(OC_6H_5)(4)$ | $C_2H_5$ | $CH_3$ | Oel |
| 76 | $C_6H_4(OC_6H_5(4)$ | $C_2H_5$ | $CH_2CH=CH_2$ | Oel |
| 77 | $C_6H_4(OC_6H_5)(4)$ | $C_2H_5$ | $C_3H_7$-n | Oel |
| 78 | $C_6H_3Cl(2)F(4)$ | $CH_3$ | $CH_3$ | Sdp. 197-206°/0,016 mbar |
| 79 | $C_6H_5$ | $C_3H_7$-n | $CH_3$ | Oel |
| 80 | $C_6H_5$ | $C_3H_7$-n | $CH_2C_6H_5$ | Oel |
| 81 | $C_6H_5$ | $C_4H_9$-n | $CH_3$ | Oel |
| 82 | $C_6H_5$ | $C_6H_5$ | $CH_2CH=CH_2$ | Oel |
| 83 | $C_6H_4F(4)$ | Cyclobutyl | $CH_3$ | Oel |
| 84 | $C_6H_4Cl(4)$ | $C_{11}H_{23}$-n | $CH_3$ | Oel |
| 85 | $C_6H_4Cl(4)$ | $C_{11}H_{23}$-n | $CH_2C_6H_5$ | Sdp. 250/0.04 mbar |
| 86 | $C_6H_3Cl_2(2,4)$ | $CH_3$ | $CH_3$ | Oel |
| 87 | $C_6H_3Cl_2(2,4)$ | $C_3H_7$-n | $C_3H_7$-n | Oel |
| 88 | $C_6H_3Cl_2(2,4)$ | $CH_3$ | $C_4H_9$-n | Oel |
| 89 | $C_6H_3Cl_2(2,4)$ | $C_3H_7$-n | $CH_2CH=CH_2$ | Oel |
| 90 | $C_6H_3Cl_2(2,4)$ | $CH_3$ | $CH_2C_6H_5$ | Oel |

<u>Tabelle:</u> (Fortsetzung)

| Verb. Nr. | $R_1$ | $R_2$ | $R_3$ | Physik.Konst. [°C] Analysen |
|---|---|---|---|---|
| 91 | $C_6H_3Cl_2(2,4)$ | $CH_3$ | $CH_2C_6H_4Cl(4)$ | Oel |
| 92 | $C_6H_3Cl_2(2,4)$ | $C_3H_7$-n | $CH_2C_6H_5$ | Oel |
| 93 | $C_6H_5-CH_2$ | $C_3H_7$-n | $CH_3$ | Oel |
| 94 | $C_6H_5-CH_2$ | $C_3H_7$-n | $CH_2C_6H_5$ | Oel |
| 95 | $C_6H_3Cl(2)Br(4)$ | $CH_3$ | $CH_3$ | Oel |
| 96 | $F(2)C_6H_3-CH_2$ | $C_4H_9$-t | $CH_3$ | Oel |
| 97 | $C_6H_4Cl(4)$ | $C_6H_5$ | $CH_2{\equiv}CH$ | Oel |
| 98 | $C_6H_3Cl_2(2,4)$ | $C_3H_7$-n | $C_3H_7$-n | Oel |
| 99 | $C_6H_4F(4)$ | $C_2H_5$ | $C_4H_9$-n | Oel |
| 100 | $C_6H_4F(4)$ | $C_2H_5$ | $CH_2CH=CHCH_3$ | Oel |
| 101 | $C_6H_3Cl_2(2,4)$ | $C_2H_5$ | $CH_2CH=CH_2$ | viskoses Oel |
| 102 | $C_6H_4F(4)$ | $C_2H_5$ | $CH_3$ | viskoses Oel |

Tabelle: (Fortsetzung)

| Verb. Nr. | $R_1$ | $R_2$ | $R_3$ | Physik.Konst. [°C] Analysen |
|---|---|---|---|---|
| 103 | $C_6H_3Cl(2)F(4)$ | $C_2H_5$ | $C_3H_7$-n | $n_D^{49}$ 1.5125 |
| 104 | $C_6H_3Br(4)Cl(2)$ | $C_2H_5$ | $C_4H_9$-n | Smp. 72-73° |
| 105 | $C_6H_3Cl_2(2,4)$ | $C_2H_5$ | $CH_2-C(CH_3)=CH_2$ | Oel |
| 106 | 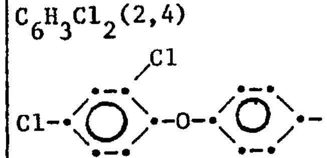 | $C_2H_5$ | $CH_3$ | Oel |
| 107 | 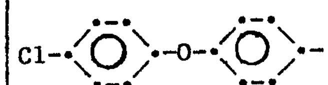 | $C_3H_7$-i | $CH_3$ | Smp. 142-143° |
| 108 | 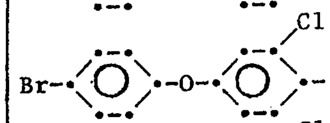 | $C_2H_5$ | $CH_3$ | Oel |
| 109 | 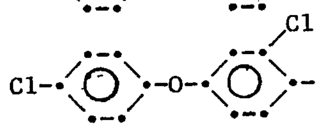 | $C_3H_7$-n | $CH_2CH=CH_2$ | Smp. 89-90° |

**Tabelle: (Fortsetzung)**

| Verb. Nr. | $R_1$ | $R_2$ | $R_3$ | Physik.Konst. [°C] Analysen |
|---|---|---|---|---|
| 110 | 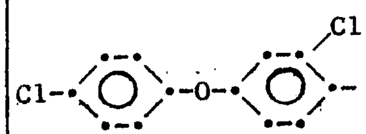 | $C_3H_7$-n | $CH_3$ | $n_D^{50}$ 1.5595 |
| 111 | 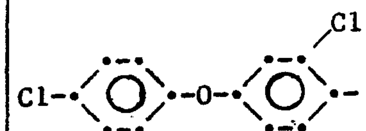 | $C_2H_5$ | $CH_2CH{=}CH_2$ | $n_D^{50}$ 1.5662 |
| 112 | 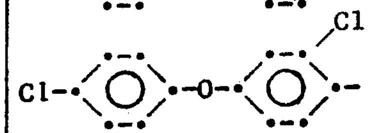 | $C_2H_5$ | $CH_2-C(CH_3){=}CH_2$ | $n_D^{50}$ 1.5610 |
| 113 | 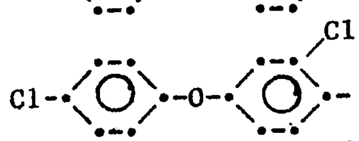 | $CH_3$ | $CH_2-CH_2{=}CH_2$ | $n_D^{50}$ 1.5689 |
| 114 | 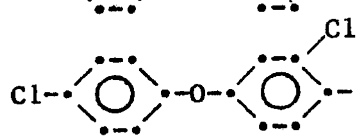 | $CH_3$ | $CH_3$ | $n_D^{50}$ 1.5765 |

Tabelle: (Fortsetzung)

| Verb. Nr. | $R_1$ | $R_2$ | $R_3$ | Physik.Konst. [°C] Analysen |
|---|---|---|---|---|
| 115 | 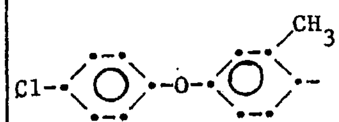 | $CH_3$ | $CH_3$ | $n_D^{50}$ 1.5738 |
| 116 | 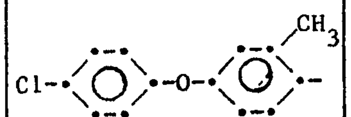 | $CH_3$ | $CH_2-CH=CH_2$ | $n_D^{50}$ 1.5695 |
| 117 | 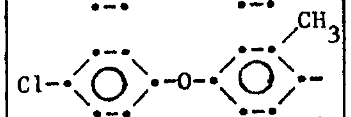 | $C_2H_5$ | $CH_3$ | Smp.91-92° |
| 118 | 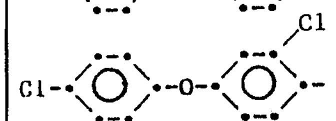 | $C_2H_5$ | $CH_2C_3H_7$-i | $n_D^{50}$ 1.5503 |
| 119 | 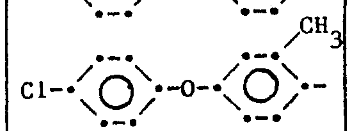 | $C_3H_7$-n | $CH_2-CH=CH_2$ | $n_D^{50}$ 1.5594 |

Tabelle: (Fortsetzung)

| Verb. Nr. | $R_1$ | $R_2$ | $R_3$ | Physik.Konst. [°C] Analysen |
|---|---|---|---|---|
| 120 | 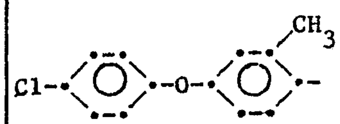 | $C_2H_5$ | $CH_3$ | $n_D^{50}$ 1.5601 |
| 121 | 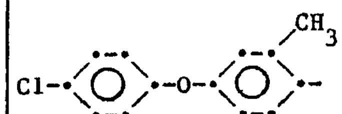 | $C_2H_5$ | $CH_2-CH=CH_2$ | $n_D^{50}$ 1.5570 |
| 122 | 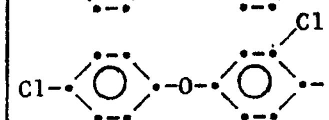 | $C_3H_7$-n | $C_3H_7$-n | $n_D^{50}$ 1.5540 |
| 123 | 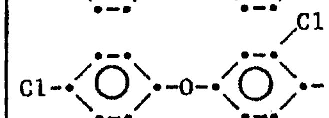 | $C_2H_5$ | $C_3H_7$-n | $n_D^{50}$ 1.5518 |
| 124 | 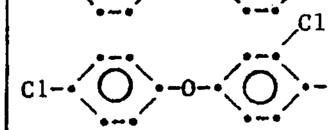 | $CH_3$ | $C_3H_7$-n | $n_D^{50}$ 1.5595 |
| 125 | 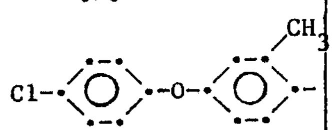 | $CH_3$ | $C_3H_7$-n | Smp. 97-98° |

Formulierungsbeispiele für Wirkstoffe der Formel Ia (%=Gewichtsprozent)

| Emulsions-Konzentrate /Spritzpulver | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden. Spritzpulver liegen vor, wenn der Xylol-Anteil durch Kieselsäure und/oder Kaolin ersetzt wird.

| Lösungen | a) | b) | c) | d) |
|---|---|---|---|---|
| Wirkstoff aus Tabelle 1 | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| Granulate | a) | b) |
|---|---|---|
| Wirkstoff aus Tabelle 1 | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| Stäubemittel | a) | b) |
| --- | --- | --- |
| Wirkstoff aus der Tabelle I | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

Biologische Beispiele:

Beispiel 2.1:Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,002%Aktivsubstanz) besprüht. Nach 24 Stunden wurden die behandelten Pflanzen mit einer Ureidosporensuspension des Pilzes infiziert. Nach einer Inkubation während 48 Stunden bei 95-100% relativer Luftfeuchtigkeit und ca. 20° C wurden die infizierten Pflanzen in einem Gewächshaus bei ca. 22° C aufgestellt. Die Beurteilung der Rostpustelnentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen (0,0006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20° C wurden die infizierten Pflanzen in einem Gewächshaus bei ca. 22° C aufgestellt. Die Beurteilung der Rostpustelnentwicklung erfolgte 12 Tage nach der Infektion.

Verbindungen aus der Tabelle I zeigten gegen Puccinia-Pilze eine gute Wirkung. Unbehandelte aber infizierte Kontrollpflanzen zeigten einen Puccinia-Befall von 100%. Unter anderen hemmten die Verbindungen Nr. 30, 31, 36, 37 sowie 103 bis 115 den Puccinia-Befall auf 0 bis 5%.

Beispiel 2.2: Wirkung gegen Cercospora arachidicola auf Erdnusspflanzen

Residual-protektive Wirkung

10-15 cm hohe Erdnusspflanzen wurden mit einer aus Spritzpulver der Wirksubstanz hergestellten Spritzbrühe (0,006%Aktivsubstanz) besprüht und 48 Stunden später mit einer Konidiensuspension des Pilzes infiziert. Die infizierten Pflanzen wurden während 72 Stunden bei ca. 21° C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus der Tabelle I behandelt wurden, einen stark reduzierten Cercospora-Befall. So verhinderten die Verbindungen Nr. 30, 31, 36, 37 sowie 103 bis 115 in obigem Versuch das Auftreten von Flecken fast vollständig (0 bis 10%).

Beispiel 2.3:Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,002% Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22° C

aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,0006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22° C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen zeigten einen Erysiphe-Befall von 100%. Unter anderen Verbindungen aus der Tabelle I hemmten Verbindungen Nr. 30, 31, 36, 37, 103 bis 115 den Pilzbefall auf Gerste auf 0 bis 5%. Besonders wirksam (kein Befall) waren die Verbindungen Nr. 32, 36 und 37.

Beispiel 2.4: Residual-protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0.006% Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24° C aufgestellt. Der Schorfbefall wurde 15 Tage nach der Infektion beurteilt. Kontrollpflanzen waren 100%ig befallen. Verbindungen Nr. 30, 31, 36, 37, 103 bis 115 hemmten den Krankheitsbefall auf weniger als 10%. Bei Behandlung mit den Wirkstoffen Nr. 32, 36, 37, 109, 111, 114, 115 und 120 trat überhaupt kein Befall auf.

Beispiel 2.5: Wirkung gegen Botrytis cinerea auf Aepfeln

Residual protektive Wirkung

Künstlich verletzte Aepfel wurden behandelt, indem eine aus Spritzpulver der Wirksubstanz hergestellte Spritzbrühe (0,02% Aktivsubstanz) auf die Verletzungsstellen aufgetropft wurde. Die behandelten Früchte wurden anschliessend mit einer Sporensuspension von Botrytis cinerea inokuliert und während einer Woche bei hoher Luftfeuchtigkeit und ca. 20° C inkubiert.

Das Vorhandensein und die Grösse der Fäulnis-Stellen an der Frucht dienten zur Bewertung der fungiziden Aktivität. Bei Behandlung mit den Verbindungen Nr. 30, 31, 36, 37, 103 bis 115, wurden keine oder fast keine Fäulnis-Stellen (0-5% Befall) beobachtet.

**Patentansprüche**

**Patentansprüche für folgende Vertragsstaaten: BE, CH, DE, FR, IT, LI, LU, NL, SE**

**1.** Verfahren zur Herstellung von 1-Triazolylethylether-Derivaten der allgemeinen Formel I

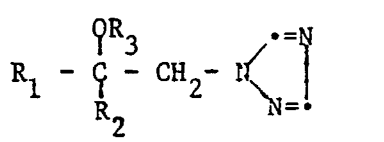

(I) ,

worin

$R_1$ für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cycloalkyl, unsubstituiertes oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Phenoxy, Halophenoxy, Phenyl, Benzyl, Halobenzyl, Nitro und/oder Cyano substituiertes Phenyl oder unsubstituiertes oder ein- bis dreifach durch Halogen, $C_1$-$C_6$-Haloalkyl, $C_1$-$C_6$-Alkoxy, $C_1$-$C_6$-Alkyl, Nitro und/oder Cyano substituiertes Benzyl steht;

$R_2$ für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cycloalkyl, unsubstituiertes oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Phenoxy, Halophenoxy, Phenyl, Benzyl, Halobenzyl, Nitro und/oder Cyano substituiertes Phenyl oder unsubstituiertes oder ein- bis dreifach durch Halogen, $C_1$-$C_6$-Haloalkyl, $C_1$-

$C_6$-Alkoxy, $C_1$-$C_6$-Alkyl, Nitro und/oder Cyano substituiertes Benzyl steht; und $R_3$ unsubstituiertes oder durch $C_1$-$C_3$-Alkoxy substituiertes $C_1$-$C_6$-Alkyl, $C_3$-$C_4$-Alkenyl, Benzyl oder Halobenzyl bedeutet, dadurch gekennzeichnet, dass man

a) ein Oxiran der Formel II

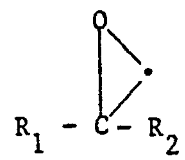

(II)

bei Temperaturen von -20° bis +100° C in Gegenwart eines sauren Katalysators bzw. eines sauren Kondensationsmittels mit einem Alkohol der Formel III

$R_3$ - OH (III)

zu einem Glykolmonoether der Formel IV

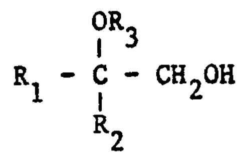

(IV)

reagieren lässt, und

b) anschliessend den Glykolmonoether der Formel IV oder einen seiner Ester in Gegenwart eines säurebindenden bzw. Kondensationsmittels bei Temperaturen von 0° bis 150° C mit einem Triazol der Formel V

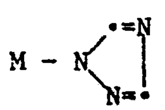

(V)

umsetzt, wobei die Substituenten $R_1$, $R_2$ und $R_3$ in den Formeln II bis IV wie unter Formel I definiert sind und M in Formel V für Wasserstoff oder ein Metallatom steht.

2. Verfahren nach Anspruch 1, dadurch gekennzeichnet, dass man die Reaktion des Oxirans der Formel II mit dem Alkohol der Formel III bei Temperaturen zwischen 0° und 40° C durchführt.

3. Verfahren nach Anspruch 1, dadurch gekennzeichnet, dass man als sauren Katalysator bzw. als saures Kondensationsmittel eine Protonensäure, eine Lewis-Säure oder einen Ionenaustauscher in der $H^+$-Form einsetzt.

4. Verfahren nach Anspruch 1, dadurch gekennzeichnet, dass man die Umsetzung des Glykolmonoethers der Formel IV mit dem Triazol der Formel V bei Temperaturen zwischen 20° und 100° C durchführt.

5. Verfahren nach Anspruch 1, dadurch gekennzeichnet, dass man vor der Umsetzung von IV mit V die freie Hydroxylgruppe in IV verestert.

6. Verfahren nach Anspruch 1, dadurch gekennzeichnet, dass man als Verfahrensprodukt eine Verbindung der Formel I herstellt, worin
$R_1$ und $R_2$ unabhängig voneinander für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cylcloalkyl, Phenyl, ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Phenyl, durch Phenoxy, Halophenoxy, Phenyl, Halophenyl, Benzyl oder Halobenzyl substituiertes Phenyl, Benzyl oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro

und/oder Cyano substituiertes Benzyl stehen; und $R_3$ $C_1$-$C_6$-Alkyl, durch $C_1$-$C_3$-Alkoxy substituiertes $C_1$-$C_6$-Alkyl, $C_3$-$C_4$-Alkenyl, Benzyl oder Halogenbenzyl bedeutet.

**7.** Verfahren nach Anspruch 1, dadurch gekennzeichnet, dass man als Verfahrensprodukt eine Verbindung der Formel I herstellt, worin $R_1$ und $R_2$ unabhängig voneinander für $C_1$-$C_6$-Alkyl, durch $C_1$-$C_3$-Alkoxy oder $C_3$-$C_6$-Cycloalkyl substituiertes $C_1$-$C_2$-Alkyl, $C_3$-$C_6$-Cycloalkyl, Phenyl, ein- bis dreifach durch Halogen, $C_1$-$C_3$-Haloalkyl, $C_1$-$C_3$-Alkoxy, $C_1$-$C_3$-Alkyl, Nitro und/oder Cyano substituiertes Phenyl, durch Phenoxy, Halophenoxy, Phenyl, Benzyl oder Halobenzyl substituiertes Phenyl, Benzyl oder ein- bis dreifach durch Halogen, $C_1$-$C_3$-Haloalkyl, Methyl, Methoxy, Nitro und/oder Cyano substituiertes Benzyl stehen; und $R_3$ $C_1$-$C_4$-Alkyl, durch $C_1$-$C_3$-Alkoxy substituiertes $C_1$-$C_2$-Alkyl, $C_3$-$C_4$-Alkenyl, Benzyl oder Halobenzyl bedeutet.

**8.** Verfahren nach Anspruch 7, dadurch gekennzeichnet, dass man als Verfahrensprodukt eine Verbindung der Formel I herstellt, worin $R_1$ für Phenyl, ein- bis dreifach durch Halogen oder einfach durch Phenoxy oder Halophenoxy substituiertes Phenyl steht; $R_2$ $C_1$-$C_6$-Alkyl oder $C_3$-$C_7$-Cycloalkyl bedeutet und $R_3$ für $C_1$-$C_6$-Alkyl, durch $C_1$-$C_3$-Alkoxy substituiertes $C_1$-$C_6$-Alkyl, $C_3$-$C_4$-Alkenyl, Benzyl oder Halobenzyl steht.

**9.** Verbindungen der Formel IV'

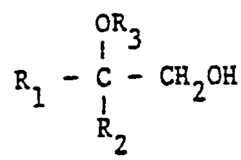

worin $R_1$ für ein durch $C_1$-$C_6$-Alkoxy oder $C_3$-$C_6$-Cycloalkyl substituiertes $C_1$-$C_6$-Alkyl, $C_3$-$C_8$-Cycloalkyl, Phenyl, ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Phenyl, durch Phenoxy, Halophenoxy, Phenyl, Halophenyl, Benzyl oder Halobenzyl substituiertes Phenyl, Benzyl oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Benzyl steht; $R_2$ für $C_1$-$C_{12}$-Alkyl oder einen unter $R_1$ angegebenen Rest steht; und $R_3$ $C_1$-$C_6$-Alkyl, durch $C_1$-$C_3$-Alkoxy substituierten $C_1$-$C_6$-Alkyl, $C_3$-$C_4$-Alkenyl, Benzyl oder Halobenzyl bedeutet.

**10.** Verbindungen der Formel IV' nach Anspruch 9, dadurch gekennzeichnet, dass $R_1$ Phenyl, ein- bis dreifach durch Halogen oder einfach durch Phenoxy oder Halophenoxy substituiertes Phenyl bedeutet; $R_2$ für $C_1$-$C_6$-Alkyl oder $C_3$-$C_7$-Cycloalkyl steht und $R_3$ für $C_1$-$C_6$-Alkyl, durch $C_1$-$C_3$-Alkoxy substituiertes $C_1$-$C_6$-Alkyl, $C_3$-$C_4$-Alkenyl, Benzyl oder Halobenzyl steht.

**11.** Verfahren zur Herstellung der in Anspruch 9 definierten Verbindungen der Formel IV', dadurch gekennzeichnet, dass man ein Oxiran der Formel II

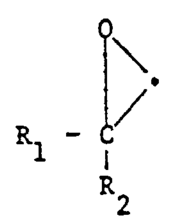

(II)

bei Temperaturen von -20° bis +100°C in Gegenwart eines sauren Katalysators bzw. eines sauren Kondensationsmittels mit einem Alkohol der Formel III

$R_3$ - OH (III)

reagieren lässt, wobei $R_1$, $R_2$ und $R_3$ in den Formeln II und III die unter Formel IV' angegebenen Bedeutungen haben.

**12.** Verbindungen der Formel Ia

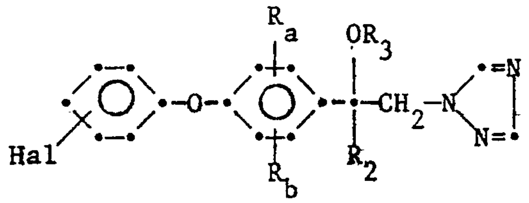

(Ia)

worin Hal für Halogen steht;
$R_a$ und $R_b$ unabhängig voneinander für Wasserstoff, Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano stehen;
$R_2$ für $C_1$-$C_{12}$-Alkyl, durch- $C_1$-$C_6$-Alkoxy oder $C_3$-$C_8$-Cycloalkyl substituiertes $C_1$-$C_6$ Alkyl, $C_3$-$C_8$-Cycloalkyl, unsubstituiertes oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Phenoxy, Halophenoxy, Phenyl, Benzyl, Halobenzyl, Nitro und/oder Cyano substituiertes Pehnyl oder unsubstituiertes oder ein- bis dreifach durch Halogen, $C_1$-$C_6$-Haloalkyl, $C_1$-$C_6$-Alkoxy, $C_1$-$C_6$-Alkyl, Nitro und/oder Cyano substituiertes Benzyl steht; und
$R_3$ unsubstituiertes oder durch $C_1$-$C_3$-Alkoxy substituiertes $C_1$-$C_6$-Alkyl, $C_3$-$C_4$-Alkenyl, Benzyl oder Halobenzyl bedeutet.

**13.** Verbindungen der Formel Ia nach Anspruch 12, dadurch gekennzeichnet, dass Hal für Fluor, Chlor und/oder Brom steht; $R_a$ und $R_b$ unabhängig voneinander für Wasserstoff, Halogen, $C_1$-$C_3$-Haloalkyl, $C_1$-$C_3$-Haloalkoxy, $C_1$-$C_3$-Alkoxy, $C_1$-$C_3$-Alkyl, Nitro und/oder Cyano stehen; $R_2$ für $C_1$-$C_6$-Alkyl steht; und $R_3$ unsubstituiertes oder durch $C_1$-$C_3$-Alkoxy substituiertes $C_1$-$C_6$-Alkyl, $C_3$-$C_4$-Alkenyl, Benzyl oder Halobenzyl bedeutet.

**14.** Eine Verbindung der Formel Ia nach Anspruch 12, ausgewählt aus der Reihe:
1-(1H-1,2,4-Triazol-1-yl)-2-methoxy-2-[4-(4-chlorphenoxy-phenyl]-butan;
1-(1H-1,2,4-Triazol-1-yl)-2-methoxy-2-[4-(4-fluorphenoxy)-phenyl]-butan;
1-(1H-1,2,4-Triazol-1-yl)-2-methoxy-2-[4-(2,4-dichlorphenoxy)-phenyl]-butan;
1-(1H-1,2,4-Triazol-1-yl)-2-methoxy-2-[4-(4-chlorphenoxy)-phenyl]-propan;
1-(1H-1,2,4-Triazol-1-yl)-2-methoxy-2-[4-(4-chlorphenoxy)-2-chlorphenyl]-butan;
1-(1H-1,2,4-Triazol-1-yl)-2-methoxy-2-[4-(4-chlorphenoxy)-phenyl]-3-methyl-butan;
1-(1H-1,2,4-Triazol-1-yl)-2-allyloxy-2-[4-(4-chlorphenoxy)-2-chlorphenyl]-pentan;
1-(1H-1,2,4-Triazol-1-yl)-2-methoxy-2-[4-(4-chlorphenoxy)-2-chlorphenyl]-pentan;
1-(1H-1,2,4-Triazol-1-yl)-2-allyloxy-2-[4-(4-chlorphenoxy)-2-chlorphenyl]-butan;
1-(1H-1,2,4-Triazol-1-yl)-2-(2-methylallyloxy)-2-[4-(4-chlorphenoxy)-2-chlorphenyl]-butan;
1-(1H-1,2,4-Triazol-1-yl)-2-allyloxy-2-[4-(4-chlorphenoxy)-2-chlorphenyl]-propan;
1-(1H-1,2,4-Triazol-1-yl)-2-methoxy-2-[4-(4-chlorphenoxy)-2-chlorphenyl]-propan;
1-(1H-1,2,4-Triazol-1-yl)-2-methoxy-2-[4-(4-chlorphenoxy)-2-methylphenyl]-propan;
1-(1H-1,2,4-Triazol-1-yl)-2-allyloxy-2-[4-(4-chlorphenoxy)-2-methylphenyl]-propan;
1-(1H-1,2,4-Triazol-1-yl)-2-methoxy-2-[4-(4-chlorphenoxy)-2-methylphenyl]-butan;
1-(1H-1,2,4-Triazol-1-yl)-2-(2-methylpropoxy)-[4-(4-chlorphenoxy)-2-chlorphenyl]-butan;
1-(1H-1,2,4-Triazol-1-yl)-2-allyloxy-2-[4-(4-chlorphenoxy)-2-methylphenyl]-pentan;
1-(1H-1,2,4-Triazol-1-yl)-2-methoxy-2-[4-(4-chlorphenoxy)-2-methylphenyl]-butan;
1-(1H-1,2,4-Triazol-1-yl)-2-allyloxy-2-[4-(4-chlorphenoxy)-2-methylphenyl]-butan;
1-(1H-1,2,4-Triazol-1-yl)-2-propoxy-2-[4-(4-chlorphenoxy)-2-chlorphenyl]-pentan;
1-(1H-1,2,4-Triazol-1-yl)-2-n-propoxy-2-[4-(4-chlorphenoxy)-2-chlorphenyl]-butan;
1-(1H-1,2,4-Triazol-1-yl)-2-n-propoxy-2-[4-(4-chlorphenoxy)-2-chlorphenyl]-propan;
1-(1H-1,2,4-Triazol-1-yl)-2-n-propoxy-2-[4-(4-chlorphenoxy)-2-methylphenyl]-propan.

**15.** Mikrobizides Mittel, dadurch gekennzeichnet, dass es 0,1 bis 99 % einer Verbindung der Formel Ia nach Anspruch 12, 99,9 bis 1 % fester oder flüssiger Zuschlagsstoffe und 0 bis 25 % eines Tensides enthält.

**16.** Verfahren zur Bekämpfung phytopathogener Mikroorganismen, dadurch gekennzeichnet, dass man 10

g bis 5 kg einer Verbindung der Formel Ia nach Anspruch 12 je Hektar Anbaufläche appliziert.

**Patentansprüche für folgende Vertragsstaat: AT**

1. Verfahren zur Herstellung von 1-Triazolylethylether-Derivaten der allgemeinen Formel I

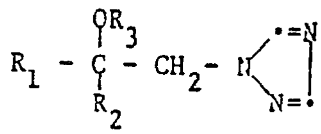

(I) ,

worin
$R_1$ für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cycloalkyl, unsubstituiertes oder ein- bis bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Phenoxy, Halophenoxy, Phenyl, Benzyl, Halobenzyl, Nitro und/oder Cyano substituiertes Phenyl oder unsubstituiertes oder ein- bis dreifach durch Halogen, $C_1$-$C_6$-Haloalkyl, $C_1$-$C_6$-Alkoxy, $C_1$-$C_6$-Alkyl, Nitro und/oder Cyano substituiertes Benzyl steht;
$R_2$ für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cycloalkyl, unsubstituiertes oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Phenoxy, Halophenoxy, Phenyl, Benzyl, Halobenzyl, Nitro und/oder Cyano substituiertes Phenyl oder unsubstituiertes oder ein- bis dreifach durch Halogen, $C_1$-$C_6$-Haloalkyl, $C_1$-$C_6$-Alkoxy, $C_1$-$C_6$-Alkyl, Nitro und/oder Cyano substituiertes Benzyl steht;
und $R_3$ unsubstituiertes oder durch $C_1$-$C_3$-Alkoxy substituiertes $C_1$-$C_6$-Alkyl, $C_3$-$C_4$-Alkenyl, Benzyl oder Halobenzyl bedeutet, dadurch gekennzeichnet, dass man

a) ein Oxiran der Formel II

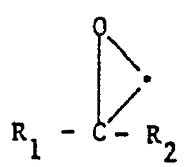

(II)

bei Temperaturen von -20° bis +100° C in Gegenwart eines sauren Katalysators bzw. eines sauren Kondensationsmittels mit einem Alkohol der Formel III

$R_3$ - OH (III)

zu einem Glykolmonoether der Formel IV reagieren lässt, und

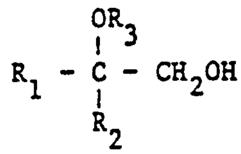

(IV)

b) anschliessend den Glykolmonoether der Formel IV oder einen seiner Ester in Gegenwart eines säurebindenden bzw. Kondensationsmittels bei Temperaturen von 0° bis 150° C mit einem Triazol der Formel V

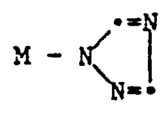

(V)

umsetzt, wobei die Substituenten $R_1$, $R_2$ und $R_3$ in den Formeln II bis IV wie unter Formel I definiert

sind und M in Formel V für Wasserstoff oder ein Metallatom steht.

2. Verfahren nach Anspruch 1, dadurch gekennzeichnet, dass man die Reaktion des Oxirans der Formel II mit dem Alkohol der Formel III bei Temperaturen zwischen 0° und 40° C durchführt.

3. Verfahren nach Anspruch 1, dadurch gekennzeichnet, dass man als sauren Katalysator bzw. als saures Kondensationsmittel eine Protonensäure, eine Lewis-Säure oder einen Ionenaustauscher in der $H^+$-Form einsetzt.

4. Verfahren nach Anspruch 1, dadurch gekennzeichnet, dass man die Umsetzung des Glykolmonoethers der Formel IV mit dem Triazol der Formel V bei Temperaturen zwischen 20° und 100° C durchführt.

5. Verfahren nach Anspruch 1, dadurch gekennzeichnet, dass man vor der Umsetzung von IV mit V die freie Hydroxylgruppe in IV verestert.

6. Verfahren nach Anspruch 1, dadurch gekennzeichnet, dass man als Verfahrensprodukt eine Verbindung der Formel I herstellt, worin
$R_1$ und $R_2$ unabhängig voneinander für $C_1$-$C_{12}$-Alkyl, durch $C_1$-$C_6$-Alkoxy oder $C_3$-$C_8$-Cycloalkyl substituiertes $C_1$-$C_6$-Alkyl, $C_3$-$C_8$-Cycloalkyl, Phenyl, ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Phenyl, durch Phenoxy, Halophenoxy, Phenyl, Halophenyl, Benzyl oder Halobenzyl substituiertes Phenyl, Benzyl oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Benzyl stehen; und $R_3$ $C_1$-$C_6$-Alkyl, durch $C_1$-$C_3$-Alkoxy substituiertes $C_1$-$C_6$-Alkyl, $C_3$-$C_4$-Alkenyl, Benzyl oder Halogenbenzyl bedeutet.

7. Verfahren nach Anspruch 1, dadurch gekennzeichnet, dass man als Verfahrensprodukt eine Verbindung der Formel I herstellt, worin $R_1$ und $R_2$ unabhängig voneinander für $C_1$-$C_6$-Alkyl, durch $C_1$-$C_3$-Alkoxy oder $C_3$-$C_6$-Cycloalkyl substituiertes $C_1$-$C_2$-Alkyl, $C_3$-$C_6$-Cycloalkyl, Phenyl, ein- bis dreifach durch Halogen, $C_1$-$C_3$-Haloalkyl, $C_1$-$C_3$-Alkoxy, $C_1$-$C_3$-Alkyl, Nitro und/oder Cyano substituiertes Phenyl, durch Phenoxy, Halophenoxy, Phenyl, Benzyl oder Halobenzyl substituiertes Phenyl, Benzyl oder ein- bis dreifach durch Halogen, $C_1$-$C_3$-Haloalkyl, Methyl, Methoxy, Nitro und/oder Cyano substituiertes Benzyl stehen; und $R_3$ $C_1$-$C_4$-Alkyl, durch $C_1$-$C_3$-Alkoxy substituiertes $C_1$-$C_2$-Alkyl, $C_3$-$C_4$-Alkenyl, Benzyl oder Halobenzyl bedeutet.

8. Verfahren nach Anspruch 7, dadurch gekennzeichnet, dass man als Verfahrensprodukt eine Verbindung der Formel I herstellt, worin $R_1$ für Phenyl, ein- bis dreifach durch Halogen oder einfach durch Phenoxy oder Halophenoxy substituiertes Phenyl steht; $R_2$ $C_1$-$C_6$-Alkyl oder $C_3$-$C_7$-Cycloalkyl bedeutet und $R_3$ für $C_1$-$C_6$-Alkyl, durch $C_1$-$C_3$-Alkoxy substituiertes $C_1$-$C_6$-Alkyl, $C_3$-$C_4$-Alkenyl, Benzyl oder Halobenzyl steht.

9. Verfahren zur Herstellung von Verbindungen der Formel IV'

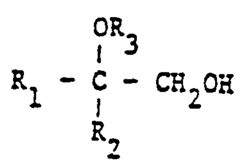

worin $R_1$ für ein durch $C_1$-$C_6$-Alkoxy oder $C_3$-$C_6$-Cycloalkyl substituiertes $C_1$-$C_6$-Alkyl, $C_3$-$C_8$-Cycloalkyl, Phenyl, ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Phenyl, durch Phenoxy, Halophenoxy, Phenyl, Halophenyl, Benzyl oder Halobenzyl substituiertes Phenyl, Benzyl oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano substituiertes Benzyl steht; $R_2$ für $C_1$-$C_{12}$-Alkyl oder einen unter $R_1$ angegebenen Rest steht; und $R_3$ $C_1$-$C_6$-Alkyl, durch $C_1$-$C_6$-Alkoxy substituierten $C_1$-$C_6$-Alkyl, $C_3$-$C_4$-Alkenyl, Benzyl oder Halobenzyl bedeutet,
dadurch gekennzeichnet, dass man ein Oxiran der Formel II

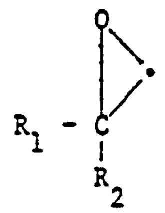

(II)

bei Temperaturen von -20° bis +100°C in Gegenwart eines sauren Katalysators bzw. eines sauren Kondensationsmittels mit einem Alkohol der Formel III

$R_3$ - OH (III)

reagieren lässt, wobei $R_1$, $R_2$ und $R_3$ in den Formeln II und III die unter Formel IV' angegebenen Bedeutungen haben.

**10.** Verfahren gemäss Anspruch 9 zur Herstellung von Verbindungen der Formel IV', worin $R_1$ Phenyl, ein- bis dreifach durch Halogen oder einfach durch Phenoxy oder Halophenoxy substituiertes Phenyl bedeutet; $R_2$ für $C_1$-$C_6$-Alkyl oder $C_3$-$C_7$-Cycloalkyl steht und $R_3$ für $C_1$-$C_6$-Alkyl, durch $C_1$-$C_3$-Alkoxy substituiertes $C_1$-$C_6$-Alkyl, $C_3$-$C_4$-Alkenyl, Benzyl oder Halobenzyl steht.

**11.** Mikrobizides Mittel, dadurch gekennzeichnet, dass es neben 99,9 bis 1% festem oder flüssigem Zuschlagstoff und 0 bis 25% eines Tensids als Wirkstoff 0,1 bis 99% einer Verbindung der Formel Ia enthält,

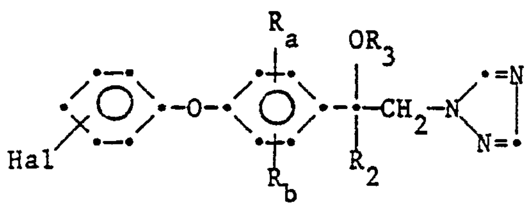

(Ia)

worin Hal für Halogen steht;
$R_a$ und $R_b$ unabhängig voneinander für Wasserstoff, Halogen, $C_1$-$C_6$-Haloalkyl, $C_1$-$C_6$-Haloalkoxy, $C_1$-$C_6$-Alkoxy, $C_1$-$C_6$-Alkyl, Nitro und/oder Cyano stehen;
$R_2$ für $C_1$-$C_{12}$-Alkyl, durch $C_1$-$C_6$-Alkoxy oder $C_3$-$C_8$-Cycloalkyl substituiertes $C_1$-$C_6$ Alkyl, $C_3$-$C_8$-Cycloalkyl, unsubstituiertes oder ein- bis dreifach durch Halogen, $C_1$-$C_6$-Haloalkyl, $C_1$-$C_6$-Haloalkoxy, $C_1$-$C_6$-Alkoxy, $C_1$-$C_6$-Alkyl, Phenoxy, Halophenoxy, Phenyl, Benzyl, Halobenzyl, Nitro und/oder Cyano substituiertes Pehnyl oder unsubstituiertes oder ein- bis dreifach durch Halogen, $C_1$-$C_6$-Haloalkyl, $C_1$-$C_6$-Alkoxy, $C_1$-$C_6$-Alkyl, Nitro und/oder Cyano substituiertes Benzyl steht; und
$R_3$ unsubstituiertes oder durch $C_1$-$C_3$-Alkoxy substituiertes $C_1$-$C_6$-Alkyl, $C_3$-$C_4$-Alkenyl, Benzyl oder Halobenzyl bedeutet.

**12.** Mittel gemäss Anspruch 11 dadurch gekennzeichnet, dass Hal für Fluor, Chlor und/oder Brom steht; $R_a$ und $R_b$ unabhängig voneinander für Wasserstoff, Halogen, $C_1$-$C_3$- Haloalkyl, $C_1$-$C_3$-Haloalkoxy, $C_1$-$C_3$-Alkoxy, $C_1$-$C_3$-Alkyl, Nitro und/oder Cyano stehen; $R_2$ für $C_1$-$C_6$-Alkyl steht; und $R_3$ unsubstituiertes oder durch $C_1$-$C_3$-Alkoxy substituiertes $C_1$-$C_6$-Alkyl, $C_3$-$C_4$-Alkenyl, Benzyl oder Halobenzyl bedeutet.

**13.** Mittel gemäss Anspruch 11, dadurch gekennzeichnet, dass es als Wirkstoff eine Verbindung der Formel Ia enthält, ausgewählt aus der Reihe:
1-(1H-1,2,4-Triazol-1-yl)-2-methoxy-2-[4-(4-chlorphenoxy-phenyl]-butan;
1-(1H-1,2,4-Triazol-1-yl)-2-methoxy-2-[4-(4-fluorphenoxy)-phenyl]-butan;
1-(1H-1,2,4-Triazol-1-yl)-2-methoxy-2-[4-(4-(2,4-dichlorphenoxy)-phenyl]-butan;
1-(1H-1,2,4-Triazol-1-yl)-2-methoxy-2-[4-(4-chlorphenoxy)-phenyl]-propan;
1-(1H-1,2,4-Triazol-1-yl)-2-methoxy-2-[4-(4-chlorphenoxy)-2-chlorphenyl]-butan;
1-(1H-1,2,4-Triazol-1-yl)-2-methoxy-2-[4-(4-chlorphenoxy)-phenyl]-3-methyl-butan;

1-(1H-1,2,4-Triazol-1-yl)-2-allyloxy-2-[4-(4-chlorphenoxy)-2-chlorphenyl]-pentan;
1-(1H-1,2,4-Triazol-1-yl)-2-methoxy-2-[4-(4-chlorphenoxy)-2-chlorphenyl]-pentan;
1-(1H-1,2,4-Triazol-1-yl)-2-allyloxy-2-[4-(4-chlorphenoxy)-2-chlorphenyl]-butan;
1-(1H-1,2,4-Triazol-1-yl)-2-(2-methylallyloxy)-2-[4-(4-chlorphenoxy)-2-chlorphenyl]-butan;
1-(1H-1,2,4-Triazol-1-yl)-2-allyloxy-2-[4-(4-chlorphenoxy)-2-chlorphenyl)-propan;
1-(1H-1,2,4-Triazol-1-yl)-2-methoxy-2-[4-(4-chlorphenoxy)-2-chlorphenyl]-propan;
1-(1H-1,2,4-Triazol-1-yl)-2-methoxy-2-[4-(4-chlorphenoxy)-2-methylphenyl]-propan;
1-(1H-1,2,4-Triazol-1-yl)-2-allyloxy-2-[4-(4-chlorphenoxy)-2-methylphenyl]-propan;
1-(1H-1,2,4-Triazol-1-yl)-2-methoxy-2-[4-(4-chlorphenoxy)-2-methylphenyl]-butan;
1-(1H-1,2,4-Triazol-1-yl)-2-(2-methylpropoxy)-[4-(4-chlorphenoxy)-2-chlorphenyl]-butan;
1-(1H-1,2,4-Triazol-1-yl)-2-allyloxy-2-[4-(4-chlorphenoxy)-2-methylphenyl]-pentan;
1-(1H-1,2,4-Triazol-1-yl)-2-methoxy-2-[4-(4-chlorphenoxy)-2-methylphenyl]-butan;
1-(1H-1,2,4-Triazol-1-yl)-2-allyloxy-2-[4-(4-chlorphenoxy)-2-methylphenyl]-butan;
1-(1H-1,2,4-Triazol-1-yl)-2-propoxy-2-[4-(4-chlorphenoxy)-2-chlorphenyl]-pentan;
1-(1H-1,2,4-Triazol-1-yl)-2-n-propoxy-2-[4-(4-chlorphenoxy)-2-chlorphenyl]-butan;
1-(1H-1,2,4-Triazol-1-yl)-2-n-propoxy-2-[4-(4-chlorphenoxy)-2-chlorphenyl]-propan;
1-(1H-1,2,4-Triazol-1-yl)-2-n-propoxy-2-[4-(4-chlorphenoxy)-2-methylphenyl]-propan.

14. Verfahren zur Bekämpfung phytopathogener Mikroorganismen, dadurch gekennzeichnet, dass man 10 g bis 5 kg einer Verbindung der Formel Ia nach Anspruch 11 je Hektar Anbaufläche appliziert.

**Claims**
**Claims for the following Contracting States: BE CH DE FR IT LI LU NL SE**

1. A process for the preparation of a 1-triazolylethyl ether derivative of the general formula I

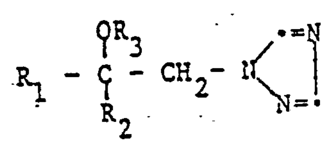

(I),

in which
$R_1$ is $C_1$-$C_{12}$alkyl, $C_1$-$C_6$alkyl which is substituted by $C_1$-$C_6$alkoxy or $C_3$-$C_8$cycloalkyl, $C_3$-$C_8$cycloalkyl, phenyl which is unsubstituted or monosubstituted to trisubstituted by halogen, $C_1$-$C_6$haloalkyl, $C_1$-$C_6$haloalkoxy, $C_1$-$C_6$alkoxy, $C_1$-$C_6$alkyl, phenoxy, halophenoxy, phenyl, benzyl, halobenzyl, nitro and/or cyano, or benzyl which is unsubstituted or monosubstituted to trisubstituted by halogen, $C_1$-$C_6$haloalkyl, $C_1$-$C_6$alkoxy, $C_1$-$C_6$alkyl, nitro and/or cyano;
$R_2$ is $C_1$-$C_{12}$alkyl, $C_1$-$C_6$alkyl which is substituted by $C_1$-$C_6$alkoxy or $C_3$-$C_8$cycloalkyl, $C_3$-$C_8$cycloalkyl, phenyl which is unsubstituted or monosubstituted to trisubstituted by halogen, $C_1$-$C_6$haloalkyl, $C_1$-$C_6$haloalkoxy, $C_1$-$C_6$alkoxy, $C_1$-$C_6$alkyl, phenoxy, halophenoxy, phenyl, benzyl, halobenzyl, nitro and/or cyano, or benzyl which is unsubstituted or monosubstituted to trisubstituted by halogen, $C_1$-$C_6$haloalkyl, $C_1$-$C_6$alkoxy, $C_1$-$C_6$alkyl, nitro and/or cyano; and
$R_3$ is $C_1$-$C_6$alkyl which is unsubstituted or substituted by $C_1$-$C_3$alkoxy, or is $C_3$-$C_4$alkenyl, benzyl or halobenzyl, which comprises

a) reacting an oxirane of the formula II

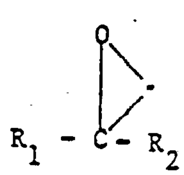

(II)

at temperatures of -20° to +100° C, in the presence of an acid catalyst or an acid condensation agent, with an alcohol of the formula III

$R_3 - OH$ (III)

to give a glycol monoether of the formula IV

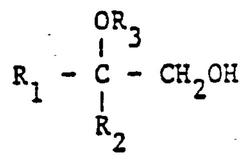

(IV)

and

b) subsequently reacting the glycol monoether of the formula IV or one of its esters, in the presence of an acid-binding agent or condensation agent at temperatures of 0° to 150° C, with a triazole of the formula V

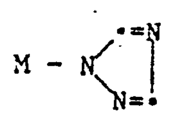

(V)

the substituents $R_1$, $R_2$ and $R_3$ in the formulae II to IV being as defined under formula I and M in formula V being hydrogen or a metal atom.

**2.** A process according to claim 1, wherein the reaction of the oxirane of the formula II with the alcohol of the formula III is carried out at temperatures between 0° and 40° C.

**3.** A process according to claim 1, wherein the acid catalyst or acid condensation agent employed in a protonic acid, a Lewis acid or an ion exchanger in the $H^+$ form.

**4.** A process according to claim 1, wherein the reaction of the glycol monoether of the formula IV with the triazole of the formula V is carried out at temperatures between 20° and 100° C.

**5.** A process according to claim 1, wherein the free hydroxyl group in IV is esterified before the reaction of IV with V.

**6.** A process according to claim 1, which comprises preparing, as the process product, a compound of the formula I in which $R_1$ and $R_2$ independently of one another are $C_1$-$C_{12}$alkyl, $C_1$-$C_6$alkyl which is substituted by $C_1$-$C_6$alkoxy or $C_3$-$C_8$cycloalkyl, $C_3$-$C_8$cycloalkyl, phenyl, phenyl which is monosubstituted to trisubstituted by halogen, $C_1$-$C_6$haloalkyl, $C_1$-$C_6$haloalkoxy, $C_1$-$C_6$alkoxy, $C_1$-$C_6$alkyl, nitro and/or cyano, phenyl which is substituted by phenoxy, halophenoxy, phenyl, halophenyl, benzyl or halobenzyl, benzyl or benzyl which is monosubstituted to trisubstituted by halogen, $C_1$-$C_6$haloalkyl, $C_1$-$C_6$haloalkoxy, $C_1$-$C_6$alkoxy, $C_1$-$C_6$alkyl, nitro and/or cyano; and $R_3$ is $C_1$-$C_6$alkyl, $C_1$-$C_6$alkyl which is substituted by $C_1$-$C_3$alkoxy, $C_3$-$C_4$alkenyl, benzyl or halobenzyl.

**7.** A process according to claim 1, which comprises preparing, as the process product, a compound of the formula I in which $R_1$ and $R_2$ independently of one another are $C_1$-$C_6$alkyl, $C_1$-$C_2$alkyl which is substituted by $C_1$-$C_3$alkoxy or $C_3$-$C_6$cycloalkyl, $C_3$-$C_6$cycloalkyl, phenyl, phenyl which is monosubstituted to trisubstituted by halogen, $C_1$-$C_3$haloalkyl, $C_1$-$C_3$alkoxy, $C_1$-$C_3$alkyl, nitro and/or cyano, phenyl which is substituted by phenoxy, halophenoxy, phenyl, benzyl or halobenzyl, benzyl or benzyl which is monosubstituted to trisubstituted by halogen, $C_1$-$C_3$haloalkyl, methyl, methoxy, nitro and/or cyano; and $R_3$ is $C_1$-$C_4$alkyl, $C_1$-$C_2$alkyl which is substituted by $C_1$-$C_3$alkoxy, $C_3$-$C_4$alkenyl, benzyl or halobenzyl.

**8.** A process according to claim 7, which comprises preparing, as the process product, a compound of the formula I in which $R_1$ is phenyl, phenyl which is monosubstituted to trisubstituted by halogen or phenyl which is monosubstituted by phenoxy or halophenoxy; $R_2$ is $C_1$-$C_6$alkyl or $C_3$-$C_7$cycloalkyl and $R_3$ is $C_1$-$C_6$alkyl, $C_1$-$C_6$alkyl which is substituted by $C_1$-$C_3$alkoxy, $C_3$-$C_4$alkenyl, benzyl or halobenzyl.

**9.** A compound of the formula IV'

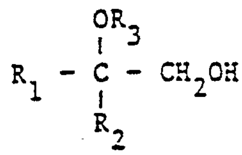

in which $R_1$ is $C_1$-$C_6$alkyl which is substituted by $C_1$-$C_6$alkoxy or $C_3$-$C_6$cycloalkyl, $C_3$-$C_8$cycloalkyl, phenyl, phenyl which is monosubstituted to trisubstituted by halogen, $C_1$-$C_6$haloalkyl, $C_1$-$C_6$haloalkoxy, $C_1$-$C_6$alkoxy, $C_1$-$C_6$alkyl, nitro and/or cyano, phenyl which is substituted by phenoxy, halophenoxy, phenyl, halophenyl, benzyl or halobenzyl, benzyl or benzyl which is monosubstituted to trisubstituted by halogen, $C_1$-$C_6$haloalkyl, $C_1$-$C_6$haloalkoxy, $C_1$-$C_6$alkoxy, $C_1$-$C_6$alkyl, nitro and/or cyano; $R_2$ is $C_1$-$C_{12}$alkyl or a radical indicated under $R_1$; and $R_3$ is $C_1$-$C_6$alkyl, $C_1$-$C_6$alkyl which is substituted by $C_1$-$C_3$alkoxy, $C_3$-$C_4$alkenyl, benzyl or halobenzyl.

**10.** A compound of the formula IV' according to claim 9, in which $R_1$ is phenyl, phenyl which is monosubstituted to trisubstituted by halogen or phenyl which is monosubstituted by phenoxy or halophenoxy; $R_2$ is $C_1$-$C_6$alkyl or $C_3$-$C_7$cycloalkyl and $R_3$ is $C_1$-$C_6$alkyl, $C_1$-$C_6$alkyl which is substituted by $C_1$-$C_3$alkoxy, $C_3$-$C_4$alkenyl, benzyl or halobenzyl.

**11.** A process for the preparation of the compounds of the formula IV' defined in claim 9, which comprises reacting an oxirane of the formula II

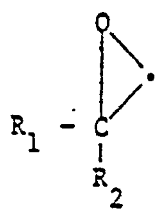

(II)

at temperatures of -20° to +100° C, in the presence of an acid catalyst or an acid condensation agent, with an alcohol of the formula III

$R_3$ - OH (III)

$R_1$, $R_2$ and $R_3$ in the formulae II and III having the meanings indicated under formula IV'.

**12.** A compound of the formula Ia

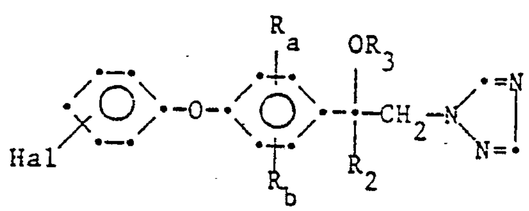

(Ia)

in which Hal is halogen; $R_a$ and $R_b$ independently of one another are hydrogen, halogen, $C_1$-$C_6$haloalkyl, $C_1$-$C_6$haloalkoxy, $C_1$-$C_6$alkoxy,$C_1$-$C_6$alkyl, nitro and/or cyano; $R_2$ is $C_1$-$C_{12}$alkyl, $C_1$-$C_6$alkyl which is substituted by $C_1$-$C_6$alkoxy or $C_3$-$C_8$cycloalkyl, $C_3$-$C_8$cycloalkyl, phenyl, which is unsubstituted or monosubstituted to trisubstituted by halogen, $C_1$-$C_6$haloalkyl, $C_1$-$C_6$haloalkoxy, $C_1$-$C_6$alkoxy, $C_1$-$C_6$alkyl, phenoxy, halophenoxy, phenyl, benzyl, halobenzyl, nitro and/or cyano, or benzyl which is unsubstituted or monosubstituted to trisubstituted by halogen, $C_1$-$C_6$haloalkyl, $C_1$-$C_6$alkoxy, $C_1$-$C_6$alkyl, nitro and/or cyano; and $R_3$ is $C_1$-$C_6$alkyl which is unsubstituted or substituted by $C_1$-$C_3$alkoxy or is $C_3$-$C_4$alkenyl, benzyl or halobenzyl.

**13.** A compound of the formula Ia according to claim 12, wherein Hal is fluorine, chlorine and/or bromine; $R_a$ and $R_b$ independently of one another are hydrogen, halogen, $C_1$-$C_3$haloalkyl, $C_1$-$C_3$haloalkoxy, $C_1$-$C_3$alkoxy,$C_1$-$C_3$alkyl, nitro and/or cyano; $R_2$ is $C_1$-$C_6$alkyl; and $R_3$ is $C_1$-$C_6$alkyl which is unsubstituted or substituted by $C_1$-$C_3$alkoxy, or is $C_3$-$C_4$alkenyl, benzyl or halobenzyl.

**14.** A compound of the formula Ia, according to claim 12, selected from the series:
1-(1H-1,2,4-triazol-1-yl)-2-methoxy-2-[4-(4-chlorophenoxy)-phenyl]-butane;
1-(1H-1,2,4-triazol-1-yl)-2-methoxy-2-[4-(4-fluorophenoxy)-phenyl]-butane;
1-(1H-1,2,4-triazol-1-yl)-2-methoxy-2-[4-(2,4-dichlorophenoxy)-phenyl]-butane;
1-(1H-1,2,4-triazol-1-yl)-2-methoxy-2-[4-(4-chlorophenoxy)-phenyl]-propane;
1-(1H-1,2,4-triazol-1-yl)-2-methoxy-2-[4-(4-chlorophenoxy)-2-chlorophenyl]-butane;
1-(1H-1,2,4-triazol-1-yl)-2-methoxy-2-[4-(4-chlorophenoxy)-phenyl]-3-methylbutane;
1-(1H-1,2,4-triazol-1-yl)-2-allyloxy-2-[4-(4-chlorophenoxy)-2-chlorophenyl]-pentane;
1-(1H-1,2,4-triazol-1-yl)-2-methoxy-2-[4-(4-chlorophenoxy)-2-chlorophenyl]-pentane;
1-(1H-1,2,4-triazol-1-yl)-2-allyloxy-2-[4-(4-chlorophenoxy)-2-chlorophenyl]-butane;
1-(1H-1,2,4-triazol-1-yl)-2-(2-methylallyloxy)-2-[4-(4-chlorophenoxy)-2-chlorophenyl]-butane;
1-(1H-1,2,4-triazol-1-yl)-2-allyloxy-2-[4-(4-chlorophenoxy)-2-chlorophenyl]-propane;
1-(1H-1,2,4-triazol-1-yl)-2-methoxy-2-[4-(4-chlorophenoxy)-2-chlorophenyl]-propane;
1-(1H-1,2,4-triazol-1-yl)-2-methoxy-2-[4-(4-chlorophenoxy)-2-methylphenyl]-propane;
1-(1H-1,2,4-triazol-1-yl)-2-allyloxy-2-[4-(4-chlorophenoxy)-2-methylphenyl]-propane;
1-(1H-1,2,4-triazol-1-yl)-2-methoxy-2-[4-(4-chlorophenoxy)-2-methylphenyl]-butane;
1-(1H-1,2,4-triazol-1-yl)-2-(2-methylpropoxy)-[4-(4-chlorophenoxy)-2-chlorophenyl]-butane;
1-(1H-1,2,4-triazol-1-yl)-2-allyloxy-2-[4-(4-chlorophenoxy)-2-methylphenyl]-pentane;
1-(1H-1,2,4-triazol-1-yl)-2-methoxy-2-[4-(4-chlorophenoxy)-2-methylphenyl]-butane;
1-(1H-1,2,4-triazol-1-yl)-2-allyloxy-2-[4-(4-chlorophenoxy)-2-methylphenyl]-butane;
1-(1H-1,2,4-triazol-1-yl)-2-propoxy-2-[4-(4-chlorophenoxy)-2-chlorophenyl]-pentane;
1-(1H-1,2,4-triazol-1-yl)-2-n-propoxy-2-[4-(4-chlorophenoxy)-2-chlorophenyl]-butane;
1-(1H-1,2,4-triazol-1-yl)-2-n-propoxy-2-[4-(4-chlorophenoxy)-2-chlorophenyl]-propane;
1-(1H-1,2,4-triazol-1-yl)-2-n-propoxy-2-[4-(4-chlorophenoxy)-2-methylphenyl]-propane.

**15.** A microbicidal composition which comprises 0.1 to 99 % of a compound of the formula Ia according to claim 12, 99.9 to 1 % of solid or liquid additives and 0 to 25 % of a surfactant.

**16.** A process for controlling phytopathogenic microorganisms, which comprises applying 10 g to 5 kg of a compound of the formula Ia, according to claim 12 per hectare of cultivated area.

**Claims for the following Contracting State: AT**

**1.** A process for the preparation of a 1-triazolylethyl ether derivative of the general formula I

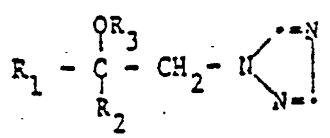

(I),

in which
$R_1$ is $C_1$-$C_{12}$alkyl, $C_1$-$C_6$alkyl which is substituted by $C_1$-$C_6$alkoxy or $C_3$-$C_8$cycloalkyl, $C_3$-$C_8$cycloalkyl, phenyl which is unsubstituted or monosubstituted to trisubstituted by halogen, $C_1$-$C_6$haloalkyl, $C_1$-$C_6$haloalkoxy, $C_1$-$C_6$alkoxy, $C_1$-$C_6$alkyl, phenoxy, halophenoxy, phenyl, benzyl, halobenzyl, nitro and/or cyano, or benzyl which is unsubstituted or monosubstituted to trisubstituted by halogen, $C_1$-$C_6$haloalkyl, $C_1$-$C_6$alkoxy, $C_1$-$C_6$alkyl, nitro and/or cyano;
$R_2$ is $C_1$-$C_{12}$alkyl, $C_1$-$C_6$alkyl which is substituted by $C_1$-$C_6$alkoxy or $C_3$-$C_8$cycloalkyl, $C_3$-$C_8$cycloalkyl, phenyl which is unsubstituted or monosubstituted to trisubstituted by halogen, $C_1$-$C_6$haloalkyl, $C_1$-$C_6$haloalkoxy, $C_1$-$C_6$alkoxy, $C_1$-$C_6$alkyl, phenoxy, halophenoxy, phenyl, benzyl, halobenzyl, nitro and/or cyano, or benzyl which is unsubstituted or monosubstituted to trisubstituted by halogen, $C_1$-$C_6$haloalkyl, $C_1$-$C_6$alkoxy, $C_1$-$C_6$alkyl, nitro and/or cyano; and

$R_3$ is $C_1$-$C_6$alkyl which is unsubstituted or substituted by $C_1$-$C_3$alkoxy, or is $C_3$-$C_4$alkenyl, benzyl or halobenzyl, which comprises

a) reacting an oxirane of the formula II

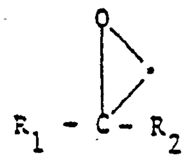

(II)

at temperatures of -20° to +100° C, in the presence of an acid catalyst or an acid condensation agent, with an alcohol of the formula III

$R_3$ - OH (III)

to give a glycol monoether of the formula IV

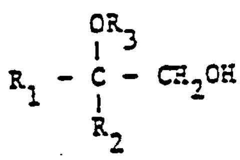

(IV)

and

b) subsequently reacting the glycol monoether of the formula IV or one of its esters, in the presence of an acid-binding agent or condensation agent at temperatures of 0° to 150° C, with a triazole of the formula V

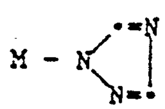

(V)

the substituents $R_1$, $R_2$ and $R_3$ in the formulae II to IV being as defined under formula I and M in formula V being hydrogen or a metal atom.

2. A process according to claim 1, wherein the reaction of the oxirane of the formula II with the alcohol of the formula III is carried out at temperatures between 0° and 40° C.

3. A process according to claim 1, wherein the acid catalyst or acid condensation agent employed in a protonic acid, a Lewis acid or an ion exchanger in the $H^+$ form.

4. A process according to claim 1, wherein the reaction of the glycol monoether of the formula IV with the triazole of the formula V is carried out at temperatures between 20° and 100° C.

5. A process according to claim 1, wherein the free hydroxyl group in IV is esterified before the reaction of IV with V.

6. A process according to claim 1, which comprises preparing, as the process product, a compound of the formula I in which $R_1$ and $R_2$ independently of one another are $C_1$-$C_{12}$alkyl, $C_1$-$C_6$alkyl which is substituted by $C_1$-$C_6$alkoxy or $C_3$-$C_8$cycloalkyl, $C_3$-$C_8$cycloalkyl, phenyl, phenyl which is monosubstituted to trisubstituted by halogen, $C_1$-$C_6$haloalkyl, $C_1$-$C_6$haloalkoxy, $C_1$-$C_6$alkoxy, $C_1$-$C_6$alkyl, nitro and/or cyano, phenyl which is substituted by phenoxy, halophenoxy, phenyl, halophenyl, benzyl or halobenzyl, benzyl or benzyl which is monosubstituted to trisubstituted by halogen, $C_1$-$C_6$haloalkyl, $C_1$-$C_6$haloalkoxy, $C_1$-$C_6$alkoxy, $C_1$-$C_6$alkyl, nitro and/or cyano; and $R_3$ is $C_1$-$C_6$alkyl, $C_1$-$C_6$alkyl which is

substituted by $C_1$-$C_3$alkoxy, $C_3$-$C_4$alkenyl, benzyl or halobenzyl.

**7.** A process according to claim 1, which comprises preparing, as the process product, a compound of the formula I in which $R_1$ and $R_2$ independently of one another are $C_1$-$C_6$alkyl, $C_1$-$C_2$alkyl which is substituted by $C_1$-$C_3$alkoxy or $C_3$-$C_6$cycloalkyl, $C_3$-$C_6$cycloalkyl, phenyl, phenyl which is monosubstituted to trisubstituted by halogen, $C_1$-$C_3$haloalkyl, $C_1$-$C_3$alkoxy, $C_1$-$C_3$alkyl, nitro and/or cyano, phenyl which is substituted by phenoxy, halophenoxy, phenyl, benzyl or halobenzyl, benzyl or benzyl which is monosubstituted to trisubstituted by halogen, $C_1$-$C_3$haloalkyl, methyl, methoxy, nitro and/or cyano; and $R_3$ is $C_1$-$C_4$alkyl, $C_1$-$C_2$alkyl which is substituted by $C_1$-$C_3$alkoxy, $C_3$-$C_4$alkenyl, benzyl or halobenzyl.

**8.** A process according to claim 7, which comprises preparing, as the process product, a compound of the formula I in which $R_1$ is phenyl, phenyl which is monosubstituted to trisubstituted by halogen or phenyl which is monosubstituted by phenoxy or halophenoxy; $R_2$ is $C_1$-$C_6$alkyl or $C_3$-$C_7$cycloalkyl and $R_3$ is $C_1$-$C_6$alkyl, $C_1$-$C_6$alkyl which is substituted by $C_1$-$C_3$alkoxy, $C_3$-$C_4$alkenyl, benzyl or halobenzyl.

**9.** A process for the preparation of compounds of the formula IV'

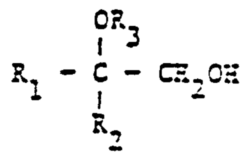

in which $R_1$ is $C_1$-$C_6$alkyl which is substituted by $C_1$-$C_6$alkoxy or $C_3$-$C_6$cycloalkyl, $C_3$-$C_8$cycloalkyl, phenyl, phenyl which is monosubstituted to trisubstituted by halogen, $C_1$-$C_6$haloalkyl, $C_1$-$C_6$haloalkoxy, $C_1$-$C_6$alkoxy, $C_1$-$C_6$alkyl, nitro and/or cyano, phenyl which is substituted by phenoxy, halophenoxy, phenyl, halophenyl, benzyl or halobenzyl, benzyl or benzyl which is monosubstituted to trisubstituted by halogen, $C_1$-$C_6$haloalkyl, $C_1$-$C_6$haloalkoxy, $C_1$-$C_6$alkoxy, $C_1$-$C_6$alkyl, nitro and/or cyano; $R_2$ is $C_1$-$C_{12}$alkyl or a radical indicated under $R_1$; and $R_3$ is $C_1$-$C_6$alkyl, $C_1$-$C_6$alkyl which is substituted by $C_1$-$C_3$alkoxy, $C_3$-$C_4$alkenyl, benzyl or halobenzyl, which comprises reacting an oxirane of the formula II

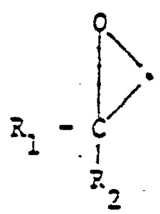

(II)

at temperatures of -20° to +100° C, in the presence of an acid catalyst or an acid condensation agent, with an alcohol of the formula III

$R_3$ - OH (III)

$R_1$, $R_2$ and $R_3$ in the formula II and III having the meanings indicated under formula IV'.

**10.** A process according to claim 9, for the preparation of compounds of the formula IV', in which $R_1$ is phenyl, phenyl which is monosubstituted to trisubstituted by halogen or phenyl which is monosubstituted by phenoxy or halophenoxy; $R_2$ is $C_1$-$C_6$alkyl or $C_3$-$C_7$cycloalkyl and $R_3$ is $C_1$-$C_6$alkyl, $C_1$-$C_6$alkyl which is substituted by $C_1$-$C_3$alkoxy, $C_3$-$C_4$alkenyl, benzyl or halobenzyl.

**11.** A microbicidal composition which comprises, in addition 99.9 to 1 % of solid or liquid additive and 0 to 25 % of a surfactant, as active ingredient 0.1 to 99 % of a compound of the formula Ia

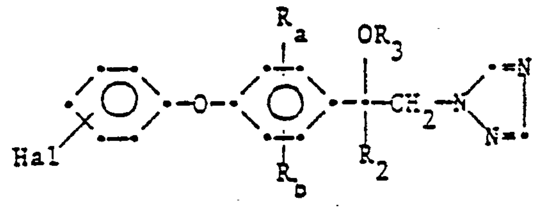

(Ia)

in which Hal is halogen; $R_a$ and $R_b$ independently of one another are hydrogen, halogen, $C_1$-$C_6$haloalkyl, $C_1$-$C_6$haloalkoxy, $C_1$-$C_6$alkoxy,$C_1$-$C_6$alkyl, nitro and/or cyano; $R_2$ is $C_1$-$C_{12}$alkyl, $C_1$-$C_6$alkyl which is substituted by $C_1$-$C_6$alkoxy or $C_3$-$C_8$cycloalkyl, $C_3$-$C_8$cycloalkyl, phenyl, which is unsubstituted or monosubstituted to trisubstituted by halogen, $C_1$-$C_6$haloalkyl, $C_1$-$C_6$haloalkoxy, $C_1$-$C_6$alkoxy, $C_1$-$C_6$alkyl, phenoxy, halophenoxy, phenyl, benzyl, halobenzyl, nitro and/or cyano, or benzyl which is unsubstituted or monosubstituted to trisubstituted by halogen, $C_1$-$C_6$haloalkyl, $C_1$-$C_6$alkoxy, $C_1$-$C_6$alkyl, nitro and/or cyano; and $R_3$ is $C_1$-$C_6$alkyl which is unsubstituted or substituted by $C_1$-$C_3$alkoxy, or is $C_3$-$C_4$alkenyl, benzyl or halobenzyl.

**12.** A composition according to claim 11, wherein Hal is fluorine, chlorine and/or bromine; $R_a$ and $R_b$ independently of one another are hydrogen, halogen, $C_1$-$C_3$haloalkyl, $C_1$-$C_3$haloalkoxy, $C_1$-$C_3$alkoxy,$C_1$-$C_3$alkyl, nitro and/or cyano; $R_2$ is $C_1$-$C_6$alkyl; and $R_3$ is $C_1$-$C_6$alkyl which is unsubstituted or substituted by $C_1$-$C_3$alkoxy, or is $C_3$-$C_4$alkenyl, benzyl or halobenzyl.

**13.** A composition according to claim 11, which comprises as active ingredient a compound of the formula Ia, selected from the series:
1-(1H-1,2,4-triazol-1-yl)-2-methoxy-2-[4-(4-chlorophenoxy)-phenyl]-butane;
1-(1H-1,2,4-triazol-1-yl)-2-methoxy-2-[4-(4-fluorophenoxy)-phenyl]-butane;
1-(1H-1,2,4-triazol-1-yl)-2-methoxy-2-[4-(2,4-dichlorophenoxy)-phenyl]-butane;
1-(1H-1,2,4-triazol-1-yl)-2-methoxy-2-[4-(4-chlorophenoxy)-phenyl]-propane;
1-(1H-1,2,4-triazol-1-yl)-2-methoxy-2-[4-(4-chlorophenoxy)-2-chlorophenyl]-butane;
1-(1H-1,2,4-triazol-1-yl)-2-methoxy-2-[4-(4-chlorophenoxy)-phenyl]-3-methylbutane;
1-(1H-1,2,4-triazol-1-yl)-2-allyloxy-2-[4-(4-chlorophenoxy)-2-chlorophenyl]-pentane;
1-(1H-1,2,4-triazol-1-yl)-2-methoxy-2-[4-(4-chlorophenoxy)-2-chlorophenyl]-pentane;
1-(1H-1,2,4-triazol-1-yl)-2-allyloxy-2-[4-(4-chlorophenoxy)-2-chlorophenyl]-butane;
1-(1H-1,2,4-triazol-1-yl)-2-(2-methylallyloxy)-2-[4-(4-chlorophenoxy)-2-chlorophenyl]-butane;
1-(1H-1,2,4-triazol-1-yl)-2-allyloxy-2-[4-(4-chlorophenoxy)-2-chlorophenyl]-propane;
1-(1H-1,2,4-triazol-1-yl)-2-methoxy-2-[4-(4-chlorophenoxy)-2-chlorophenyl]-propane;
1-(1H-1,2,4-triazol-1-yl)-2-methoxy-2-[4-(4-chlorophenoxy)-2-methylphenyl]-propane;
1-(1H-1,2,4-triazol-1-yl)-2-allyloxy-2-[4-(4-chlorophenoxy)-2-methylphenyl]-propane;
1-(1H-1,2,4-triazol-1-yl)-2-methoxy-2-[4-(4-chlorophenoxy)-2-methylphenyl]-butane;
1-(1H-1,2,4-triazol-1-yl)-2-(2-methylpropoxy)-[4-(4-chlorophenoxy)-2-chlorophenyl]-butane;
1-(1H-1,2,4-triazol-1-yl)-2-allyloxy-2-[4-(4-chlorophenoxy)-2-methylphenyl]-pentane;
1-(1H-1,2,4-triazol-1-yl)-2-methoxy-2-[4-(4-chlorophenoxy)-2-methylphenyl]-butane;
1-(1H-1,2,4-triazol-1-yl)-2-allyloxy-2-[4-(4-chlorophenoxy)-2-methylphenyl]-butane;
1-(1H-1,2,4-triazol-1-yl)-2-propoxy-2-[4-(4-chlorophenoxy)-2-chlorophenyl]-pentane;
1-(1H-1,2,4-triazol-1-yl)-2-n-propoxy-2-[4-(4-chlorophenoxy)-2-chlorophenyl]-butane;
1-(1H-1,2,4-triazol-1-yl)-2-n-propoxy-2-[4-(4-chlorophenoxy)-2-chlorophenyl]-propane;
1-(1H-1,2,4-triazol-1-yl)-2-n-propoxy-2-[4-(4-chlorophenoxy)-2-methylphenyl]-propane.

**14.** A process for controlling phytopathogenic microorganisms, which comprises applying 10 g to 5 kg of a compound of the formula Ia, according to claim 11 per hectare of cultivated area.

**Revendications**
**Revendications pour les Etats contractants suivants: BE CH DE FR IT LI LU NL SE**

**1.** Procédé pour la préparation de dérivés de 1-triazolyléthyléther de formule générale I

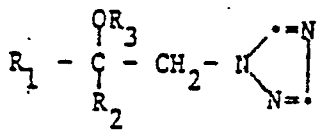

(I) ,

où

$R_1$ représente un alkyle en $C_1$-$C_{12}$, un alkyle en $C_1$-$C_6$ substitué par un groupe alcoxy en $C_1$-$C_6$ ou cycloalkyle en $C_3$-$C_8$, un cycloalkyle en $C_3$-$C_8$, un phényle non substitué ou mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, phénoxy, halogénophénoxy, phényle, benzyle, halogénobenzyle, nitro et/ou cyano, ou un benzyle non substitué ou mono- à trisubstitué par un groupe halogène, halogénoalkyle en $C_1$-$C_6$, alcoxy en $C_1$-$C_6$, alkyle en $C_1$-$c_6$, nitro et/ou cyano ;

$R_2$ représente un alkyle en $C_1$-$C_{12}$, un alkyle en $C_1$-$C_6$ substitué par un groupe alcoxy en $C_1$-$C_6$ ou cycloalkyle en $C_3$-$C_8$, un cycloalkyle en $C_3$-$C_8$, un phényle non substitué ou mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, phénoxy, halogénophénoxy, phényle, benzyle, halogénobenzyle, nitro et/ou cyano, ou un benzyle non substitué ou mono- à trisubstitué par un groupe halogène, halogénoalkyle en $C_1$-$C_6$, alcoxy en $C_1$-$C_6$, alkyle en $C_1$-$C_6$, nitro et/ou cyano ; et

$R_3$ représente un alkyle en $C_1$-$C_6$ non substitué ou substitué par un groupe alcoxy en $C_1$-$C_3$, un alcényle en $C_3$-$C_4$, un benzyle ou un halogénobenzyle, caractérisé en ce qu'on

a) fait réagir un oxiranne de formule II

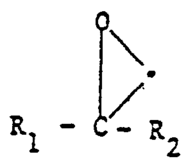

(II)

à des températures de -20° à +100° C en présence d'un catalyseur acide ou d'un agent de condensation acide avec un alcool de formule III

$R_3$ - OH (III)

pour former un monoéther de glycol de formule IV

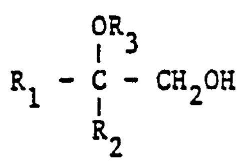

(IV)

et

b) fait ensuite réagir le monoéther de glycol de formule IV ou un de des esters en présence d'un agent de neutralisation ou d'un agent de condensation à des températures de 0° à 150° C avec un triazole de formule V

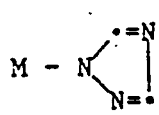

(V)

les substituants $R_1$, $R_2$ et $R_3$ dans les formules II à IV étant définis comme dans la formule I et M dans la formule V désignant l'hydrogène ou un atome de métal.

2. Procédé selon la revendication 1, caractérisé en ce qu'on effectue la réaction de l'oxiranne de formule II avec l'alcool de formule III à des températures de 0° à 40° C.

3. Procédé selon la revendication 1, caractérisé en ce qu'on utilise comme catalyseur acide ou comme agent de condensation acide un acide protonique, un acide de Lewis ou un échangeur d'ions sous forme $H^+$.

4. Procédé selon la revendication 1, caractérisé en ce qu'on effectue la réaction du monoéther de glycol de formule IV avec le triazole de formule V à des températures de 20° à 100° C.

5. Procédé selon la revendication 1, caractérisé en ce qu'on estérifie les groupes hydroxyle libres dans IV avant la réaction de IV avec V.

6. Procédé selon la revendication 1, caractérisé en ce qu'on prépare comme produit du procédé un composé de formule I, dans laquelle $R_1$ et $R_2$ représentent, indépendamment l'un de l'autre, un alkyle en $C_1$-$C_{12}$, un alkyle en $C_1$-$C_6$ substitué par un groupe alcoxy en $C_1$-$C_6$ ou cycloalkyle en $C_3$-$C_8$, un cycloalkyle en $C_3$-$C_8$, un phényle, un phényle mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, nitro et/ou cyano, un phényle substitué par un groupe phénoxy, halogénophénoxy, phényle, halogénophényle, benzyle ou halogénobenzyle, un benzyle on un benzyle mono- à trisubstitué par des groupes halogène, halogénoalkyle en $C_1$-$C_6$, alcoxy en $C_1$-$C_6$, halogénoalcoxy en $C_1$-$C_6$, alkyle en $C_1$-$C_6$, nitro et/ou cyano ; et $R_3$ représente un alkyle en $C_1$-$C_6$, un alkyle en $C_1$-$C_6$ substitué par un groupe alcoxy en $C_1$-$C_3$, un alcényle en $C_3$-$C_4$, un benzyle ou un halogénobenzyle.

7. Procédé selon la revendication 1, caractérisé en ce qu'on prépare comme produit du procédé un composé de formule I, dans laquelle $R_1$ et $R_2$ représentent, indépendamment l'un de l'autre, un alkyle en $C_1$-$C_6$, un alkyle en $C_1$-$C_2$ substitué par un groupe alcoxy en $C_1$-$C_3$ ou cycloalkyle en $C_3$-$C_6$, un cycloalkyle en $C_3$-$C_6$, un phényle, un phényle mono- à trisubstitué par des groupes halogène, halogénoalkyle en $C_1$-$C_3$, alcoxy en $C_1$-$C_3$, alkyle en $C_1$-$C_3$, nitro et/ou cyano, un phényle substitué par un groupe phénoxy, halogénophénoxy, phényle, benzyle ou halogénobenzyle, un benzyle ou un benzyle mono- à trisubstitué par des groupes halogène, halogénoalkyle en $C_1$-$C_3$, méthyle, méthoxy, nitro et/ou cyano ; et $R_3$ représente un alkyle en $C_1$-$C_4$, un alkyle en $C_1$-$C_2$ substitué par un groupe alcoxy en $C_1$-$C_3$, un alcényle en $C_3$-$C_4$, un benzyle ou un halogénobenzyle.

8. Procédé selon la revendication 7, caractérisé en ce qu'on prépare comme produit du procédé un composé de formule I, où $R_1$ représente un phényle, un phényle mono- à trisubstitué par des groupes halogène ou monosubstitué par un groupe phénoxy ou halogénophénoxy ; $R_2$ représente un alkyle en $C_1$-$C_6$ ou un cycloalkyle en $C_3$-$C_7$ et $R_3$ est un alkyle en $C_1$-$C_6$, un alkyle en $C_1$-$C_6$ substitué par un groupe alcoxy en $C_1$-$C_3$, un alcényle en $C_3$-$C_4$, un benzyle ou un halogénobenzyle.

9. Composés de formule IV'

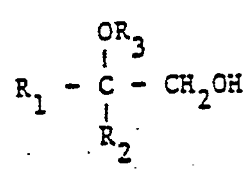

où $R_1$ représente un alkyle en $C_1$-$C_6$ substitué par un groupe alcoxy en $C_1$-$C_6$ ou cycloalkyle en $C_3$-$C_6$, un cycloalkyle en $C_3$-$C_8$, un phényle, un phényle mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, nitro et/ou cyano, un phényle substitué par un groupe phénoxy, halogénophénoxy, phényle, halogénophényle, benzyle ou halogénobenzyle, un benzyle ou un benzyle mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, nitro et/ou cyano ; $R_2$ désigne un alkyle en $C_1$-$C_{12}$ ou un reste tel qu'indiqué sous $R_1$ ; et $R_3$ est un alkyle en $C_1$-$C_6$, un alkyle en $C_1$-$C_6$ substitué par un groupe alcoxy en $C_1$-$C_3$, un alcényle en $C_3$-$C_4$, un benzyle ou un halogénobenzyle.

10. Composés de formule IV' selon la revendication 9, caractérisés en ce que $R_1$ représente un phényle, un phényle mono- à trisubstitué par des groupes halogène ou monosubstitué par un groupe phénoxy

ou halogénophénoxy ; $R_2$ représente un alkyle en $C_1$-$C_6$ ou un cycloalkyle en $C_3$-$C_7$ et $R_3$ désigne un alkyle en $C_1$-$C_6$, un alkyle en $C_1$-$C_6$ substitué par un groupe alcoxy en $C_1$-$C_3$, un alcényle en $C_3$-$C_4$, un benzyle ou un halogénobenzyle.

**11.** Procédé pour la préparation des composés de formule IV' définis dans la revendication 9, caractérisé en ce qu'on fait réagir un oxiranne de formule II

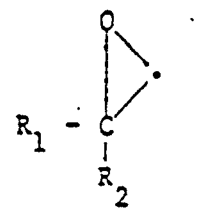

(II)

à des températures de -20° à +100°C en présence d'un catalyseur acide ou d'un agent de condensation acide avec un alcool de formule III

$R_3$ - OH (III)

$R_1$, $R_2$ et $R_3$ dans les formules II et III ayant les significations indiquées pour la formule IV'.

**12.** Composés de formule Ia

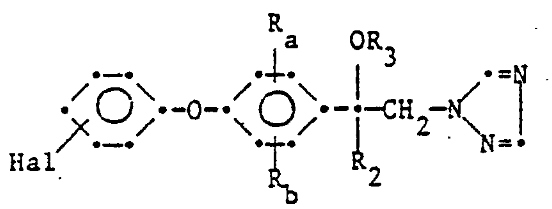

(Ia)

où Hal désigne un halogène ;
$R_a$ et $R_b$ représentent, indépendamment l'un de l'autre, l'hydrogène, un halogène, un halogénoalkyle en $C_1$-$C_6$, un halogénoalcoxy en $C_1$-$C_6$, un alcoxy en $C_1$-$C_6$, un alkyle en $C_1$-$C_6$, un groupe nitro et/ou un groupe cyano ;
$R_2$ représente un alkyle en $C_1$-$C_{12}$, un alkyle en $C_1$-$C_6$ substitué par un groupe alcoxy en $C_1$-$C_6$ ou cycloalkyle en $C_3$-$C_8$, un cycloalkyle en $C_3$-$C_8$, un phényle non substitué ou mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, phénoxy, halogénophénoxy, phényle, benzyle, halogénobenzyle, nitro et/ou cyano ou un benzyle non substitué ou mono- à trisubstitué par des groupes halogène, halogénoalkyle en $C_1$-$C_6$, alcoxy en $C_1$-$C_6$, alkyle en $C_1$-$C_6$, nitro et/ou cyano ; et
$R_3$ représente un alkyle en $C_1$-$C_6$ non substitué ou substitué par un groupe alcoxy en $C_1$-$C_3$, un alcényle en $C_3$-$C_4$, un benzyle ou un halogénobenzyle.

**13.** Composés de formule Ia selon la revendication 12, caractérisés en ce que Hal désigne le fluor, le chlore et/ou le brome ; $R_a$ et $R_b$ représentent, indépendamment l'un de l'autre, l'hydrogène, un halogène, un halogénoalkyle en $C_1$-$C_3$, un halogénoalcoxy en $C_1$-$C_3$, un alcoxy en $C_1$-$C_3$, un alkyle en $C_1$-$C_3$, un groupe nitro et/ou un groupe cyano ; $R_2$ désigne un alkyle en $C_1$-$C_6$ ; et $R_3$ représente un alkyle en $C_1$-$C_6$ non substitué ou substitué par un groupe alcoxy en $C_1$-$C_3$, un alkyle en $C_1$-$C_6$, un alcényle en $C_3$-$C_4$, un benzyle ou un halogénobenzyle.

**14.** Composé de formule Ia selon la revendication 12, choisi parmi les suivants :
1-(1H-1,2,4-triazol-1-yl)-2-méthoxy-2-[4-(4-chlorophénoxy)-phényl]-butane ;
1-(1H-1,2,4-triazol-1-yl)-2-méthoxy-2-[4-(4-fluorophénoxy)-phényl]-butane ;
1-(1H-1,2,4-triazol-1-yl)-2-méthoxy-2-[4-(2,4-dichlorophénoxy)-phényl]-butane ;
1-(1H-1,2,4-triazol-1-yl)-2-méthoxy-2-[4-(4-chlorophénoxy)-phényl]-propane ;
1-(1H-1,2,4-triazol-1-yl)-2-méthoxy-2-[4-(4-chlorophénoxy)-2-chlorophényl]-butane ;

1-(1H-1,2,4-triazol-1-yl)-2-méthoxy-2-[4-(4-chlorophénoxy)-phényl]-3-méthyl-butane ;
1-(1H-1,2,4-triazol-1-yl)-2-allyloxy-2-[4-(4-chlorophénoxy)-2-chlorophényl]-pentane ;
1-(1H-1,2,4-triazol-1-yl)-2-methoxy-2-[4-(4-chlorophénoxy)-2-chlorophényl]-pentane ;
1-(1H-1,2,4-triazol-1-yl)-2-allyloxy-2-[4-(4-chlorophénoxy)-2-chlorophényl]butane ;
1-(1H-1,2,4-triazol-1-yl)-2-(2-méthylallyloxy)-2-[4-(4-chlorophénoxy)-2-chlorophényl]-butane ;
1-(1H-1,2,4-triazol-1-yl)-2-allyloxy-2-[4-(4-chlorophénoxy)-2-chlorophényl]-propane ;
1-(1H-1,2,4-triazol-1-yl)-2-méthoxy-2-[4-(4-chlorophénoxy)-2-chlorophényl]-propane ;
1-(1H-1,2,4-triazol-1-yl)-2-méthoxy-2-[4-(4-chlorophénoxy)-2-méthylphényl]-propane ;
1-(1H-1,2,4-triazol-1-yl)-2-allyloxy-2-[4-(4-chlorophénoxy)-2-méthylphényl]-propane ;
1-(1H-1,2,4-triazol-1-yl)-2-méthoxy-2-[4-(4-chlorophénoxy)-2-méthylphényl]-butane ;
1-(1H-1,2,4-triazol-1-yl)-2-(2-méthylpropoxy)-[4-(4-chlorophénoxy)-2-chlorophényl]-butane ;
1-(1H-1,2,4-triazol-1-yl)-2-allyloxy-2-[4-(4-chlorophénoxy)-2-méthylphényl]-pentane ;
1-(1H-1,2,4-triazol-1-yl)-2-méthoxy-2-[4-(4-chlorophénoxy)-2-méthylphényl]-butane ;
1-(1H-1,2,4-triazol-1-yl)-2-allyloxy-2-[4-(4-chlorophénoxy)-2-méthylphényle]-butane ;
1-(1H-1,2,4-triazol-1-yl)-2-propoxy-2-[4-(4-chlorophénoxy)-2-chlorophényl]-pentane ;
1-(1H-1,2,4-triazol-1-yl)-2-n-propoxy-2-[4-(4-chlorophénoxy)-2-chlorophényl]-butane ;
1-(1H-1,2,4-triazol-1-yl)-2-n-propoxy-2-[4-(4-chlorophénoxy)-2-chlorophényl]-propane ;
1-(1H-1,2,4-triazol-1-yl)-2-n-propoxy-2-[4-(4-chlorophénoxy)-2-méthylphényl]-propane.

**15.** Agent microbicide, caractérisé en ce qu'il contient 0,1 à 99% d'un composé de formule Ia selon la revendication 12, 99,9 à 1% d'additifs solides ou liquides et 0 à 25% d'un agent de surface.

**16.** Procédé pour la lutte contre les microorganismes phytopathogènes, caractérisé en ce qu'on applique 10 g à 5 kg d'un composé de formule Ia selon la revendication 12 par hectare de surface cultivable.

**Revendications pour l'Etat contractant suivant: AT**

**1.** Procédé pour la préparation de dérivés de 1-triazolyléthyléther de formule générale I

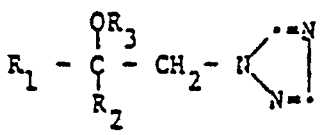

(I) ,

où
$R_1$ représente un alkyle en $C_1$-$C_{12}$, un alkyle en $C_1$-$C_6$ substitué par un groupe alcoxy en $C_1$-$C_6$ ou cycloalkyle en $C_3$-$C_8$, un cycloalkyle en $C_3$-$C_8$, un phényle non substitué ou mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, phénoxy, halogénophénoxy, phényle, benzyle, halogénobenzyle, nitro et/ou cyano, ou un benzyle non substitué ou mono- à trisubstitué par un groupe halogène, halogénoalkyle en $C_1$-$C_6$, alcoxy en $C_1$-$C_6$, alkyle en $C_1$-$C_6$, nitro et/ou cyano ;
$R_2$ représente un alkyle en $C_1$-$C_{12}$, un alkyle en $C_1$-$C_6$ substitué par un groupe alcoxy en $C_1$-$C_6$ ou cycloalkyle en $C_3$-$C_8$, un cycloalkyle en $C_3$-$C_8$, un phényle non substitué ou mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, phénoxy, halogénophénoxy, phényle, benzyle, halogénobenzyle, nitro et/ou cyano, ou un benzyle non substitué ou mono- à trisubstitué par un groupe halogène, halogénoalkyle en $C_1$-$C_6$, alcoxy en $C_1$-$C_6$, alkyle en $C_1$-$C_6$, nitro et/ou cyano ; et
$R_3$ représente un alkyle en $C_1$-$C_6$ non substitué ou substitué par un groupe alcoxy en $C_1$-$C_3$, un alcényle en $C_3$-$C_4$, un benzyle ou un halogénobenzyle, caractérisé en ce qu'on

a) fait réagir un oxiranne de formule II

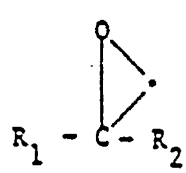

(II)

à des températures de -20° à +100°C en présence d'un catalyseur acide ou d'un agent de condensation acide avec un alcool de formule III

$R_3$ - OH (III)

pour former un monoéther de glycol de formule IV

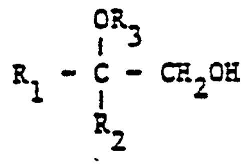

(IV)

et

b) fait ensuite réagir le monoéther de glycol de formule IV ou un de des esters en présence d'un agent de neutralisation ou d'un agent de condensation à des températures de 0° à 150°C avec un triazole de formule V

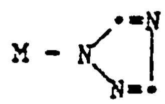

(V)

les substituants $R_1$, $R_2$ et $R_3$ dans les formules II à IV étant définis comme dans la formule I et M dans la formule V désignant l'hydrogène ou un atome de métal.

**2.** Procédé selon la revendication 1, caractérisé en ce qu'on effectue la réaction de l'oxiranne de formule II avec l'alcool de formule III à des températures de 0° à 40°C.

**3.** Procédé selon la revendication 1, caractérisé en ce qu'on utilise comme catalyseur acide ou comme agent de condensation acide un acide protonique, un acide de Lewis ou un échangeur d'ions sous forme $H^+$.

**4.** Procédé selon la revendication 1, caractérisé en ce qu'on effectue la réaction du monoéther de glycol de formule IV avec le triazole de formule V à des températures de 20° à 100°C.

**5.** Procédé selon la revendication 1, caractérisé en ce qu'on estérifie les groupes hydroxyle libres dans IV avant la réaction de IV avec V.

**6.** Procédé selon la revendication 1, caractérisé en ce qu'on prépare comme produit du procédé un composé de formule I, dans laquelle $R_1$ et $R_2$ représentent, indépendamment l'un de l'autre, un alkyle en $C_1$-$C_{12}$, un alkyle en $C_1$-$C_6$ substitué par un groupe alcoxy en $C_1$-$C_6$ ou cycloalkyle en $C_3$-$C_8$, un cycloalkyle en $C_3$-$C_8$, un phényle, un phényle mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, nitro et/ou cyano, un phényle substitué par un groupe phénoxy, halogénophénoxy, phényle, halogénophényle, benzyle ou halogénobenzyle, un benzyle on un benzyle mono- à trisubstitué par des groupes halogène, halogénoalkyle en $C_1$-$C_6$, alcoxy en $C_1$-$C_6$, halogénoalcoxy en $C_1$-$C_6$, alkyle en $C_1$-$C_6$, nitro et/ou cyano ; et $R_3$ représente un alkyle en $C_1$-$C_6$, un alkyle en $C_1$-$C_6$ substitué par un groupe alcoxy en $C_1$-$C_3$, un alcényle en $C_3$-$C_4$, un benzyle ou un halogénobenzyle.

**7.** Procédé selon la revendication 1, caractérisé en ce qu'on prépare comme produit du procédé un composé de formule I, dans laquelle $R_1$ et $R_2$ représentent, indépendamment l'un de l'autre, un alkyle en $C_1$-$C_6$, un alkyle en $C_1$-$C_2$ substitué par un groupe alcoxy en $C_1$-$C_3$ ou cycloalkyle en $C_3$-$C_6$, un cycloalkyle en $C_3$-$C_6$, un phényle, un phényle mono- à trisubstitué par des groupes halogène, halogénoalkyle en $C_1$-$C_3$, alcoxy en $C_1$-$C_3$, alkyle en $C_1$-$C_3$, nitro et/ou cyano, un phényle substitué par un groupe phénoxy, halogénophénoxy, phényle, benzyle ou halogénobenzyle, un benzyle ou un benzyle mono- à trisubstitué par des groupes halogène, halogénoalkyle en $C_1$-$C_3$, méthyle, méthoxy, nitro et/ou cyano ; et $R_3$ représente un alkyle en $C_1$-$C_4$, un alkyle en $C_1$-$C_2$ substitué par un groupe

alcoxy en $C_1$-$C_3$, un alcényle en $C_3$-$C_4$, un benzyle ou un halogénobenzyle.

**8.** Procédé selon la revendication 7, caractérisé en ce qu'on prépare comme produit du procédé un composé de formule I, où $R_1$ représente un phényle, un phényle mono- à trisubstitué par des groupes halogène ou monosubstitué par un groupe phénoxy ou halogénophénoxy ; $R_2$ représente un alkyle en $C_1$-$C_6$ ou un cycloalkyle en $C_3$-$C_7$ et $R_3$ est un alkyle en $C_1$-$C_6$, un alkyle en $C_1$-$C_6$ substitué par un groupe alcoxy en $C_1$-$C_3$, un alcényle en $C_3$-$C_4$, un benzyle ou un halogénobenzyle.

**9.** Procédé pour la préparation de composés de formule IV' :

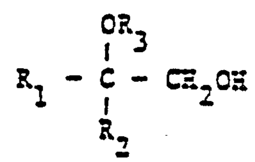

où $R_1$ représente un alkyle en $C_1$-$C_6$ substitué par un groupe alcoxy en $C_1$-$C_6$ ou cycloalkyle en $C_3$-$C_6$, un cycloalkyle en $C_3$-$C_8$, un phényle, un phényle mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, nitro et/ou cyano, un phényle substitué par un groupe phénoxy, halogénophénoxy, phényle, halogénophényle, benzyle ou halogénobenzyle, un benzyle ou un benzyle mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, nitro et/ou cyano ; $R_2$ désigne un alkyle en $C_1$-$C_{12}$ ou un reste tel qu'indiqué sous $R_1$ ; et $R_3$ est un alkyle en $C_1$-$C_6$, un alkyle en $C_1$-$C_6$ substitué par un groupe alcoxy en $C_1$-$C_3$, un alcényle en $C_3$-$C_4$, un benzyle ou un halogénobenzyle,
caractérisé en ce qu'on fait réagir un oxiranne de formule II

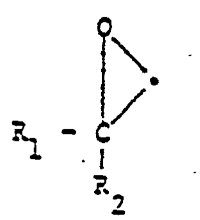

(II)

à des températures de -20° à +100°C en présence d'un catalyseur acide ou d'un agent de condensation acide avec un alcool de formule III

$R_3$ - OH (III)

$R_1$, $R_2$ et $R_3$ dans les formules II et III ayant les significations indiquées pour la formule IV'.

**10.** Procédé selon la revendication 9 pour la préparation de composés de formule IV', où $R_1$ représente un phényle, un phényle mono- à trisubstitué par des groupes halogène ou un phényle monosubstitué par un groupe phénoxy ou halogénophénoxy ; $R_2$ représente un alkyle en $C_1$-$C_6$ ou un cycloalkyle en $C_3$-$C_7$ et $R_3$ représente un alkyle en $C_1$-$C_6$, un alkyle en $C_1$-$C_6$ substitué par un groupe alcoxy en $C_1$-$C_3$, un alcényle en $C_3$-$C_4$, un benzyle ou un halogénobenzyle.

**11.** Agent microbicide, caractérisé en ce qu'il contient, outre 99,9 à 1% d'additif solide ou liquide et 0 à 25% d'un agent de surface, en tant qu'agent actif 0,1 à 99% d'un composé de formule Ia

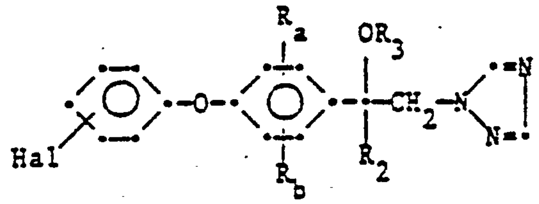

(Ia)

où Hal désigne un halogène ;
$R_a$ et $R_b$ représentent, indépendamment l'un de l'autre, l'hydrogène, un halogène, un halogénoalkyle en $C_1$-$C_6$, un halogénoalcoxy en $C_1$-$C_6$, un alcoxy en $C_1$-$C_6$, un alkyle en $C_1$-$C_6$, un groupe nitro et/ou un groupe cyano ;
$R_2$ représente un alkyle en $C_1$-$C_{12}$, un alkyle en $C_1$-$C_6$ substitué par un groupe alcoxy en $C_1$-$C_6$ ou cycloalkyle en $C_3$-$C_8$, un cycloalkyle en $C_3$-$C_8$, un phényle non substitué ou mono- à trisubstitué par des groupes halogène, halogénoalkyle en $C_1$-$C_6$, halogénoalcoxy en $C_1$-$C_6$, alcoxy en $C_1$-$C_6$, alkyle en $C_1$-$C_6$, phénoxy, halogénophénoxy, phényle, benzyle, halogénobenzyle, nitro et/ou cyano ou un benzyle non substitué ou mono- à trisubstitué par des groupes halogène, halogénoalkyle en $C_1$-$C_6$, alcoxy en $C_1$-$C_6$, alkyle en $C_1$-$C_6$, nitro et/ou cyano ; et
$R_3$ représente un alkyle en $C_1$-$C_6$ non substitué ou substitué par un groupe alcoxy en $C_1$-$C_3$, un alcényle en $C_3$-$C_4$, un benzyle ou un halogénobenzyle.

**12.** Agent selon la revendication 11, caractérisé en ce que Hal désigne le fluor, le chlore et/ou le brome ; $R_a$ et $R_b$ représentent, indépendamment l'un de l'autre, l' hydrogène, un halogène, un halogénoalkyle en $C_1$-$C_3$, un halogénoalcoxy en $C_1$-$C_3$, un alcoxy en $C_1$-$C_3$, un alkyle en $C_1$-$C_3$, un groupe nitro et/ou un groupe cyano ; $R_2$ désigne un alkyle en $C_1$-$C_6$ ; et $R_3$ représente un alkyle en $C_1$-$C_6$ non substitué ou substitué par un groupe alcoxy en $C_1$-$C_3$, un alkyle en $C_1$-$C_6$, un alcényle en $C_3$-$C_4$, un benzyle ou un halogénobenzyle.

**13.** Agent selon la revendication 11, caractérisé en ce qu'il contient comme agent actif un composé de formule Ia, choisi parmi les suivants :
1-(1H-1,2,4-triazol-1-yl)-2-méthoxy-2-[4-(4-chlorophénoxy)-phényl]-butane ;
1-(1H-1,2,4-triazol-1-yl)-2-méthoxy-2-[4-(4-fluorophénoxy)-phényl]-butane ;
1-(1H-1,2,4-triazol-1-yl)-2-méthoxy-2-[4-(2,4-dichlorophénoxy)-phényl]-butane ;
1-(1H-1,2,4-triazol-1-yl)-2-méthoxy-2-[4-(4-chlorophénoxy)-phényl]-propane ;
1-(1H-1,2,4-triazol-1-yl)-2-méthoxy-2-[4-(4-chlorophénoxy)-2-chlorophényl]-butane ;
1-(1H-1,2,4-triazol-1-yl)-2-méthoxy-2-[4-(4-chlorophénoxy)-phényl]-3-méthyl-butane ;
1-(1H-1,2,4-triazol-1-yl)-2-allyloxy-2-[4-(4-chlorophénoxy)-2-chlorophényl]-pentane ;
1-(1H-1,2,4-triazol-1-yl)-2-méthoxy-2-[4-(4-chlorophénoxy)-2-chlorophényl]-pentane ;
1-(1H-1,2,4-triazol-1-yl)-2-allyloxy-2-[4-(4-chlorophénoxy)-2-chlorophényl]-butane ;
1-(1H-1,2,4-triazol-1-yl)-2-(2-méthylallyloxy)-2-[4-(4-chlorophénoxy)-2-chlorophényl]-butane ;
1-(1H-1,2,4-triazol-1-yl)-2-allyloxy-2-[4-(4-chlorophénoxy)-2-chlorophényl]-propane ;
1-(1H-1,2,4-triazol-1-yl)-2-méthoxy-2-[4-(4-chlorophénoxy)-2-chlorophényl]-propane ;
1-(1H-1,2,4-triazol-1-yl)-2-méthoxy-2-[4-(4-chlorophénoxy)-2-méthylphényl]-propane ;
1-(1H-1,2,4-triazol-1-yl)-2-allyloxy-2-[4-(4-chlorophénoxy)-2-méthylphényl]-propane ;
1-(1H-1,2,4-triazol-1-yl)-2-méthoxy-2-[4-(4-chlorophénoxy)-2-méthylphényl]-butane ;
1-(1H-1,2,4-triazol-1-yl)-2-(2-méthylpropoxy)-[4-(4-chlorophénoxy)-2-chlorophényl]-butane ;
1-(1H-1,2,4-triazol-1-yl)-2-allyloxy-2-[4-(4-chlorophénoxy)-2-méthylphényl]-pentane ;
1-(1H-1,2,4-triazol-1-yl)-2-méthoxy-2-[4-(4-chlorophénoxy)-2-méthylphényl]-butane ;
1-(1H-1,2,4-triazol-1-yl)-2-allyloxy-2-[4-(4-chlorophénoxy)-2-méthylphényle]-butane ;
1-(1H-1,2,4-triazol-1-yl)-2-propoxy-2-[4-(4-chlorophénoxy)-2-chlorophényl]-pentane ;
1-(1H-1,2,4-triazol-1-yl)-2-n-propoxy-2-[4-(4-chlorophénoxy)-2-chlorophényl]-butane ;
1-(1H-1,2,4-triazol-1-yl)-2-n-propoxy-2-[4-(4-chlorophénoxy)-2-chlorophényl]-propane ;
1-(1H-1,2,4-triazol-1-yl)-2-n-propoxy-2-[4-(4-chlorophénoxy)-2-méthylphényl]-propane.

**14.** Procédé pour la lutte contre les microorganismes phytopathogènes, caractérisé en ce qu'on applique 10 g à 5 kg d'un composé de formule Ia selon la revendication 11 par hectare de surface cultivable.